# EUROPÄISCHE PATENTSCHRIFT

(11) **EP 3 693 437 B1**
(45) Veröffentlichungstag und Bekanntmachung des Hinweises auf die Patenterteilung: **25.08.2021**
(21) Anmeldenummer: 20160631.6
(22) Anmeldetag: 26.11.2014
(51) Int. Cl.: C09K 11/06, C07C 211/61, C07D 209/86, C07D 209/88, C07D 307/81, C07D 307/91, C07D 333/76, H01L 51/50

(54) **VERBINDUNGEN UND ORGANISCHE ELEKTRONISCHE VORRICHTUNGEN**
COMPOUNDS AND ORGANIC ELECTRONIC DEVICES
COMPOSÉS ET DISPOSITIFS ÉLECTRO-ORGANIQUES

(30) Priorität: 06.12.2013 EP 13005697
(43) Veröffentlichungstag der Anmeldung: 12.08.2020
(62) Teilanmeldung aus: 18157771.9
(73) Patentinhaber: Merck Patent GmbH, 64293 Darmstadt (DE)
(72) Erfinder: Pfister, Jochen, 64342 SEEHEIM-JUGENHEIM (DE); Stieber, Frank, 64683 Einhausen (DE); Montenegro, Elvira, 69469 WEINHEIM (DE); Mujica-Fernaud, Teresa, 38004 SANTA CRUZ DE TENERIFE (ES); Voges, Frank, 67098 BAD DUERKHEIM (DE)

(56) Entgegenhaltungen:
- WO-A1-2013/017192
- US-A1- 2011 198 581

## Beschreibung

Die vorliegende Erfindung betrifft neue organische Verbindungen, die Verwendung von Verbindung in einer elektronischen Vorrichtung, sowie eine elektronische Vorrichtung enthaltend wenigstens eine der Verbindungen. Weiterhin betrifft die vorliegende Erfindung ein Verfahren zur Herstellung der Verbindungen sowie Zusammensetzungen und Formulierungen enthaltend wenigstens eine der Verbindungen.

Die Entwicklung von funktionellen Verbindungen zur Verwendung in elektronischen Vorrichtungen ist aktuell Gegenstand intensiver Forschung. Ziel ist hierbei insbesondere die Entwicklung von Verbindungen, mit denen verbesserte Eigenschaften der elektrolumineszierenden Vorrichtungen in einem oder mehreren relevanten Punkten erzielt werden können, wie beispielsweise Leistungseffizienz, Lebensdauer oder Farbkoordinaten des emittierten Lichts.

Unter dem Begriff elektronische Vorrichtung werden gemäß der vorliegenden Erfindung unter anderem organische integrierte Schaltungen (OICs), organische Feld-Effekt-Transistoren (OFETs), organische Dünnfilmtransistoren (OTFTs), organische lichtemittierende Transistoren (OLETs), organische Solarzellen (OSCs), organische optische Detektoren, organische Photorezeptoren, organische Feld-Quench-Devices (OFQDs), organische lichtemittierende elektrochemische Zellen (OLECs), organische Laserdioden (O-Laser) und organische Elektrolumineszenzvorrichtungen (OLEDs) verstanden.

Von besonderem Interesse ist die Bereitstellung von Verbindungen zur Verwendung in den zuletzt genannten, als OLEDs bezeichneten elektronischen Vorrichtungen. Der allgemeine Aufbau sowie das Funktionsprinzip von OLEDs ist dem Fachmann gut bekannt und unter anderem in US 4539507, US 5151629, EP 0676461 und WO 1998/27136 beschrieben.

Betreffend die Leistungsdaten von OLEDs sind noch weitere Verbesserungen erforderlich, insbesondere in Hinblick auf eine breite kommerzielle Verwendung, beispielsweise in Anzeigevorrichtungen oder als Lichtquellen. Von besonderer Bedeutung sind in diesem Zusammenhang die Lebensdauer, die Effizienz und die Betriebsspannung der OLEDs sowie die realisierten Farbwerte. Zudem ist es wünschenswert, dass die Verbindungen zur Verwendung als Funktionsmaterialien in elektronischen Vorrichtungen eine hohe thermische Stabilität und eine hohe Glasübergangstemperatur aufweisen und sich unzersetzt sublimieren lassen.

In diesem Zusammenhang besteht insbesondere Bedarf an alternativen Lochtransportmaterialien. Bei Lochtransportmaterialien gemäß dem Stand der Technik steigt im Allgemeinen die Spannung mit zunehmender Schichtdicke der Lochtransportschicht an. In der Praxis wäre häufig eine höhere Schichtdicke der Lochtransportschicht wünschenswert, dies hat jedoch oftmals eine höhere Betriebsspannung und schlechtere Leistungsdaten zur Folge. In diesem Zusammenhang besteht Bedarf an neuen Lochtransportmaterialien, die eine hohe Ladungsträgerbeweglichkeit aufweisen, so dass dickere Lochtransportschichten mit lediglich geringem Anstieg der Betriebsspannung realisiert werden können.

Im Stand der Technik wird die Verwendung von verschiedenen Fluorenen als Ladungstransportmaterial in elektronischen und elektrolumineszierenden Vorrichtungen beschrieben.

In WO 2011/055493 werden sekundäre Amine offenbart, die mehrfach mit Fluorenen in Position 3 substituiert sind.

JP 2008-34701 und WO 2007/072952 offenbaren Fluorene, die in Position 4 mit einer Amingruppe substituiert sind, wobei die Amingruppe selbst wieder mehrere Fluorene enthält.

WO 2010/110553 offenbart mit Amingruppen in Position 2, 3 oder 4 substituierte Fluorene, wobei die Amingruppen Carbazolgruppen enthalten. In JP 05303221 werden Fluorene offenbart, die in Position 2 oder 4 mit einer Amingruppe substituiert sein können. Die Verbindungen mit der Amingruppe in Position 4 des Fluorens enthalten Phenyl-Reste. Die Verbindungen werden als Photorezeotoren eingesetzt.

In WO2013017192 A1 werden weiter Fluorene offenbart.

Trotz der bereits bekannten Verbindungen besteht unverändert Bedarf an neuen Lochtransport- und Lochinjektionsmaterialien zur Verwendung in OLEDs. Insbesondere besteht Bedarf an Materialien, mit denen die oben genannten, hoch erwünschten Verbesserungen der Leistungsdaten und Eigenschaften der OLEDs erreicht werden können.

Ebenfalls besteht Bedarf an neuen Matrixmaterialien zur Verwendung in OLEDs sowie in anderen elektronischen Vorrichtungen. Insbesondere besteht Bedarf an Matrixmaterialien für phosphoreszierende Dotanden sowie an Matrixmaterialien für Mixed-Matrix-Systeme, welche bevorzugt zu guter Effizienz, hoher Lebensdauer und geringer Betriebsspannung der elektronischen Vorrichtungen führen.

Der vorliegenden Erfindung liegt somit die Aufgabe zu Grunde, elektroluminesziernede Vorrichtungen und Verbindungen, welche sich zur Verwendung in elektrolumineszierenden Vorrichtungen wie beispielsweise in fluoreszierenden oder phosphoresziereneden OLEDs eignen, bereitzustellen und welche insbesondere als Lochtransportmaterialien und/oder als Lochinjektionsmaterialien in einer Lochtransport- bzw. Exzitonenblockierschicht oder als Matrixmaterial in eienr emittierenden Schicht eingesetzt werden können.

Im Rahmen der vorliegenden Erfindung wurde überraschend gefunden, dass sich Verbindungen der unten angegebenen Formel (1) ausgezeichnet für die oben genannten Verwendungen in elektronischen und insbesondere in elektrolumineszierenden Vorrichtungen eignen.

Gegenstand der Erfindung ist somit eine Verbindung der allgemeinen Formel (1) 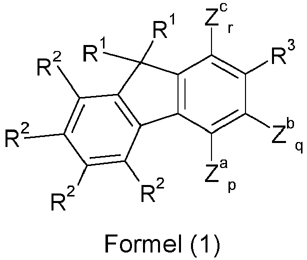 wobei für die verwendeten Symbole und Indices gilt:
- R¹: ist bei jedem Auftreten gleich oder verschieden, bevorzugt gleich, H, D, F, Cl, Br, I, C(=O)R⁴, CN, Si(R⁴)₃, NO₂, P(=O)(R⁴)₂, S(=O)R⁴, S(=O)₂R⁴, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch -R⁴C=CR⁴-, -C≡C-, Si(R⁴)₂, C=O, C=S, C=NR⁴, -C(=O)O-, -C(=O)NR⁴-, P(=O)(R⁴), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R⁴ substituiert sein kann, oder eine Aryloxygruppe mit 5 bis 60 aromatischen Ringatomen, die mit einem oder mehreren Resten R⁴ substituiert sein kann, oder einer Aralkylgruppe mit 5 bis 60 aromatischen Ringatomen, die jeweils mit einem oder mehreren Resten R⁴ substituiert sein kann, wobei die beiden Reste R¹ miteinander verknüpft sein können und einen Ring bilden können, so dass eine Spiroverbindung in Position 9 des Fluorens entsteht, wobei Spirobifluorene ausgeschlossen sind;
- R², R³: sind bei jedem Auftreten gleich oder verschieden, bevorzugt gleich, H, D, F, Cl, Br, I, C(=O)R⁴, CN, Si(R⁴)₃, NO₂, P(=O)(R⁴)₂, S(=O)R⁴, S(=O)₂R⁴, N(R⁴)₂, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch -R⁴C=CR⁴-, -C≡C-, Si(R⁴)₂, C=O, C=S,
C=NR⁴, -C(=O)O-, -C(=O)NR⁴-, P(=O)(R⁴), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R⁴ substituiert sein kann, oder eine Aryloxygruppe mit 5 bis 60 aromatischen Ringatomen, die mit einem oder mehreren Resten R⁴ substituiert sein kann, oder einer Aralkylgruppe mit 5 bis 60 aromatischen Ringatomen, die jeweils mit einem oder mehreren Resten R⁴ substituiert sein kann, wobei zwei oder mehr Reste R² oder zwei oder mehr Reste R³ miteinander verknüpft sein können und einen Ring bilden können;
- R⁴: ist bei jedem Auftreten gleich oder verschieden H, D, F, Cl, Br, I, C(=O)R⁵, CN, Si(R⁵)₃, NO₂, P(=O)(R⁵)₂, S(=O)R⁵, S(=O)₂R⁵, , N(R⁵)₂, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁵ substituiert sein können und wobei eine oder mehrere CH₂-Gruppen in den oben genannten Gruppen durch -R⁵C=CR⁵-, -C≡C-, Si(R⁵)₂, C=O, C=S, C=NR⁵, -C(=O)O-, -C(=O)NR⁵-, P(=O)(R⁵), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R⁵ substituiert sein kann, oder eine Aryloxy- oder Heteroaryloxygruppe mit 5 bis 30 aromatischen Ringatomen, die durch einen oder mehrere Reste R⁵ substituiert sein kann;
- R⁵: ist ausgewählt aus der Gruppe bestehend aus H, D, F, einem aliphatischem Kohlenwasserstoffrest mit 1 bis 20 C-Atomen oder einem aromatischem oder heteroaromatischen Ringsystem mit 5 bis 30 C-Atomen, in dem ein oder mehrere H-Atome durch D oder F ersetzt sein können, wobei zwei oder mehr benachbarte Substituenten R⁵ miteinander ein mono- oder polycyclisches, aliphatisches, Ringsystem bilden können;
- p, q, r: sind 0 oder 1, wobei p + q + r = 1 gilt, bevorzugt ist p =1 oder r = 1 und ganz bevorzugt ist p = 1;
- Z^{a}₀, Z^{b}₀, Z^{c}₀: sind gleich oder verschieden bei jedem Auftreten gleich R³
- Z^{a}₁, Z^{b}₁, Z^{c}₁: sind gleich 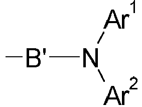
- B': ist eine Arylengruppe mit 6 bis 30 Ringatomen oder eine Heteroarylengruppe mit 5 bis 30 Ringatomen, die jeweils mit einem oder mehreren Resten R⁴ substituiert sein können,
bevorzugt ist B'eine Arylengruppe mit 6 bis 30 Ringatomen oder eine mono- oder bicyclische Heteroarylengruppe mit 5 bis 30 Ringatomen, die jeweils mit einem oder mehreren Resten R⁴ substituiert sein können,
ganz bevorzugt eine Phenylen-, Biphenylen-, Terphenylen-, Naphthylen-, Pyridinylen-, Pyrimidinylen-, Pyrazinylen-, Pyridazinylen-, Triazinylen-, Fluorenylen-, Dibenzofuranylen- oder Dibenzothiophenylengruppe, die mit einem oder mehreren Resten R⁴ substituiert sein können,
besonders bevorzugt eine Phenylen-, Biphenylen-, Terphenylen-, Naphthylen-, Dibenzofuranylen- oder Dibenzothiophenylengruppe, die mit einem oder mehreren Resten R⁴ substituiert sein können,
ganz besonders bevorzugt ist B' eine Phenylengruppe, die mit einem oder mehreren Resten R⁴ substituiert sein kann,
insbesondere bevorzugt ist B' eine Phenylengruppe, die unsubstituiert ist;
- Ar¹, Ar²: sind bei jedem Auftreten gleich oder verschieden ein Aromat mit 6 bis 60 Ringatomen oder ein Heteroaromat mit 5 bis 60 Ringatomen, welche mit einem oder mehreren Resten R⁶, die gleich oder verschieden voneinander sind, substituiert sein können; wobei wenigstens eine der beiden Gruppen Ar¹ und Ar² eine Fluorengruppe enthalten muss;
wobei zwei aromatische oder heteroaromatische Ringe in Ar¹ bzw. zwei aromatische oder heteroaromatische Ringe in Ar² zusätzlich kondensiert sein können, bevorzugt liegt keine zusätzliche Kondensation vor,
und wobei zwei aromatische oder heteroaromatische Ringe in Ar¹ zusätzlich durch eine bivalente Gruppe -O-, -S-, C(R⁶)₂ oder-Si(R⁶)₂- verbrückt sein können, wobei eine Verbrückung über -O-, C(R⁶)₂ oder -Si(R⁶)₂- bevorzugt ist, bzw. zwei aromatische oder heteroaromatische Ringe in Ar² durch eine bivalente Gruppe -O-,-S-, C(R⁶)₂ oder -Si(R⁶)₂- verbrückt sein können, wobei eine Verbrückung über -O-, C(R⁶)₂ oder -Si(R⁶)₂- bevorzugt ist, wobei ganz bevorzugt ist wenn keine zusätzliche Verbrückung der Ringe aus in Ar1 und keine zusätzliche Verbrückung der Ringe in Ar2 vorliegt;
dabei bedeutet eine Verbrückung der Ringe in Ar¹ oder in Ar² mit-O-, dass, beispielsweise, ein Dibenzofuran, entsteht; entsprechend kann durch Verbrückung mit -S-, beispielsweise, ein Dibenzothiophen entstehen; eine Verbrückung mit C(R⁶)₂ kann dazu führen, dass, beispielsweise, eine weitere Fluorengruppe oder ein Indenofluorengruppe gebildet wird; eine Indenofluorengruppe kann gebildet werden, wenn ein Phenylfluoren mit der Gruppe C(R⁶)₂ verbrückt wrd;
und wobei ein aromatischer oder heteroaromatischer Ring aus Ar¹ mit einem aromatischen oder heteroaromatischen Ring aus Ar² durch eine bivalente Gruppe -O-, -S-, -Si(R⁶)₂-, -NR⁶- oder -C(R⁶)₂-verbrückt sein kann, wobei bevorzugt ist, wenn die Gruppe Ar¹ nicht mit der Gruppe Ar² verbrückt ist;
und wobei weiterhin bevorzugt ist, wenn Ar¹ und Ar² mindestens zwei aromatische oder heteroaromatische Ringe enthalten;
- R⁶: ist bei jedem Auftreten gleich oder verschieden H, D, F, Cl, Br, I, C(=O)R⁵, CN, Si(R⁵)₃, NO₂, P(=O)(R⁵)₂, S(=O)R⁵, S(=O)₂R⁵, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁵ substituiert sein können und wobei eine oder mehrere CH₂-Gruppen in den oben genannten Gruppen durch -R⁵C=CR⁵⁻, -C=C-, Si(R⁵)₂, C=O, C=S, C=NR⁵, -C(=O)O-, -C(=O)NR⁵-, P(=O)(R⁵), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R⁵ substituiert sein kann, oder eine Aryloxy- oder Heteroaryloxygruppe mit 5 bis 30 aromatischen Ringatomen, die durch einen oder mehrere Reste R⁵ substituiert sein kann.

Die obigen Definitionen machen deutlich, dass die erfindungsgemäße Verbindung der Formel (1) in jedem Fall wenigstens eine Fluorengruppe in entweder der Gruppe Ar¹ oder in der Gruppe Ar² aufweist. Daher stellt der obige Zusatz "zusätzlich" in den Definitionen der Gruppen Ar¹ und Ar² klar, dass die in den Gruppen Ar¹ und Ar² wenigstens eine erforderliche Fluorengruppe von den bevorzugten Ausführungsformen der unverbrückten Ringe in Ar¹ und Ar² ausgenommen wird, d.h., die Verbindunge der Formel (1) enthält in jedem Fall wenigstens eine Fluorengruppe in entweder Ar¹ oder in Ar².

In einer bevorzugten Ausführungsform enthalten sowohl Ar¹ und Ar² jeweils eine Fluorengruppe.

Ganz bevorzugt ist, wenn die Verbindung der allgemeinen Formel (1) in den Gruppen Ar¹ und Ar² keine Carbazole enthält, wobei noch mehr bevorzugt ist, wenn die Verbindung der allgemeinen Formel (1) gar keine Carbazole enthält.

In einer ganz bevorzugten Ausführungsform der vorliegenden Erfindung enthält nur eine der beiden Gruppen Ar¹ und Ar² eine Fluorengruppe.

In einer weiterhin bevorzugten Ausführungsform enthält die Verbindung der Formel (1) keine benzannelierten Phenyle mit mehr als 12 verbundenen aromatischen Ringatomen. Unter benzannelierten Phenyle werden vorliegend Gruppen Verstanden, die durch Kondensation zweier oder mehrer Benzole erhalten werden. Dazu zählen, beispielsweise, Naphthalyl, Anthracenyl-, Tetracenyl-, Pentacenyl- und Pyrenylgruppen.

In einer weiterhin bevorzugten Ausführungsform enthält die Verbindung der Formel (1) keine kondensierten aromatischen Gruppen mit mehr als 13 verbundenen Ringatomen.

Ferner bevorzugt ist, wenn die Verbindung der Formel (1) keine kondensierten aromatischen Gruppen mit mehr als 13 verbundenen Ringatomen und keine kondensierten heteroaromatischen Gruppen mit mehr als 13 verbundenen Ringatomen enthält, wobei noch bevorzugter ist, wenn die Verbindung der allgemeinen Formel (1) gar keine keine kondensierten aromatischen oder heteroaromatischen Gruppen enthält.

Die Fluorene in der Verbindung der Formel (1) zählen dabei nicht zu den kondensierten aromatischen Gruppen, da zwei Phenyl an eine aliphatischen Ring kondensiert wurden.

Unter verbundenen Ringatomen werden vorliegend solche verstanden, die ausschließlich über Ringbindungen miteinander verbunden sind. Insofern weist 2-Phenyl-Naphthalin einen Ring (Phenylring) mit 6 verbundenen Ringatomen und einen Ring mit 10 verbundenen Ringatomen auf. 2-Phenyl-Naphthalin weist daher maximal 10 verbundene Ringatome auf. 2-Naphtylanthracen weist dementsprechend 14 verbundene Ringatome auf.

Die Zählweise am Fluoren ist dabei wie folgt festgelegt.

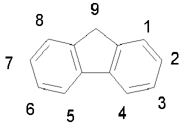

Eine Arylgruppe (Aromat) im Sinne dieser Erfindung enthält 6 bis 60 aromatische Ringatome; eine Heteroarylgruppe (Heteroaromat) im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Unter einer Aryloxygruppe gemäß der Definition der vorliegenden Erfindung wird eine Arylgruppe, wie oben definiert, verstanden, welche über ein Sauerstoffatom gebunden ist. Eine analoge Definition gilt für Heteroaryloxygruppen.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, Si-, N- oder O-Atom, ein sp²-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Es ist weiterhin bevorzugt, wenn B' in der Verbindung nach Formel (1) eine o-Phenylen-, m-Phenylen- oder p-Phenylengruppe, eine 1,4-Naphthylen-, 2,4-Naphthylen-, 1,5-Naphthylen- oder 2,6-Naphthylengruppe, eine 2,8-Dibenzofuranylengruppe oder eine 2,8-Dibenzothiophenylengruppe ist, oder eine 2,7-Fluorengruppe wobei ganz bevorzugt ist, wenn B' eine o-Phenylen-, m-Phenylen- oder p-Phenylengruppe und ganz besonders bevorzugt ist, wenn B' eine p-Phenylengruppe ist, wobei die Gruppen mit einem oder mehreren Resten R⁴ substituiert sein können, die bei jedem Auftreten gleich oder verschieden sein können, wobei bevorzugt ist, wenn die Gruppen unsubstituiert sind.

Bevorzugt im Sinne der vorliegenden Erfindung ist eine Verbindung der allgemeinen Formel (2) 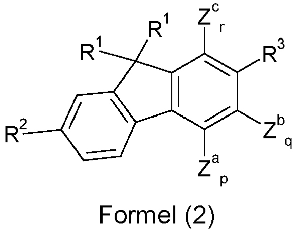 wobei für die verwendeten Indices und Symbole obige Definitionen gelten.

Weiterhin bevorzugt ist eine Verbindung der allgemeinen Formel (1) oder (2), dadurch charakterisiert, dass R¹ bei jedem Auftreten gleich oder verschieden, bevorzugt gleich, eine geradkettige Alkyl- oder Alkoxygruppe mit 1 bis 20 C-Atomen oder eine verzweigte oder cyclische Alkyl- oder Alkoxygruppe mit 3 bis 20 C-Atomen ist, wobei die oben genannten Gruppen jeweils mit einem oder mehreren Resten R⁴ substituiert sein können oder ein aromatisches oder heteroaromatisches Ringsystem mit 5 bis 30 aromatischen Ringatomen, das jeweils durch einen oder mehrere Reste R⁴ substituiert sein kann, oder eine Aryloxygruppe mit 5 bis 60 aromatischen Ringatomen, die mit einem oder mehreren Resten R⁴ substituiert sein kann, oder einer Aralkylgruppe mit 5 bis 60 aromatischen Ringatomen, die jeweils mit einem oder mehreren Resten R⁴ substituiert sein kann, wobei die beiden Reste R¹ miteinander verknüpft sein können und einen Ring bilden können, so dass eine Spiroverbindung in Position 9 des Fluorens entsteht, wobei Spirobifluorene ausgeschlossen sind.

Weiterhin ganz bevorzugt ist eine Verbindung der allgemeinen Formel (1) oder (2), dadurch charakterisiert, dass R¹ bei jedem Auftreten gleich oder verschieden, bevorzugt gleich, eine geradkettige Alkylgruppe mit 1 bis 20 C-Atomen oder eine verzweigte oder cyclische Alkylgruppe mit 3 bis 20 C-Atomen ist, wobei die oben genannten Gruppen jeweils mit einem oder mehreren Resten R⁴ substituiert sein können oder ein aromatisches oder heteroaromatisches Ringsystem mit 6 bis 30 aromatischen Ringatomen, das jeweils durch einen oder mehrere Reste R⁴ substituiert sein kann, wobei die beiden Reste R¹ miteinander verknüpft sein können und einen Ring bilden können, so dass eine Spiroverbindung in Position 9 des Fluorens entsteht, wobei Spirobifluorene ausgeschlossen sind. sind

Weiterhin ganz besonders bevorzugt ist eine Verbindung der allgemeinen Formel (1) oder (2), dadurch charakterisiert, dass R¹ bei jedem Auftreten gleich eine geradkettige Alkylgruppe mit 1 bis 20 C-Atomen oder eine verzweigte oder cyclische Alkylgruppe mit 3 bis 20 C-Atomen ist, wobei die oben genannten Gruppen jeweils mit einem oder mehreren Resten R⁴ substituiert sein können, wobei insbesondere bevorzugt ist, wenn R¹ eine Methyl-, Ethyl-, n-/i-Propyl- oder n-/i-/t-Butylgruppe ist.

Schließlich ist weiterhin ganz besonders bevorzugt eine Verbindung der allgemeinen Formel (1) oder (2), dadurch charakterisiert, dass R¹ bei jedem Auftreten ein aromatisches oder heteroaromatisches Ringsystem mit 6 bis 30 aromatischen Ringatomen ist, das jeweils durch einen oder mehrere Reste R⁴ substituiert sein kann, wobei das Ringsystem insbesondere bevorzugt ausgewählt ist aus der Gruppe bestehend aus einer Phenyl-, Biphenyl-, Terphenyl- oder Pyridylgruppe.

Die beiden in Position 9 des Fluorens befindichen Reste R¹ können gleich oder verscheiden sein, wobei bevorzugt ist, wenn sie gleich sind.

Weiterhin bevorzugt ist eine Verbindung der allgemeinen Formel (1) oder (2), dadurch charakterisiert, dass R² bei jedem Auftreten gleich oder verschieden, bevorzugt gleich, ausgewählt ist aus H, D, F, Cl, Br, I, N(R⁵)₂, einer geradkettigen Alkyl-, Alkoxylgruppe mit 1 bis 20 C-Atomen oder einer verzweigten oder cyclischen Alkyl-, Alkoxygruppe mit 3 bis 20 C-Atomen oder einer Alkenyl- oder Alkinylgruppe mit 2 bis 20 C-Atomen, wobei die oben genannten Gruppen jeweils mit einem oder mehreren Resten R⁴ substituiert sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 6 bis 30 aromatischen Ringatomen, das jeweils durch einen oder mehrere Reste R⁴ substituiert sein kann, oder eine Aryloxygruppe mit 5 bis 60 aromatischen Ringatomen, die mit einem oder mehreren Resten R⁴ substituiert sein kann, oder einer Aralkylgruppe mit 5 bis 60 aromatischen Ringatomen, die jeweils mit einem oder mehreren Resten R⁴ substituiert sein kann, wobei zwei oder mehr Reste R² miteinander verknüpft sein können und einen Ring bilden können;

Weiterhin ganz bevorzugt ist eine Verbindung der allgemeinen Formel (1) oder (2), dadurch charakterisiert, dass R² bei jedem Auftreten gleich oder verschieden, bevorzugt gleich, ausgewählt ist aus H, D, F, Cl, Br, I, N(R⁵)₂, einer geradkettigen Alkylgruppe mit 1 bis 20 C-Atomen oder einer verzweigten oder cyclischen Alkylgruppe mit 3 bis 20 C-Atomen, wobei die oben genannten Gruppen jeweils mit einem oder mehreren Resten R⁴ substituiert sein können und wobei ein oder mehrere H-Atome in den oben genannten Gruppen durch D, F, Cl, Br, I, CN oder NO₂ ersetzt sein können, oder einem aromatischen oder heteroaromatischen Ringsystem mit 6 bis 30 aromatischen Ringatomen, das jeweils durch einen oder mehrere Reste R⁴ substituiert sein kann.

In einer ganz besonders bevorzugten Ausführungsform betrifft die vorliegende Erfindung eine Verbindung der allgemeinen Formel (1), die dadurch charakterisiert ist, dass R² gleich H ist.

In einer weiteren ganz besonders bevorzugten Ausführungsform betrifft die vorliegende Erfindung eine Verbindung der allgemeinen Formel (1), die dadurch charakterisiert ist, dass R² eine geradkettigen Alkylgruppe mit 1 bis 20 C-Atomen oder einer verzweigten oder cyclischen Alkylgruppe mit 3 bis 20 C-Atomen ist.

In noch einer weiteren ganz besonders bevorzugten Ausführungsform betrifft die vorliegende Erfindung eine Verbindung der allgemeinen Formel (1), die dadurch charakterisiert ist, dass R² ein aromatisches oder heteroaromatisches Ringsystem mit 6 bis 30 aromatischen Ringatomen darstellt.

Weiterhin bevorzugt ist eine Verbindung der allgemeinen Formel (1) oder (2), dadurch charakterisiert, dass R³ bei jedem Auftreten gleich oder verschieden ausgewählt ist aus H, D, F, Cl, Br, I, N(R⁵)₂, eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei die oben genannten Gruppen jeweils mit einem oder mehreren Resten R⁴ substituiert sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 6 bis 30 aromatischen Ringatomen, das jeweils durch einen oder mehrere Reste R⁴ substituiert sein kann, oder eine Aryloxygruppe mit 5 bis 60 aromatischen Ringatomen, die mit einem oder mehreren Resten R⁴ substituiert sein kann, oder einer Aralkylgruppe mit 5 bis 60 aromatischen Ringatomen, die jeweils mit einem oder mehreren Resten R⁴ substituiert sein kann, wobei zwei oder mehr Reste R³ miteinander verknüpft sein können und einen Ring bilden können.

Bevorzugt im Sinne der vorliegenden Erfindung ist eine Verbindung der allgemeinen Formel (1a) 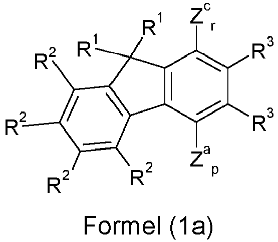 wobei obige Definitionen für die verwendeten Symbole und Indices, sowie die hierin beschriebenen bevorzugten Ausführungsformen gelten.

Ganz bevorzugt im Sinne der vorliegenden Erfindung ist eine Verbindung der allgemeinen Formel (2a) 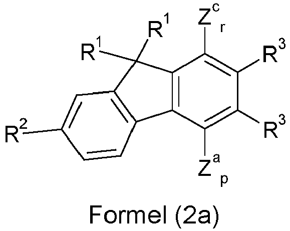 wobei obige Definitionen für die verwendeten Symbole und Indices, sowie die hierin beschriebenen bevorzugten Ausführungsformen gelten.

In einer bevorzugten Ausführungsform betrifft die vorliegende Erfindung eine Verbindung der allgemeinen Formel (3) 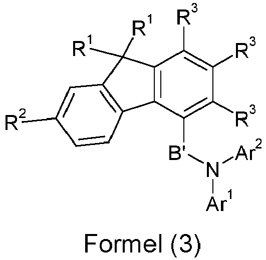 wobei für die verwendeten Symbole und Indices obige Definitionen gelten.

In einer weiterhin bevorzugten Ausführungsform betrifft die vorliegende Erfindung eine Verbindung der allgemeinen Formel (4) 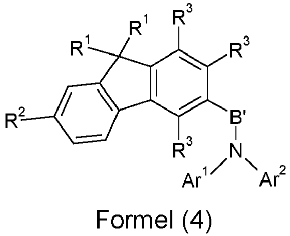 wobei für die verwendeten Symbole und Indices obige Definitionen gelten.

Wenn sich das Amin mit den Gruppen Ar¹ und Ar² in der Position 3 des Fluorens befindet ist besonders bevorzugt, wenn die Gruppe Ar¹ bzw. die Gruppe Ar² keine Verbrückung über Sauerstoff aufweist, da die Verwendung dieser Verbindungen in OLEDs zu besonders vorteilhaften Leistungsdaten führen.

In einer weiterhin bevorzugten Ausführungsform betrifft die vorliegende Erfindung eine Verbindung der allgemeinen Formel (5) 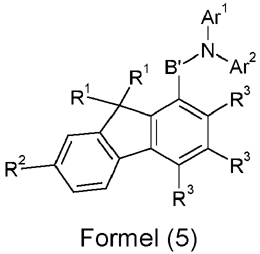 wobei für die verwendeten Symbole und Indices obige Definitionen gelten.

In einer ganz bevorzugten Ausführungsform betrifft die vorliegende Erfindung eine Verbindung der allgemeinen Formel (6) 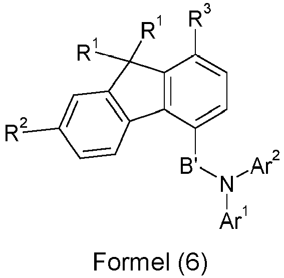 wobei für die verwendeten Symbole und Indices obige Definitionen gelten.

In einer weiterhin ganz bevorzugten Ausführungsform betrifft die vorliegende Erfindung eine Verbindung der allgemeinen Formel (7) 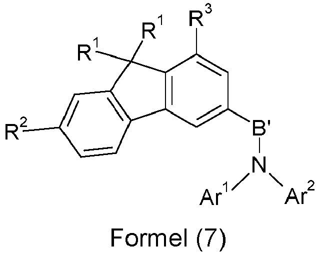 wobei für die verwendeten Symbole und Indices obige Definitionen gelten.

In einer weiterhin ganz bevorzugten Ausführungsform betrifft die vorliegende Erfindung eine Verbindung der allgemeinen Formel (8) 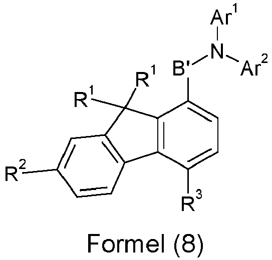 wobei für die verwendeten Symbole und Indices obige Definitionen gelten.

In einer ganz besonders bevorzugten Ausführungsform betrifft die vorliegende Erfindung eine Verbindung der allgemeinen Formel (9) 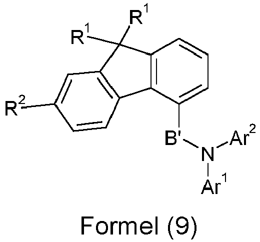 wobei für die verwendeten Symbole und Indices obige Definitionen gelten.

In einer weiterhin ganz besonders bevorzugten Ausführungsform betrifft die vorliegende Erfindung eine Verbindung der allgemeinen Formel (10) 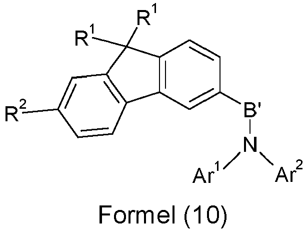 wobei für die verwendeten Symbole und Indices obige Definitionen gelten.

In einer weiterhin ganz besonders bevorzugten Ausführungsform betrifft die vorliegende Erfindung eine Verbindung der allgemeinen Formel (11) 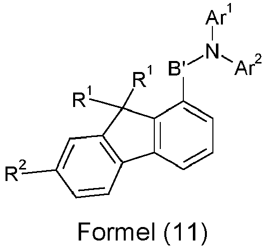 wobei für die verwendeten Symbole und Indices obige Definitionen gelten.

In einer insbesondere bevorzugten Ausführungsform betrifft die vorliegende Erfindung eine Verbindung der allgemeinen Formel (12) 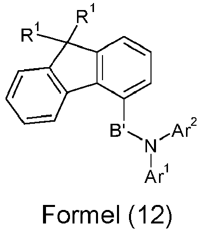 wobei für die verwendeten Symbole und Indices obige Definitionen gelten.

In einer weiterhin insbesondere bevorzugten Ausführungsform betrifft die vorliegende Erfindung eine Verbindung der allgemeinen Formel (13) 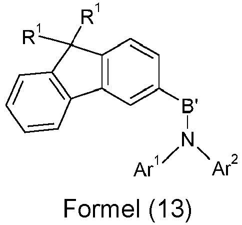 wobei für die verwendeten Symbole und Indices obige Definitionen gelten.

In einer weiterhin insbesondere bevorzugten Ausführungsform betrifft die vorliegende Erfindung eine Verbindung der allgemeinen Formel (14) 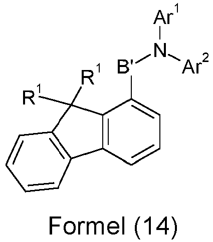 wobei für die verwendeten Symbole und Indices obige Definitionen gelten.

Weiterhin bevorzugt ist eine Verbindung der oben angegebenen Formeln (1) bis (14) in denen B' ausgewählt ist aus den Gruppen der Formeln (15) bis (36b), wobei diese Gruppen noch durch ein oder mehrere voneinander unabhängige Reste R⁴ substituiert sein können und wobei R⁴ wie oben angegeben definiert ist. 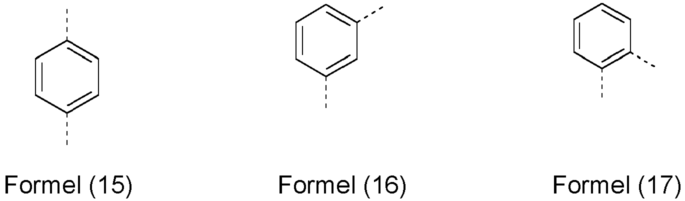 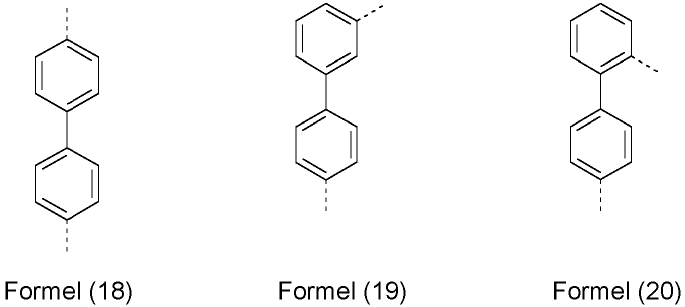 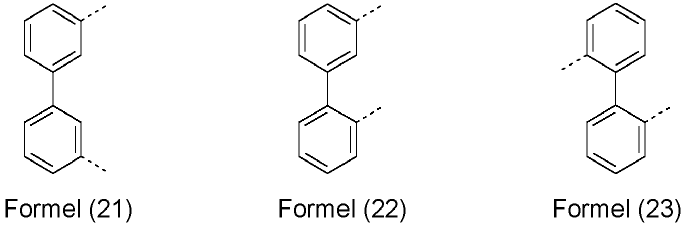 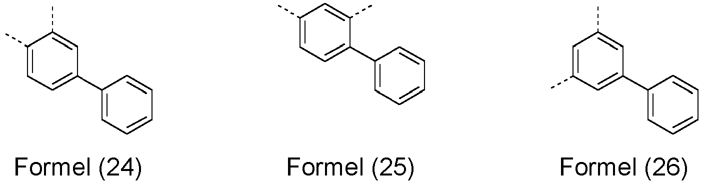 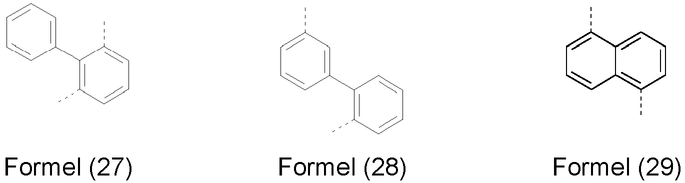 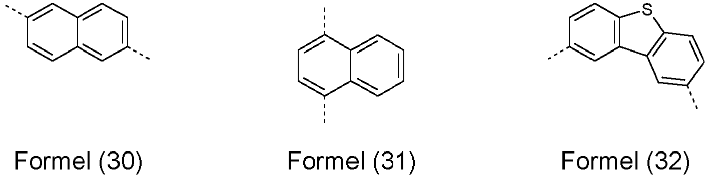 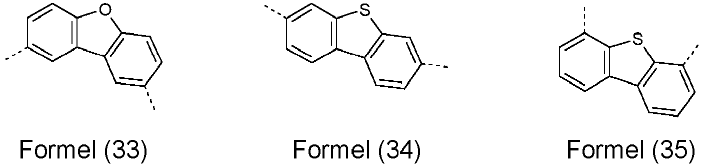 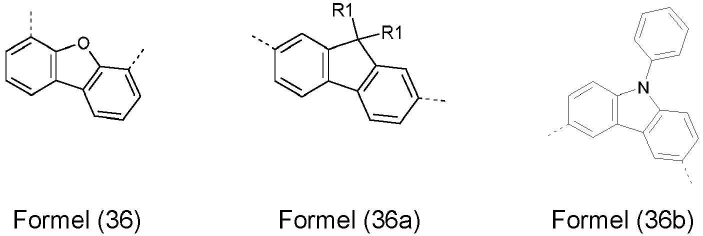 wobei die gestrichelten Linien die Verknüpfungspositionen kennzeichnen.

Ganz bevorzugt ist eine Verbindung der oben angegebenen Formeln (1) bis (14) in denen B' ausgewählt ist aus den Gruppen der Formeln (15) bis (36b), wobei diese Gruppen in einer ganz besonders bevorzugten Ausführungsform unsubstituiert sind.

Ganz besonders bevorzugt ist eine Verbindung der oben angegebenen Formeln (1) bis (14) in denen B' den Formeln (15) bis (17) entspricht, wobei diesen Gruppen bevorzugt unsubstituiert ist.

Bevorzugt sind Ar¹ und Ar² bevorzugt ausgewählt aus einer Phenyl-, Phenyl-Pyridyl-, Phenyl-Naphthyl-, Biphenyl-, Terphenyl- oder Quarterphenylgruppe, die mit einem oder mehreren Resten R⁶, die gleich oder verschieden voneinander sein können, substituiert sein können, wobei zwei der aromatischen oder heteroaromatischen Ringe in Ar¹ durch eine bivalente Gruppe -O-, -S-, C(R⁶)₂ oder -Si(R⁶)₂- zusätzlich verbrückt sein können bzw. zwei der aromatischen oder heteroaromatischen Ringe in Ar² durch eine bivalente Gruppe -O-, -S- -, C(R⁶)₂ oder -Si(R⁶)₂- zusätzlich verbrückt sein können, wobei bevorzugt ist, wenn keine zusätzliche Verbrückung vorliegt, und wobei ein aromatischer oder heteroaromatischer Ring aus Ar¹ mit einem aromatischen oder heteroaromatischen Ring aus Ar² durch eine bivalente Gruppe -O-, -S-, -Si(R⁶)₂-, -NR⁶- oder -C(R⁶)₂-verbrückt sein kann, wobei unverbrückte Gruppen Ar¹ und Ar² bevorzugt sind und wobei wenigstens eine der beiden Gruppen Ar¹ und Ar² eine Fluorengruppe enthält.

In einer ganz bevorzugten Ausführungsform der vorliegenden Erfindung sind Ar¹ und Ar² ausgewählt aus der folgenden Gruppen der Formeln (37) bis (122g), die mit einem oder mehreren Resten R⁶ substituiert sein können und wobei wenigstens eine der beiden Gruppen Ar¹ und Ar² eine Fluorengruppe enthält. 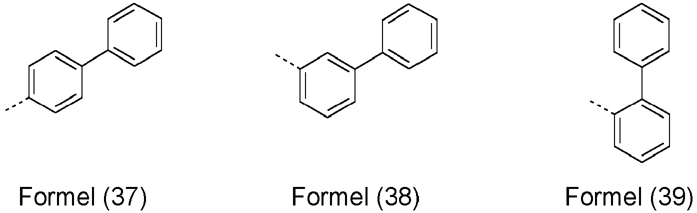 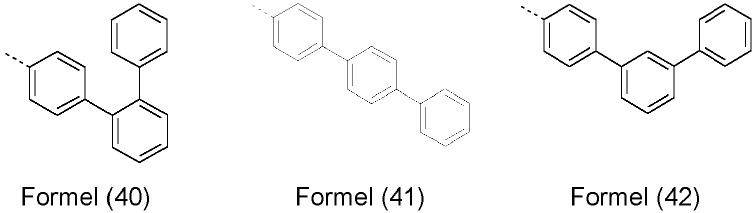 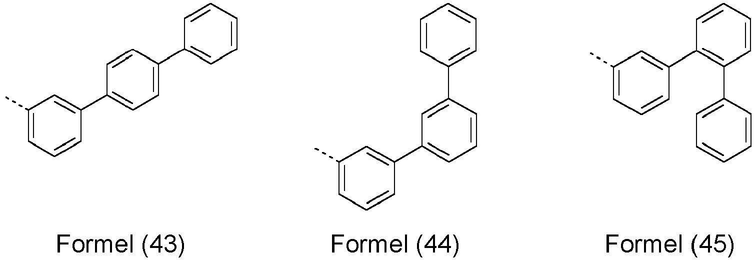 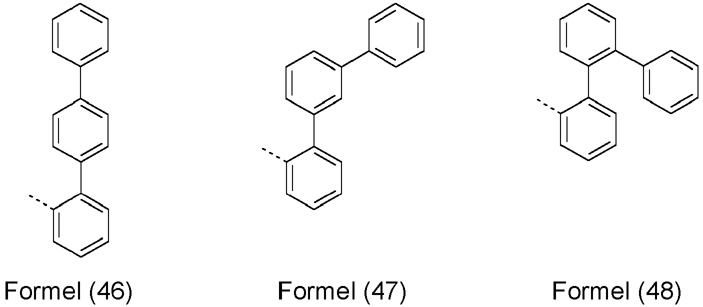 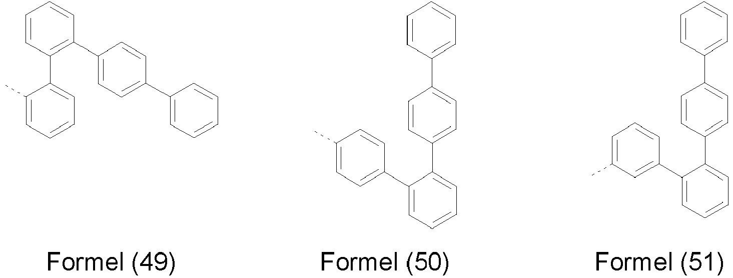 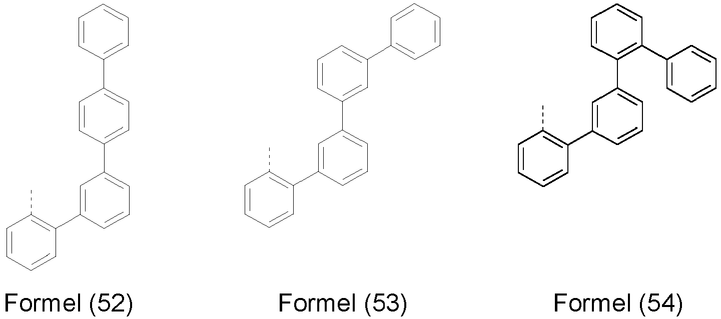 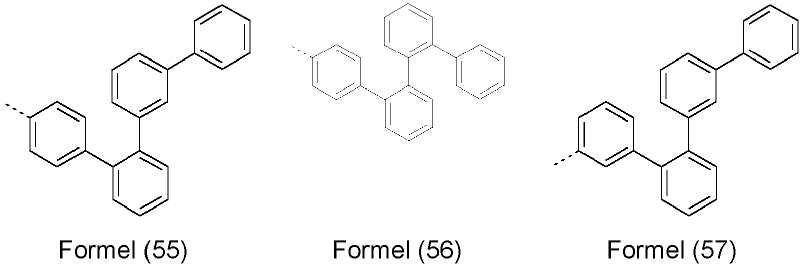 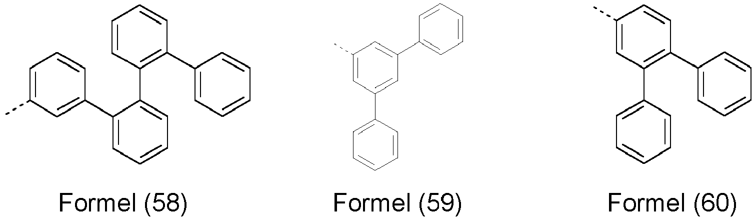 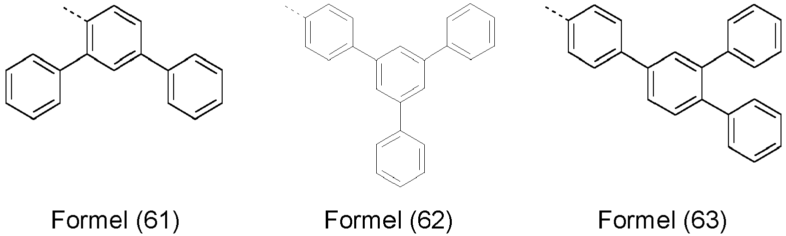 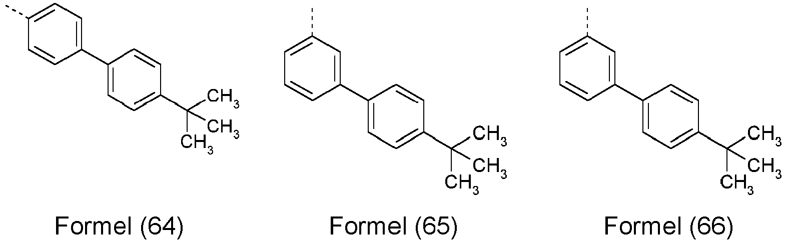 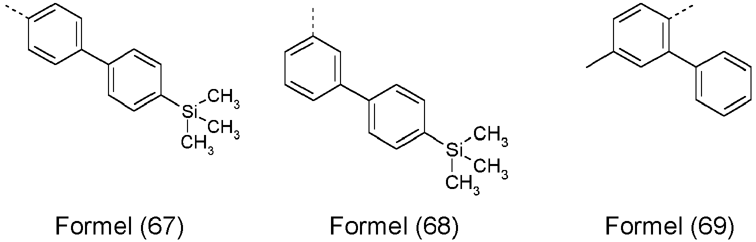 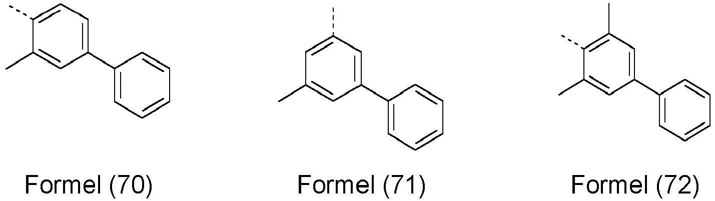 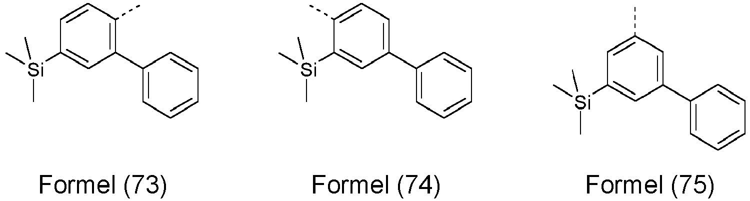 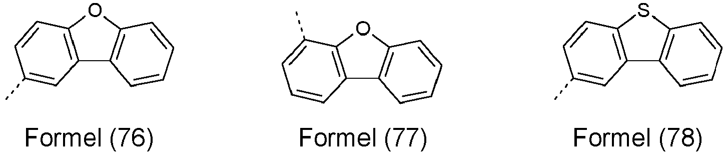 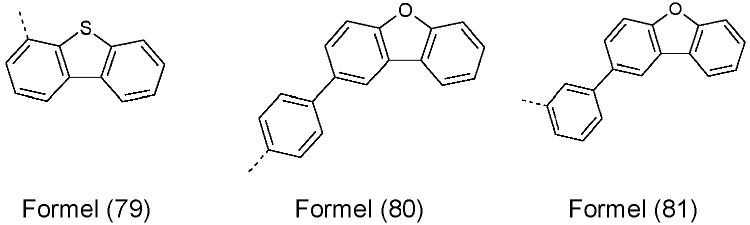 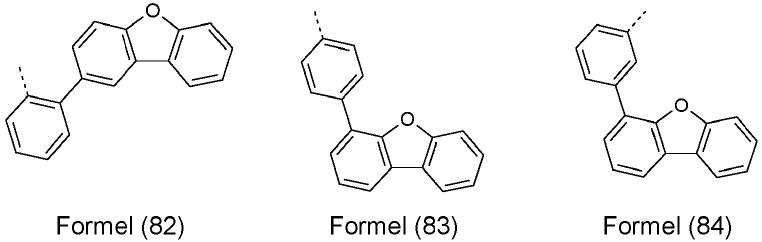 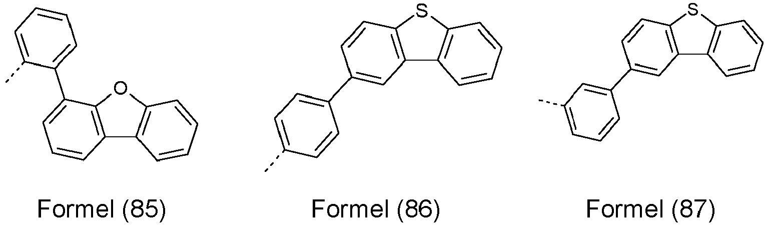 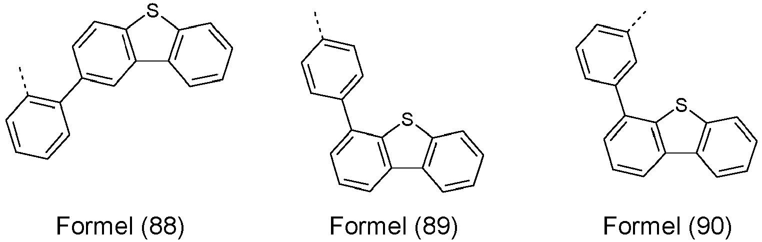 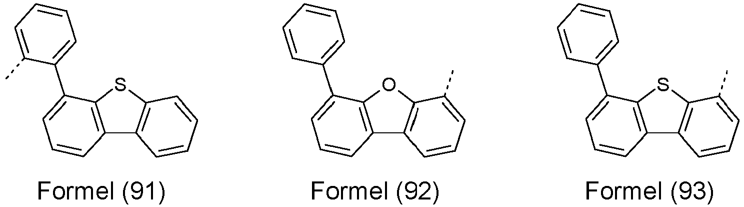 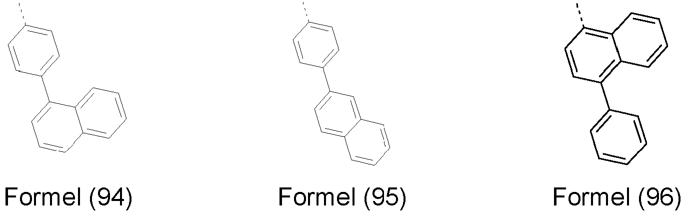 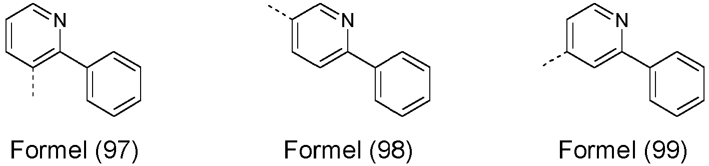 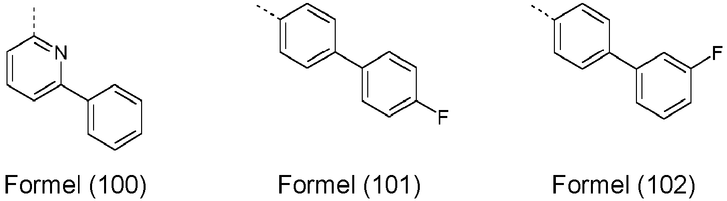 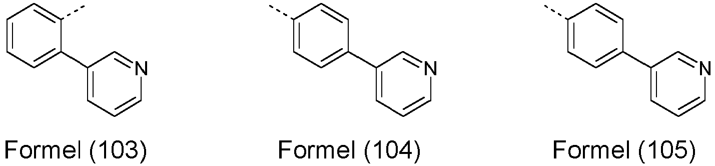 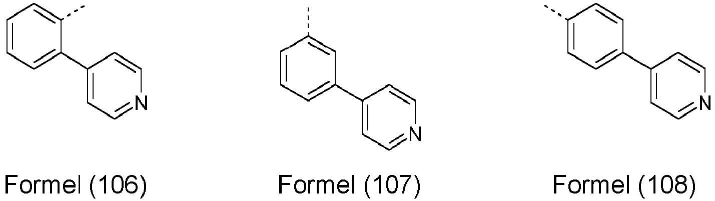 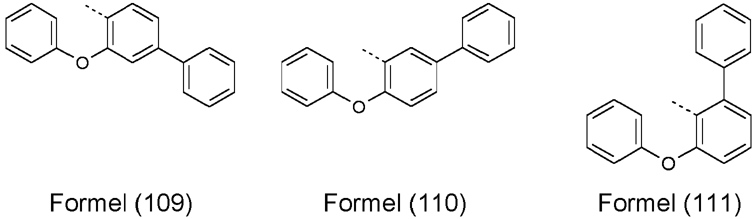 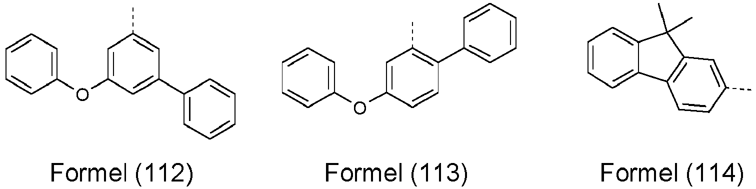 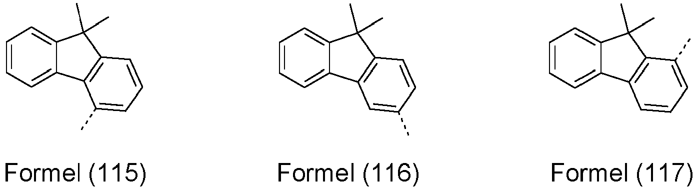 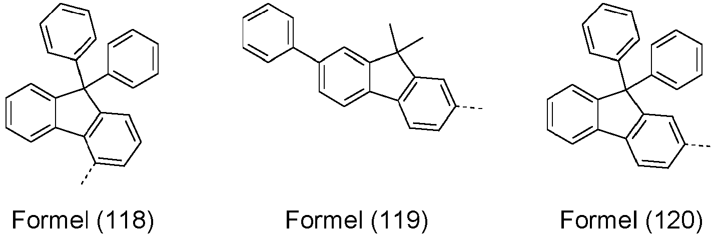 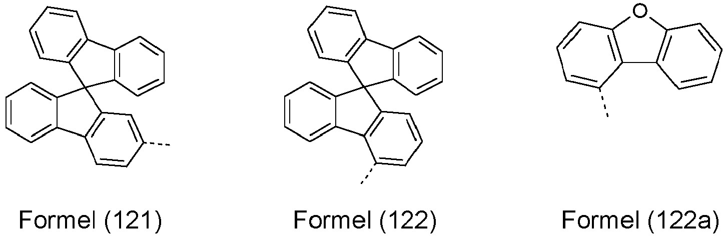 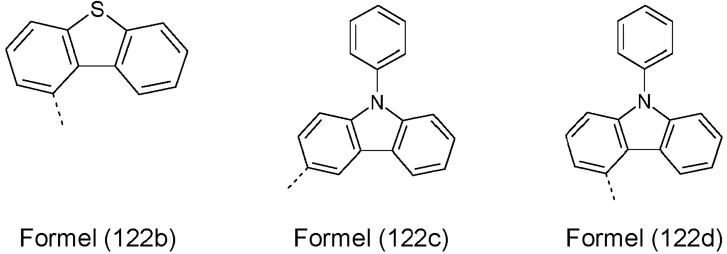 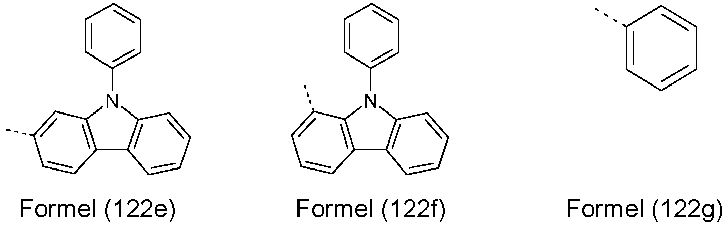 wobei die gestrichelte Linie die Verknüpfungsposition zum Stickstoffatom kennzeichnet.

Die wenigstens eine Fluorengruppe der Gruppen Ar¹ und Ar² ist in einer bevorzugten Ausführungsform der vorliegenden Erfindung direkt an das Stickstoffatom der Gruppen Z^{a}₁, Z^{b}₁ oder Z^{c}₁ gebunden. Ganz bevorzugt ist, wenn die wenigstens eine Fluorengruppe der Gruppen Ar¹ und Ar² über dessen Positionen 1, 2, 3 und 4 an das Stickstoffatom der Gruppen Z^{a}₁, Z^{b}₁ oder Z^{c}₁ gebunden ist, wobei die Bindung über die Position 2 ganz besonders bevorzugt ist.

Die vorliegende Erfindung betrifft in einer bevorzugten Ausführungsform eine Verbindung der allgemeinen Formel (6), wobei für die verwendeten Symbole gilt, dass
- R¹: bei jedem Auftreten gleich oder verschieden, bevorzugt gleich, eine geradkettige Alkylgruppe mit 1 bis 20 C-Atomen oder eine verzweigte oder cyclische Alkylgruppe mit 3 bis 20 C-Atomen ist, wobei die oben genannten Gruppen jeweils mit einem oder mehreren Resten R⁴ substituiert sein können oder ein aromatisches oder heteroaromatisches Ringsystem mit 6 bis 30 aromatischen Ringatomen, das jeweils durch einen oder mehrere Reste R⁴ substituiert sein kann, wobei die beiden Reste R¹ miteinander verknüpft sein können und einen Ring bilden können, so dass eine Spiroverbindung in Position 9 des Fluorens entsteht, wobei Spirobifluorene ausgeschlossen sind;
- R²: gleich H, eine geradkettigen Alkylgruppe mit 1 bis 20 C-Atomen oder einer verzweigten oder cyclischen Alkylgruppe mit 3 bis 20 C-Atomen ist, wobei die Gruppen durch einen oder mehrere Reste R⁴ substituiert sein können, ein aromatisches oder heteroaromatisches Ringsystem mit 6 bis 30 aromatischen Ringatomen, das durch ein oder mehrere Reste R⁴ substituiert sein kann;
- R³: gleich H, D, F, Cl, Br, I, eine geradkettige Alkyl- oder Alkoxygruppe mit 1 bis 20 C-Atomen oder eine verzweigte oder cyclische Alkyl- oder Alkoxygruppe mit 3 bis 20 C-Atomen ist, wobei die oben genannten Gruppen jeweils mit einem oder mehreren Resten R⁴ substituiert sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 6 bis 30 aromatischen Ringatomen, das jeweils durch einen oder mehrere Reste R⁴ substituiert sein kann, oder eine Aryloxygruppe mit 5 bis 60 aromatischen Ringatomen, die mit einem oder mehreren Resten R⁴ substituiert sein kann, oder einer Aralkylgruppe mit 5 bis 60 aromatischen Ringatomen, die jeweils mit einem oder mehreren Resten R⁴ substituiert sein kann, wobei zwei oder mehr Reste R³ miteinander verknüpft sein können und einen Ring bilden können;
- B': ausgewählt ist aus den Gruppen der Formeln (15) bis (36), bevorzugt (15) bis (17), wobei diese Gruppen noch durch ein oder mehrere voneinander unabhängige Reste R⁴ substituiert sein können und wobei R⁴ wie oben angegeben definiert ist;
- Ar¹, Ar²: eine Phenyl-, Phenyl-Pyridyl-, Phenyl-Naphthyl-, Biphenyl-, Terphenyl- oder Quarterphenylgruppe sind, die mit einem oder mehreren Resten R⁶, die gleich oder verschieden voneinander sein können, substituiert sein können, wobei zwei der aromatischen oder heteroaromatischen Ringe in Ar¹ durch eine bivalente Gruppe -O-, -S--, C(R⁶)₂ oder -Si(R⁶)₂- zusätzlich verbrückt sein können bzw. zwei der aromatischen oder heteroaromatischen Ringe in Ar² durch eine bivalente Gruppe -O-, -S--, C(R⁶)₂ oder -Si(R⁶)₂- zusätzlich verbrückt sein können, wobei bevorzugt ist, wenn keine zusätzliche Verbrückung vorliegt, und wobei ein aromatischer oder heteroaromatischer Ring aus Ar¹ mit einem aromatischen oder heteroaromatischen Ring aus Ar² durch eine bivalente Gruppe -O-, -S-, -Si(R⁶)₂-, -NR⁶- oder-C(R⁶)₂- verbrückt sein kann, wobei unverbrückte Gruppen Ar¹ und Ar² bevorzugt sind und wobei wenigstens eine der beiden Gruppen Ar¹ und Ar² eine Fluorengruppe enthält.

Die vorliegende Erfindung betrifft in einer ganz bevorzugten Ausführungsform eine Verbindung der allgemeinen Formel (6), wobei für die verwendeten Symbole gilt, dass
- R¹: bei jedem Auftreten gleich oder verschieden, bevorzugt gleich, eine geradkettige Alkylgruppe mit 1 bis 20 C-Atomen ist, wobei die genannte Gruppe mit einem oder mehreren Resten R⁴ substituiert sein kann oder ein aromatisches oder heteroaromatisches Ringsystem mit 6 bis 30 aromatischen Ringatomen, das jeweils durch einen oder mehrere Reste R⁴ substituiert sein kann, wobei die beiden Reste R¹ miteinander verknüpft sein können und einen Ring bilden können, so dass eine Spiroverbindung in Position 9 des Fluorens entsteht, wobei Spirobifluorene ausgeschlossen sind;
- R²: gleich H, eine geradkettigen Alkylgruppe mit 1 bis 20 C-Atomen oder einer verzweigten oder cyclischen Alkylgruppe mit 3 bis 20 C-Atomen ist, wobei die Gruppen durch einen oder mehrere Reste R⁴ substituiert sein können, ein aromatisches oder heteroaromatisches Ringsystem mit 6 bis 30 aromatischen Ringatomen, das durch ein oder mehrere Reste R⁴ substituiert sein kann;
- R³: gleich H oder ein aromatisches oder heteroaromatisches Ringsystem mit 6 bis 30 aromatischen Ringatomen, das jeweils durch einen oder mehrere Reste R⁴ substituiert sein kann, wobei R³ gleich H insbesondere bevorzugt ist;
- B': eine Phenylengruppe ist;
- Ar¹, Ar²: eine Phenyl-, Biphenyl-, Terphenyl- oder Quarterphenylgruppe sind, die mit einem oder mehreren Resten R⁶, die gleich oder verschieden voneinander sein können, substituiert sein können, wobei die Ringe in Ar¹ und Ar² unverbrückt sind, insbesondere bevorzugt sind Ar¹ und Ar² eine Biphenylgruppe, die mit einem oder mehreren Resten R⁶, die gleich oder verschieden voneinander sein können, substituiert sein kann und wobei wenigstens eine der beiden Gruppen Ar¹ und Ar² eine Fluorengruppe enthält.

Die vorliegende Erfindung betrifft in einer ganz besonders bevorzugten Ausführungsform eine Verbindung der allgemeinen Formel (6), wobei für die verwendeten Symbole gilt, dass
- R¹: bei jedem Auftreten gleich ist und eine geradkettige Alkylgruppe mit 1 bis 5 C-Atomen, bevorzugt eine Methylgruppe oder Ethylgruppe, ist, wobei die genannte Gruppe mit einem oder mehreren Resten R⁴ substituiert sein kann oder eine Phenyl-, Biphenyl, Pyridylgruppe, die jeweils durch einen oder mehrere Reste R⁴ substituiert sein kann, darstellt, wobei die beiden Alkylgruppen nach R¹ miteinander verknüpft sein können und einen Ring bilden können, so dass eine Spiroverbindung in Position 9 des Fluorens entsteht, wobei Spirobifluorene ausgeschlossen sind;
- R²: gleich H, eine geradkettigen Alkylgruppe mit 1 bis 20 C-Atomen oder einer verzweigten oder cyclischen Alkylgruppe mit 3 bis 20 C-Atomen ist, wobei die Gruppen durch einen oder mehrere Reste R⁴ substituiert sein können, ein aromatisches oder heteroaromatisches Ringsystem mit 6 bis 30 aromatischen Ringatomen, das durch ein oder mehrere Reste R⁴ substituiert sein kann;
- R³: gleich H oder ein aromatisches oder heteroaromatisches Ringsystem mit 6 bis 30 aromatischen Ringatomen, das jeweils durch einen oder mehrere Reste R⁴ substituiert sein kann, wobei R³ gleich H insbesondere bevorzugt ist;
- B': eine Phenylengruppe ist;
- Ar¹, Ar²: eine Phenyl-, Biphenyl-, Terphenyl- oder Quarterphenylgruppe sind, die mit einem oder mehreren Resten R⁶, die gleich oder verschieden voneinander sein können, substituiert sein können, wobei die Ringe in Ar¹ und Ar² unverbrückt sind, insbesondere bevorzugt sind Ar¹ und Ar² eine Biphenylgruppe, die mit einem oder mehreren Resten R⁶, die gleich oder verschieden voneinander sein können, substituiert sein kann und wobei wenigstens eine der beiden Gruppen Ar¹ und Ar² eine Fluorengruppe enthält.

Besonders bevorzugt ist folglich eine Verbindung der allgemeinen Formel (123) 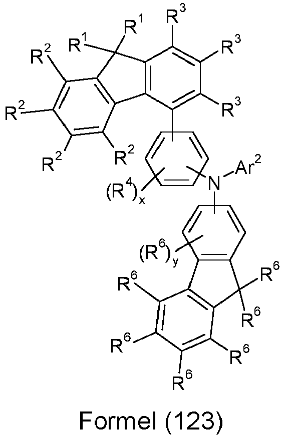 wobei x gleich 0, 1, 2, 3 oder 4 ist und wobei y gleich 0, 1, 2 oder 3 ist.

Weiterhin bevorzugt ist auch eine Verbindung der allgemeinen Formel (124)

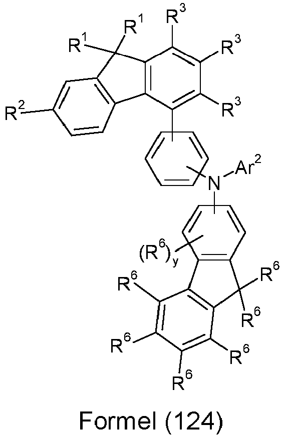

Noch bevorzugter ist eine Verbindung der allgemeinen Formel (125)

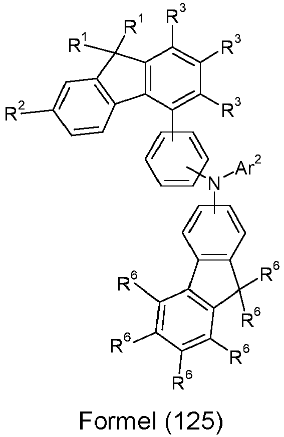

Noch mehr bevorzugt ist eine Verbindung der allgemeinen Formel (126)

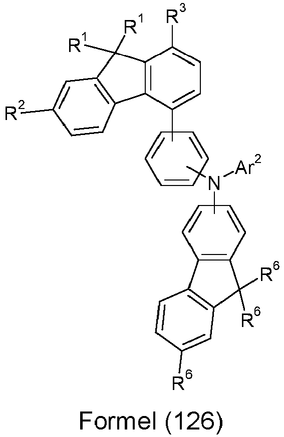

Und noch mehr bevorzugt ist eine Verbindung der allgemeinen Formel (127)

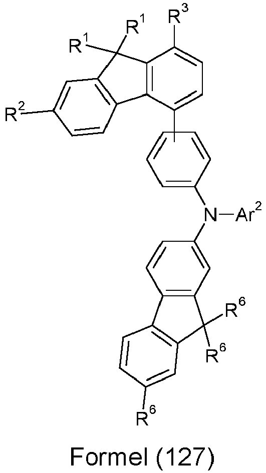

Schließlich stellt die Verbindung der folgenden Formel (128) eine insbesonders bevorzugte Ausführungsform der vorliegenden Erfindung dar

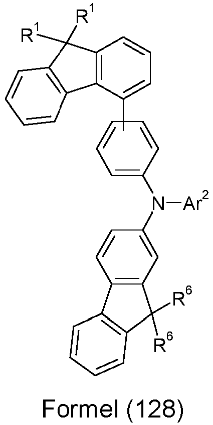

Die vorliegende Erfindung betrifft in einer weiteren bevorzugten Ausführungsform eine Verbindung der allgemeinen Formel (8), wobei für die verwendeten Symbole gilt, dass
- R¹: bei jedem Auftreten gleich oder verschieden, bevorzugt gleich, eine geradkettige Alkylgruppe mit 1 bis 20 C-Atomen oder eine verzweigte oder cyclische Alkylgruppe mit 3 bis 20 C-Atomen ist, wobei die oben genannten Gruppen jeweils mit einem oder mehreren Resten R⁴ substituiert sein können oder ein aromatisches oder heteroaromatisches Ringsystem mit 6 bis 30 aromatischen Ringatomen, das jeweils durch einen oder mehrere Reste R⁴ substituiert sein kann, wobei die beiden Reste R¹ miteinander verknüpft sein können und einen Ring bilden können, so dass eine Spiroverbindung in Position 9 des Fluorens entsteht, wobei Spirobifluorene ausgeschlossen sind;
- R²: gleich H, eine geradkettigen Alkylgruppe mit 1 bis 20 C-Atomen oder einer verzweigten oder cyclischen Alkylgruppe mit 3 bis 20 C-Atomen ist, wobei die Gruppen durch einen oder mehrere Reste R⁴ substituiert sein können, ein aromatisches oder heteroaromatisches Ringsystem mit 6 bis 30 aromatischen Ringatomen, das durch ein oder mehrere Reste R⁴ substituiert sein kann;
- R³: gleich H, D, F, Cl, Br, I, eine geradkettige Alkyl- oder Alkoxygruppe mit 1 bis 20 C-Atomen oder eine verzweigte oder cyclische Alkyl- oder Alkoxygruppe mit 3 bis 20 C-Atomen ist, wobei die oben genannten Gruppen jeweils mit einem oder mehreren Resten R⁴ substituiert sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 6 bis 30 aromatischen Ringatomen, das jeweils durch einen oder mehrere Reste R⁴ substituiert sein kann, oder eine Aryloxygruppe mit 5 bis 60 aromatischen Ringatomen, die mit einem oder mehreren Resten R⁴ substituiert sein kann, oder einer Aralkylgruppe mit 5 bis 60 aromatischen Ringatomen, die jeweils mit einem oder mehreren Resten R⁴ substituiert sein kann, wobei zwei oder mehr Reste R³ miteinander verknüpft sein können und einen Ring bilden können;
- B': ausgewählt ist aus den Gruppen der Formeln (15) bis (36), bevorzugt (15) bis (17), wobei diese Gruppen noch durch ein oder mehrere voneinander unabhängige Reste R⁴ substituiert sein können und wobei R⁴ wie oben angegeben definiert ist;
- Ar¹, Ar²: eine Phenyl-, Phenyl-Pyridyl-, Phenyl-Naphthyl-, Biphenyl-, Terphenyl- oder Quarterphenylgruppe sind, die mit einem oder mehreren Resten R⁶, die gleich oder verschieden voneinander sein können, substituiert sein können, wobei zwei aromatische oder heteroaromatische Ringe in Ar¹ durch eine bivalente Gruppe -O-, -S--, C(R⁶)₂ oder -Si(R⁶)₂- zusätzlich verbrückt sein können bzw. zwei der aromatischen oder heteroaromatischen Ringe in Ar² durch eine bivalente Gruppe -O-, -S- -, C(R⁶)₂ oder -Si(R⁶)₂- zusätzlich verbrückt sein können, wobei bevorzugt ist, wenn keine zusätzliche Verbrückung vorliegt, und wobei ein aromatischer oder heteroaromatischer Ring aus Ar¹ mit einem aromatischen oder heteroaromatischen Ring aus Ar² durch eine bivalente Gruppe -O-,-S-, -Si(R⁶)₂-, -NR⁶- oder -C(R⁶)₂- verbrückt sein kann, wobei unverbrückte Gruppen Ar¹ und Ar² bevorzugt sind und wobei wenigstens eine der beiden Gruppen Ar¹ und Ar² eine Fluorengruppe enthält.

Die vorliegende Erfindung betrifft in einer ganz bevorzugten Ausführungsform eine Verbindung der allgemeinen Formel (8), wobei für die verwendeten Symbole gilt, dass
- R¹: bei jedem Auftreten gleich oder verschieden, bevorzugt gleich, eine geradkettige Alkylgruppe mit 1 bis 20 C-Atomen ist, wobei die genannte Gruppe mit einem oder mehreren Resten R⁴ substituiert sein kann oder ein aromatisches oder heteroaromatisches Ringsystem mit 6 bis 30 aromatischen Ringatomen, das jeweils durch einen oder mehrere Reste R⁴ substituiert sein kann, wobei die beiden Reste R¹ miteinander verknüpft sein können und einen Ring bilden können, so dass eine Spiroverbindung in Position 9 des Fluorens entsteht, wobei Spirobifluorene ausgeschlossen sind;
- R²: gleich H, eine geradkettigen Alkylgruppe mit 1 bis 20 C-Atomen oder einer verzweigten oder cyclischen Alkylgruppe mit 3 bis 20 C-Atomen ist, wobei die Gruppen durch einen oder mehrere Reste R⁴ substituiert sein können, ein aromatisches oder heteroaromatisches Ringsystem mit 6 bis 30 aromatischen Ringatomen, das durch ein oder mehrere Reste R⁴ substituiert sein kann;
- R³: gleich H oder ein aromatisches oder heteroaromatisches Ringsystem mit 6 bis 30 aromatischen Ringatomen, das jeweils durch einen oder mehrere Reste R⁴ substituiert sein kann, wobei R³ gleich H insbesondere bevorzugt ist;
- B': eine Phenylengruppe ist;
- Ar¹, Ar²: eine Phenyl-, Biphenyl-, Terphenyl- oder Quarterphenylgruppe sind, die mit einem oder mehreren Resten R⁶, die gleich oder verschieden voneinander sein können, substituiert sein können, wobei die Ringe in Ar¹ und Ar² nicht zusätzlich verbrückt sind, insbesondere bevorzugt sind Ar¹ und Ar² eine Biphenylgruppe, die mit einem oder mehreren Resten R⁶, die gleich oder verschieden voneinander sein können, substituiert sein kann und wobei wenigstens eine der beiden Gruppen Ar¹ und Ar² eine Fluorengruppe enthält.

Die vorliegende Erfindung betrifft in einer ganz besonders bevorzugten Ausführungsform eine Verbindung der allgemeinen Formel (8), wobei für die verwendeten Symbole gilt, dass
- R¹: bei jedem Auftreten gleich ist und eine geradkettige Alkylgruppe mit 1 bis 5 C-Atomen, bevorzugt eine Methylgruppe oder Ethylgruppe, ist, wobei die genannte Gruppe mit einem oder mehreren Resten R⁴ substituiert sein kann oder eine Phenyl-, Biphenyl, Pyridylgruppe, die jeweils durch einen oder mehrere Reste R⁴ substituiert sein kann, darstellt, wobei die beiden Alkylgruppen nach R¹ miteinander verknüpft sein können und einen Ring bilden können, so dass eine Spiroverbindung in Position 9 des Fluorens entsteht, wobei Spirobifluorene ausgeschlossen sind;
- R²: gleich H, eine geradkettigen Alkylgruppe mit 1 bis 20 C-Atomen oder einer verzweigten oder cyclischen Alkylgruppe mit 3 bis 20 C-Atomen ist, wobei die Gruppen durch einen oder mehrere Reste R⁴ substituiert sein können, ein aromatisches oder heteroaromatisches Ringsystem mit 6 bis 30 aromatischen Ringatomen, das durch ein oder mehrere Reste R⁴ substituiert sein kann;
- R³: gleich H oder ein aromatisches oder heteroaromatisches Ringsystem mit 6 bis 30 aromatischen Ringatomen, das jeweils durch einen oder mehrere Reste R⁴ substituiert sein kann, wobei R³ gleich H insbesondere bevorzugt ist;
- B': eine Phenylengruppe ist;
- Ar¹, Ar²: eine Phenyl-, Biphenyl-, Terphenyl- oder Quarterphenylgruppe sind, die mit einem oder mehreren Resten R⁶, die gleich oder verschieden voneinander sein können, substituiert sein können, wobei die Ringe in Ar¹ und Ar² nicht zusätzlich verbrückt sind, insbesondere bevorzugt sind Ar¹ und Ar² eine Biphenylgruppe, die mit einem oder mehreren Resten R⁶, die gleich oder verschieden voneinander sein können, substituiert sein kann und wobei wenigstens eine der beiden Gruppen Ar¹ und Ar² eine Fluorengruppe enthält.

In einer insbesonders bevorzugten Ausführungsform betrifft die vorliegende Erfindung Verbindungen der allgemeinen Formel (1) dadurch charakterisiert, dass es sich um Monoaminverbindungen handelt.

Besonders bevorzugt ist folglich eine Verbindung der allgemeinen Formel (129) 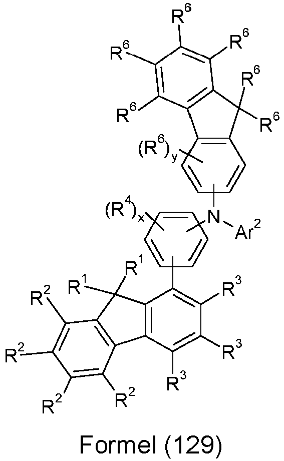 wobei x gleich 0, 1, 2, 3 oder 4 ist und wobei y gleich 0, 1, 2 oder 3 ist.

Weiterhin bevorzugt ist auch eine Verbindung der allgemeinen Formel (130)

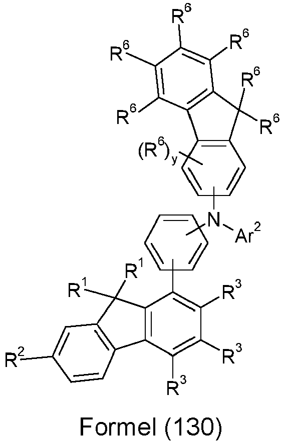

Noch bevorzugter ist eine Verbindung der allgemeinen Formel (131)

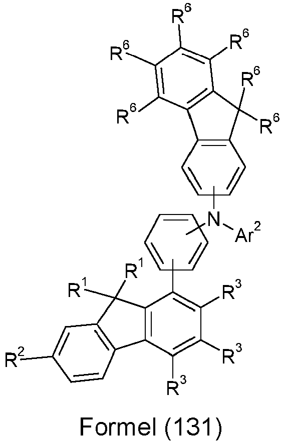

Noch mehr bevorzugt ist eine Verbindung der allgemeinen Formel (132)

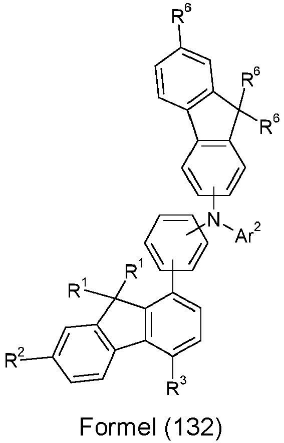

Und noch mehr bevorzugt ist eine Verbindung der allgemeinen Formel (133)

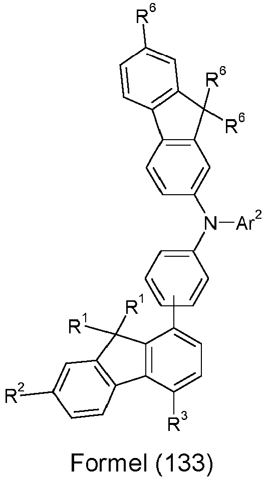

Schließlich stellt die Verbindung der folgenden Formel (134) eine insbesonders bevorzugte Ausführungsform der vorliegenden Erfindung dar

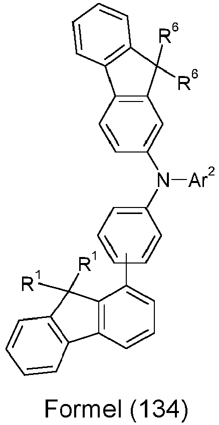

Das folgende Reaktionsschema zeigt einen bevorzugten Syntheseweg zur Herstellung der erfindungsgemäßen Verbindungen der Formel (1). Zur Synthese der erfindungsgemäßen Verbindungen wird die Fluoren-Verbindung A zuerst durch dem Fachmann geläufigen Methoden (wie zum Beispiel Boronierung und Suzuki-Kupplung) in eine Verbindung B umgewandelt und nach einer Buchwald-Kupplung mit einem Amin C der Formel Ar¹-NH-Ar² weiter zu der erfindungsgemäßen Verbindung umgesetzt. 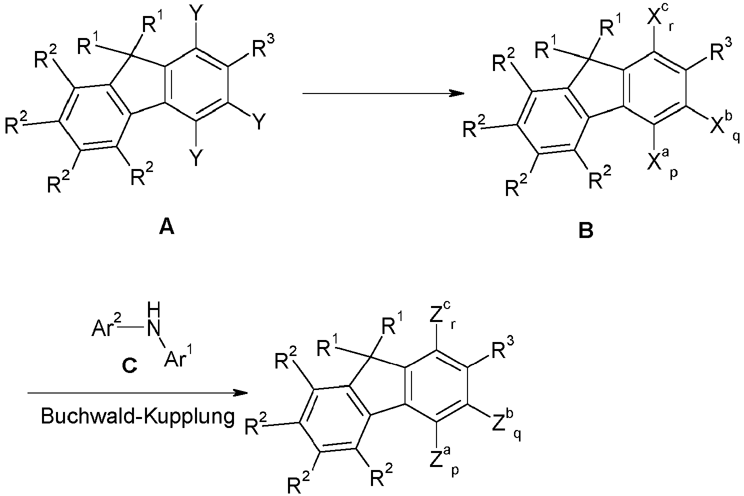 wobei für die verwendeten Symbole und Indices obige Definitionen gelten und wobei
Y eine Abgangsgruppe, vorzugsweise Halogen, ist;
X^{a}₀, X^{b}₀, X^{c}₀ gleich oder verschieden bei jedem Auftreten gleich R³ und
X^{a}₁, X^{b}₁, X^{c}₁ sind gleich -B'-Y, wobei Y eine Abgangsgruppe, beispielsweise Halogen, sind.

Ein anderer bevorzugter Syntheseweg zur Herstellung der erfindungsgemäßen Verbindungen wird im folgenden Reaktionsschema dargestellt. Der Syntheseweg umfasst zwei Kupplungsreaktionen: zunächst wird das das Fluoren-Verbindung B in einer ersten Buchwald-Kupplung mit einem Amin D der Formel Ar¹-NH₂ umgesetzt. Schließlich erfolgt eine zweite Buchwald-Kupplung mit einer Verbindung F, beispielsweise mit einer Bromaryl-Verbindung. 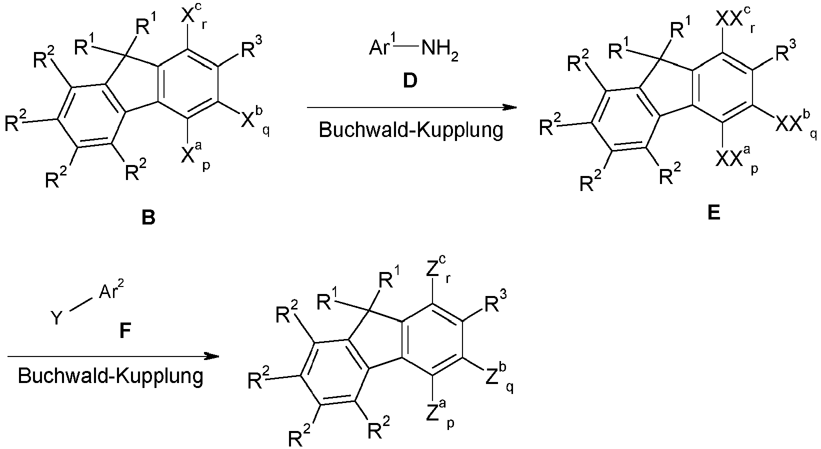
wobei Y wieder eine Abgangsgruppe, vorzugsweise Halogen, ist;
und wobei
XX^{a}₀, XX^{b}₀, X X^{c}₀ gleich oder verschieden bei jedem Auftreten gleich R³ und XX^{a}₁, XX^{b}₁, XX^{c}₁ gleich -B'-NH-Ar¹ sind.

Ein anderer bevorzugter Syntheseweg zur Herstellung der erfindungsgemäßen Verbindung wird im folgenden Reaktionsschema dargestellt. Hierzu wird die Fluoren-Verbindung A nach einer dem Fachmann geläufigen C-C Kupplungsmethoden (wie zum Beispiel Suzuki-Kupplung) zu der erfindungsgemäßen Verbindung umgesetzt

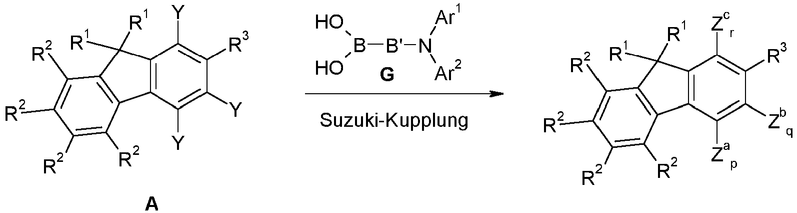

Das folgende Schema zeigt einen weiteren bevorzugten Syntheseweg zur Herstellung der erfindungsgemäßen Verbindung. Hierzu dienen Benzochromenone H als Ausgangspunkt. Die Addition von einem Organometallo-Reagenz beispielweise Grignard- oder Organolithium-Reagenz und anschließende säurekatalysierte Cyclisierung des intermediären Alkoholats führt zu dem entsprechenden 4-Hydroxy-Fluoren I. Anschließend wird die Hydroxygruppe in eine Abgangsgruppe Y bzw. -B'-Y (=X^{a}₁), beispielweise in ein Triflat (TfO) oder ein Halogenid (bevorzugt Br oder Cl), umgewandelt und nach einer dem Fachmann geläufigen Methode (C-C-Kuplung wie Zusuki-, Negishi-, Yamamoto-, Grignard-Cross-, Stille-, Heck-Kupplung, etc.; C-N-Kupplung wie Buchwald- Kupplung) weiter zu der erfindungsgemäßen Verbindung umgesetzt, wobei eine Buchwald Kupplung oder eine Suzuki Kupplung bevorzugt sind. Im Fall von X^{a}₁ ist selbstverständlich lediglich Y die Abgangsgruppe. 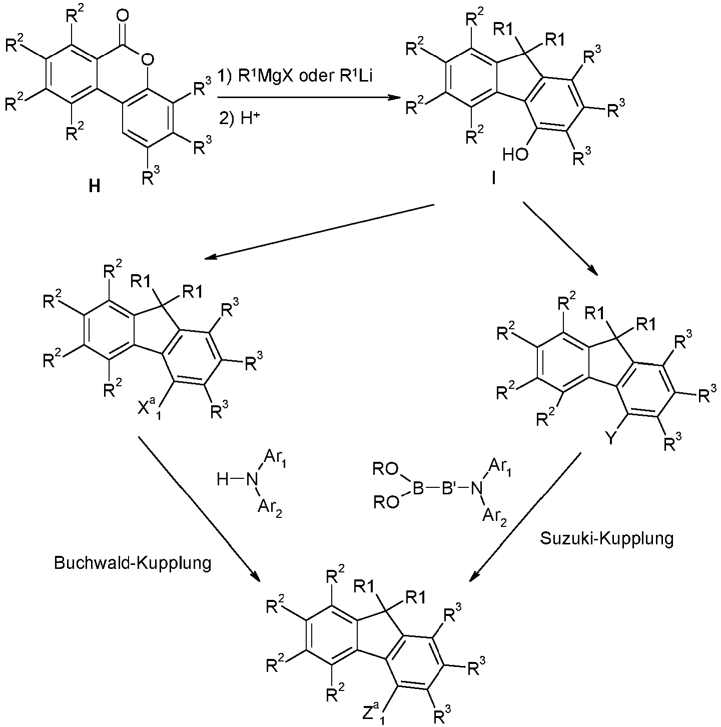 wobei die verwendeten Indizes wie oben angegeben definiert sind und B für ein Boratom steht.

Hiedurch lassen sich Fluorene darstellen, die das Amin in der bevorzugten Position 4 aufweisen.

Ganz analog hierzu können erfindungsgemäße Fluorene dargestellt werden, die das Amin in Position 1 des Fluorens haben.

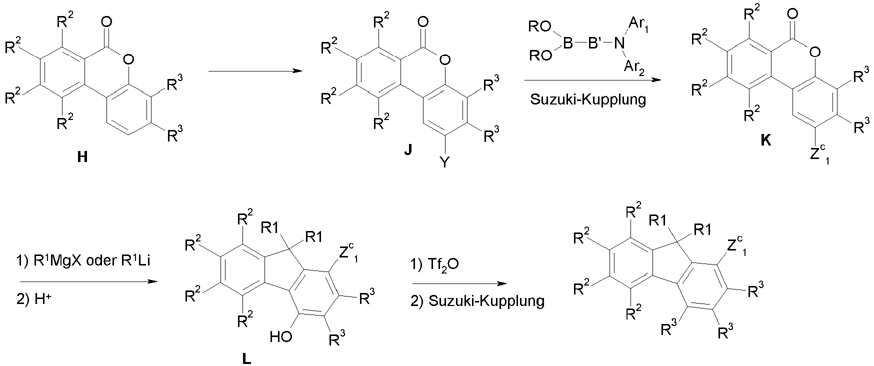

Die für die Suzuki-Kupplung eingesetzten Boronsäureester mit den Gruppen R sind dem Fachmann gut bekannt.

Synthesewege für die Ausgangs- und Zwischenverbindungen A, B, C, D, E, F, G, H, I, J, K und L. welche in der Synthese der erfindungsgemäßen Verbindungen eingesetzt werden, sind dem Fachmann geläufig. Weiterhin werden in den Ausführungsbeispielen einige explizite Syntheseverfahren im Detail dargestellt.

Bevorzugte Kupplungsreaktionen zur Herstellung der Verbindung der allgemeinen Formel (1) sind Buchwald-Kupplungen und Suzuki-Kupplungen.

Bevorzugte erfindungsgemäße Verbindungen sind in der nachfolgenden Tabelle exemplarisch aufgeführt.

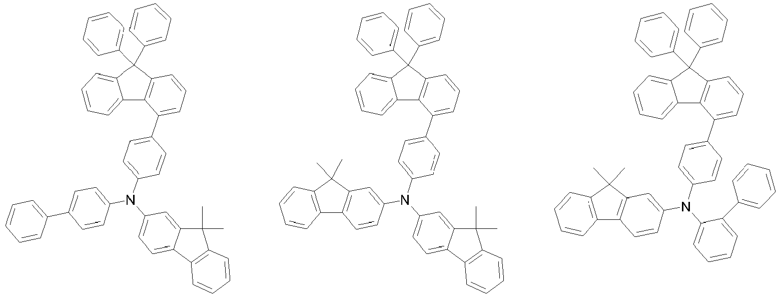

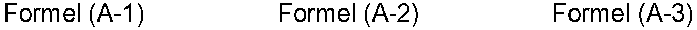

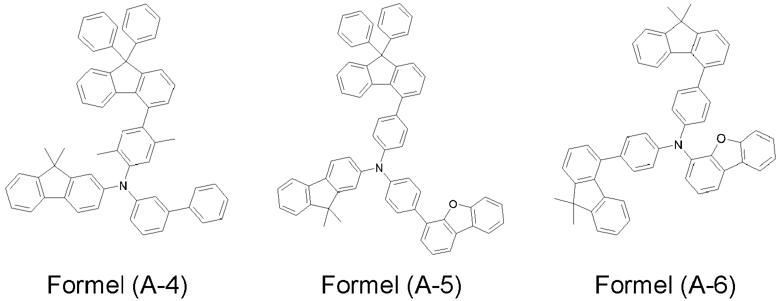

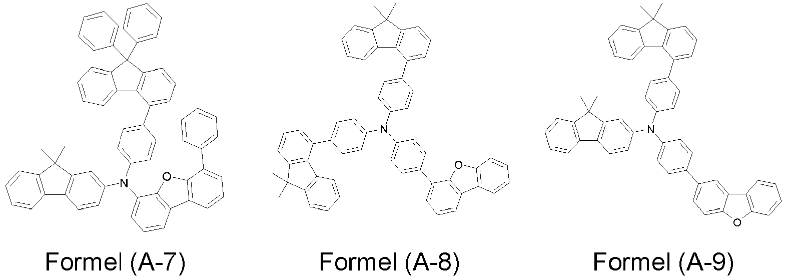

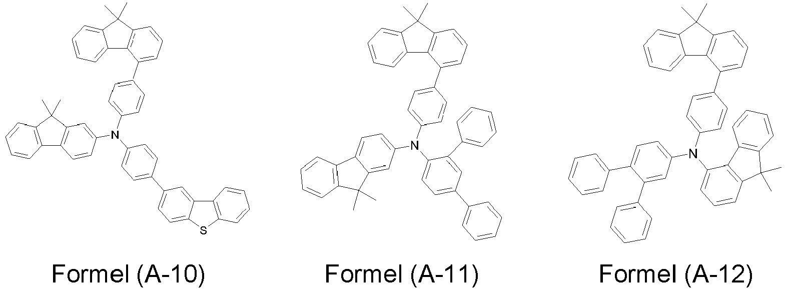

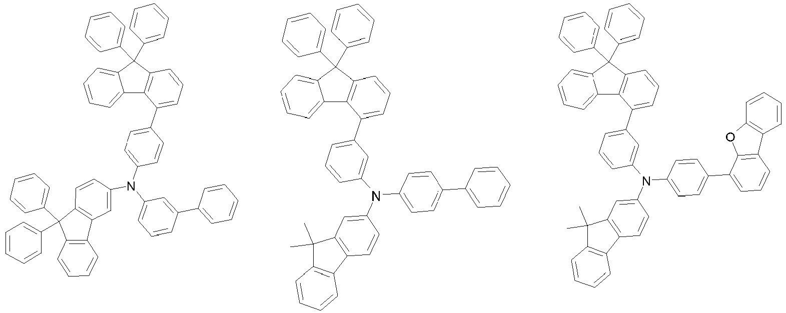

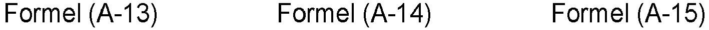

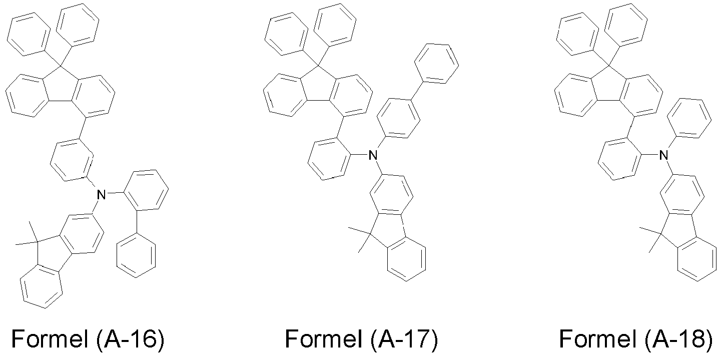

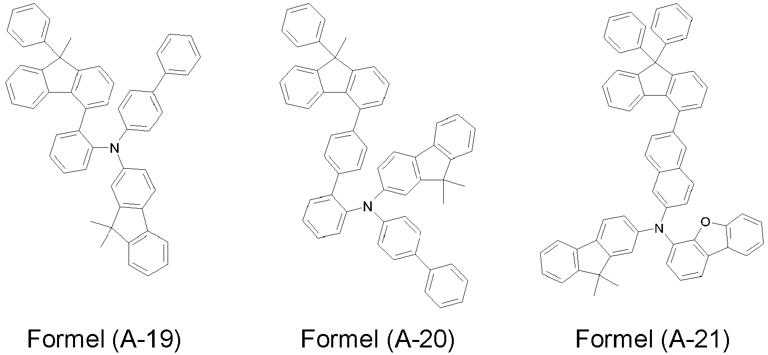

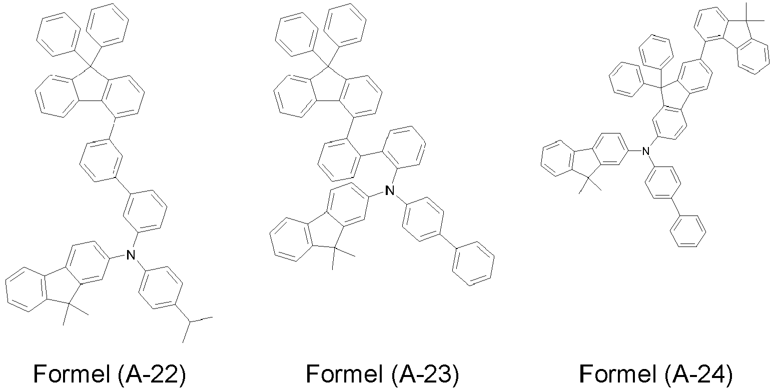

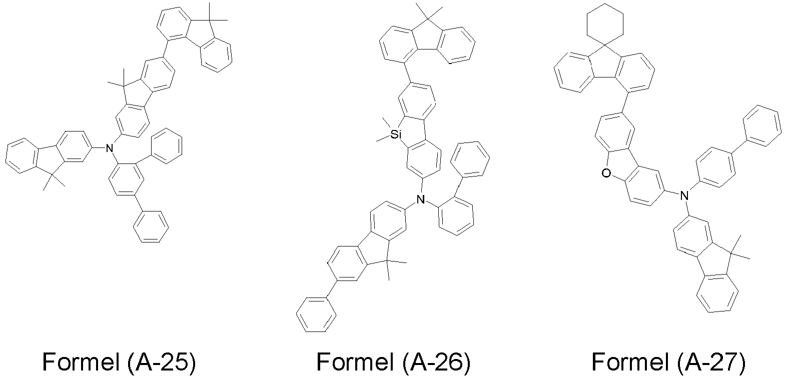

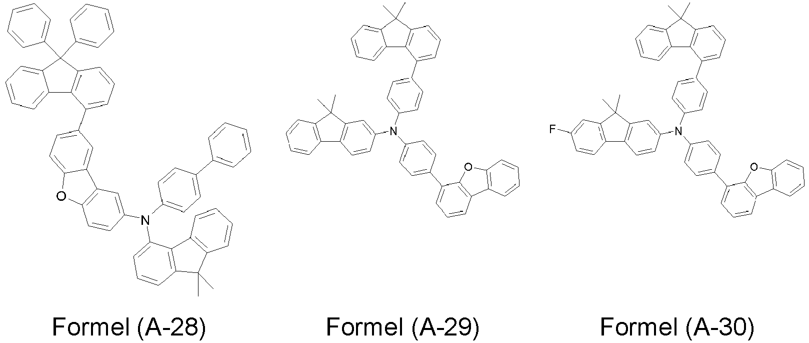

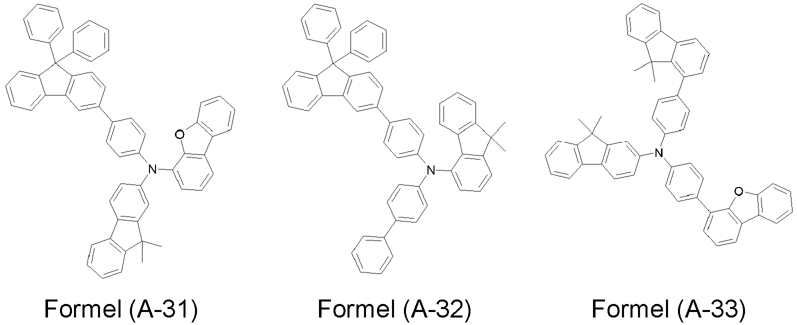

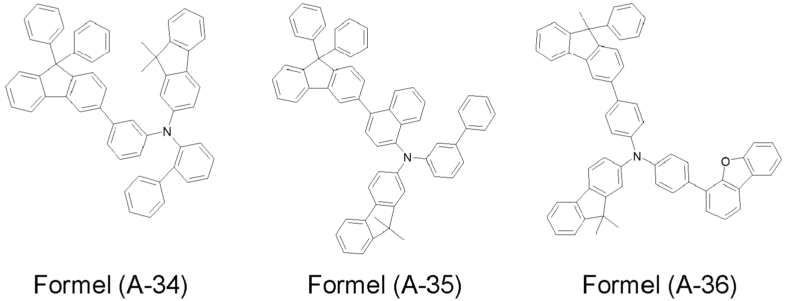

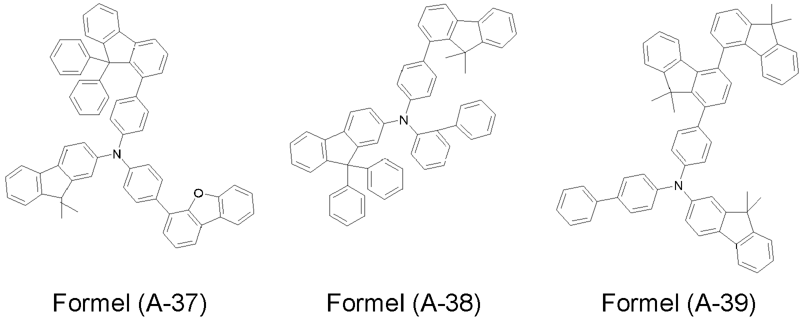

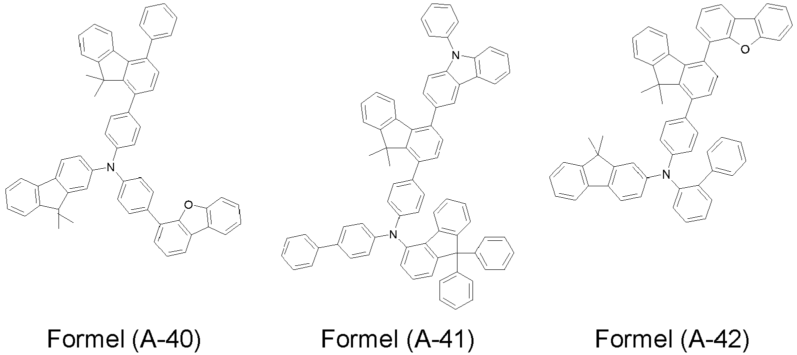

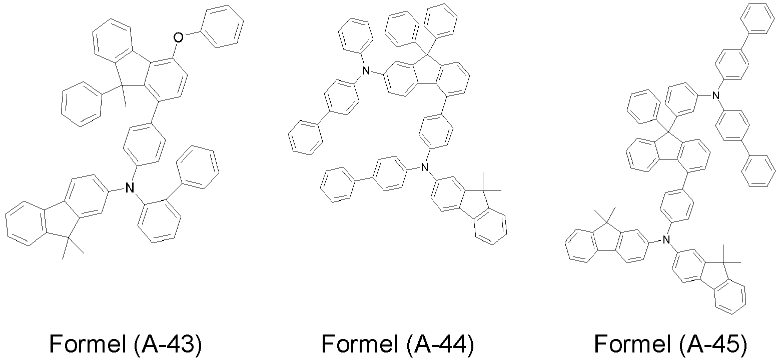

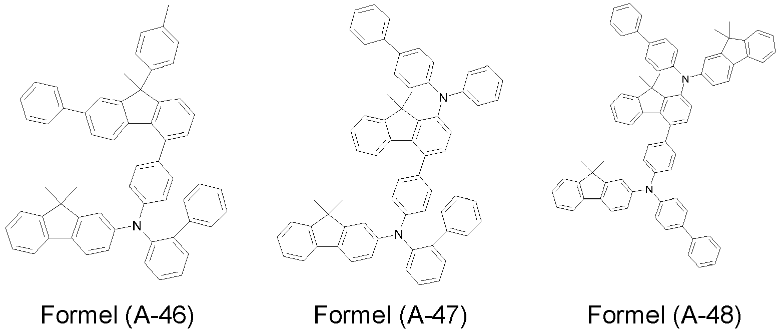

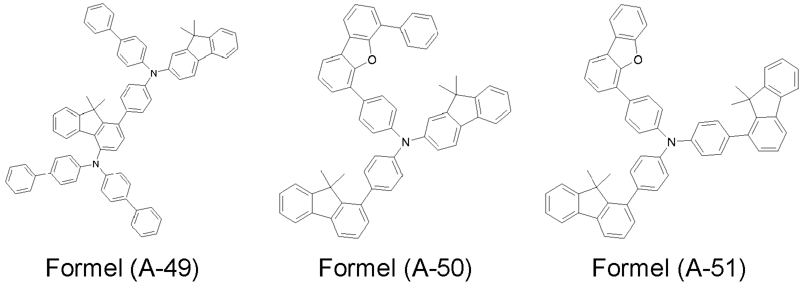

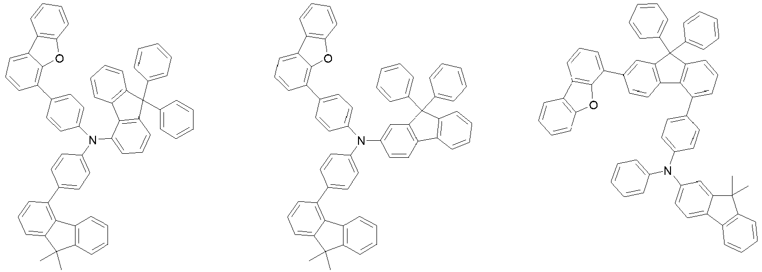

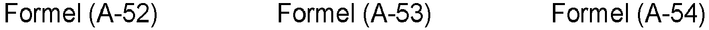

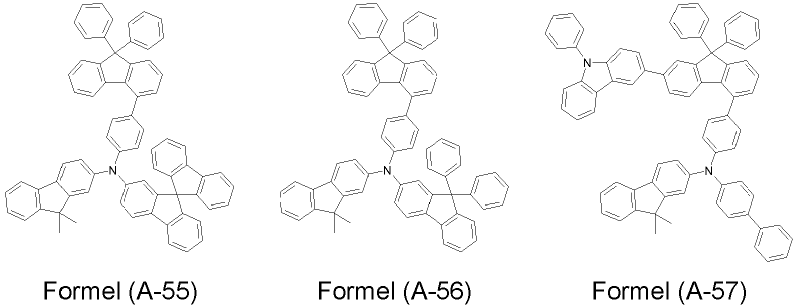

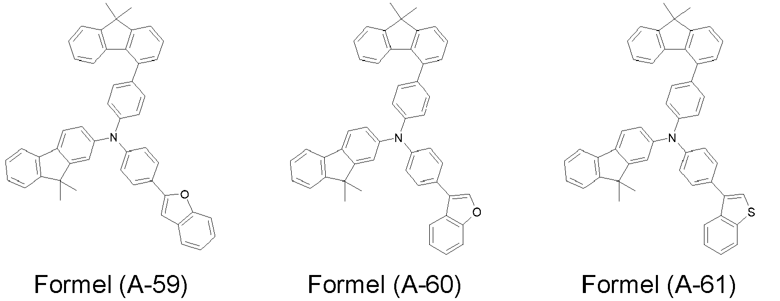

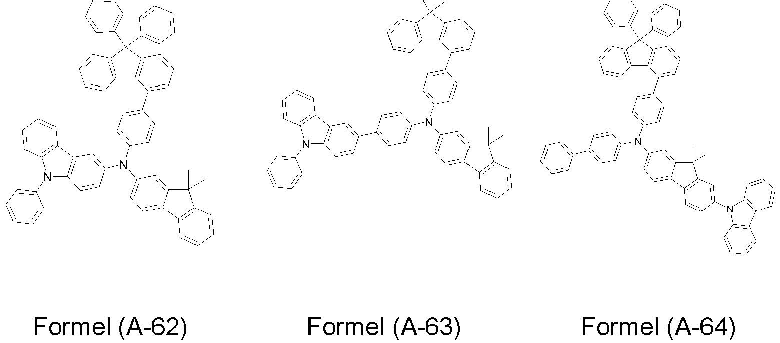

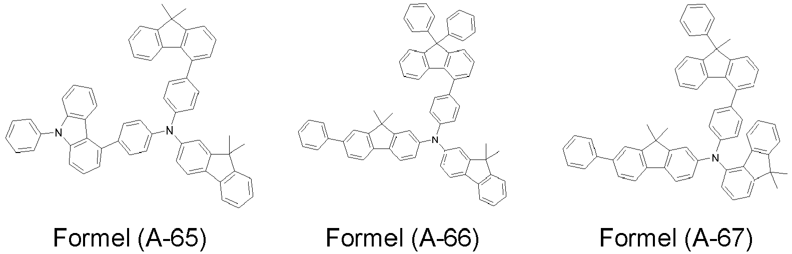

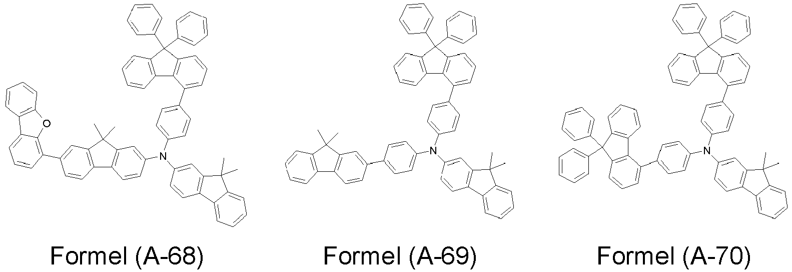

Die oben beschriebenen Verbindungen der Formel (1) können mit reaktiven Abgangsgruppen, wie Brom, Iod, Chlor, Boronsäure oder Boronsäureester substituiert werden. Diese können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere enthaltend eine oder mehrere Verbindungen gemäß Formel (1), wobei die Bindung(en) zum Polymer, Oligomer oder Dendrimer an beliebigen in Formel (1) möglichen Positionen lokalisiert sein können. Je nach Verknüpfung der Verbindung gemäß Formel (1) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten gemäß Formel (1) ddirekt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formeln (1) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (1) in Oligomeren, Dendrimeren und Polymeren gelten dieselben Bevorzugungen wie oben für Verbindungen gemäß Formel (1) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (1) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Gegenstand der vorliegenden Erfindung ist somit auch ein Verfahren zur Herstellung der erfindungsgemäßen Polymere, Oligomere und Dendrimere, das dadurch gekennzeichnet ist, dass sie durch Polymerisation gemäß SUZUKI, Polymerisation gemäß YAMAMOTO, Polymerisation gemäß STILLE oder Polymerisation gemäß HARTWIG-BUCHWALD hergestellt werden. Die erfindungsgemäßen Dendrimere können gemäß dem Fachmann bekannten Verfahren oder in Analogie dazu hergestellt werden. Geeignete Verfahren sind in der Literatur beschrieben, wie z. B. in Frechet, Jean M. J.; Hawker, Craig J., "Hyperbranched polyphenylene and hyperbranched polyesters: new soluble, three-dimensional, reactive polymers", Reactive & Functional Polymers (1995), 26(1-3), 127-36; Janssen, H. M.; Meijer, E. W., "The synthesis and characterization of dendritic molecules", Materials Science and Technology (1999), 20 (Synthesis of Polymers), 403-458; Tomalia, Donald A., "Dendrimer molecules", Scientific American (1995), 272(5), 62-6; WO 2002/067343 A1 und WO 2005/026144 A1.

Die erfindungsgemäßen Verbindungen, Polymere, Oligomere und Dendrimere können mit anderen organisch funktionellen Materialien, die in elektronischen Vorrichtungen verwendet werden, als Zusammensetzungen eingesetzt werden. Dem Fachmann ist hierbei eine Vielzahl möglicher organisch funktioneller Materialien aus dem Stand der Technik bekannt.

Die vorliegende Erfindung betrifft daher auch eine Zusammensetzung enthaltend eine oder mehrere erfindungsgemäße Verbindungen der Formel (1) oder wenigstes ein erfindungsgemäßes Polymer, Oligomer oder Dendrimer und wenigstens ein weiteres funktionelles Material ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, Elektronenblockiermaterialien, Loch-blockiermaterialien und p-Dotanden.

Bevorzugt sind dabei Zusammenetzungen enthaltend eine oder mehrere erfindungsgemäße Verbindungen der Formel (1) oder wenigstes ein erfindungsgemäßes Polymer, Oligomer oder Dendrimer und wenigstens ein weiteres organisches Halbleitermaterial ausgewählt aus der Gruppe bestehend aus Elektronentransportmaterialien.

Weiterhin bevorzugt sind Zusammenetzungen enthaltend eine oder mehrere erfindungsgemäße Verbindungen der Formel (1) oder wenigstes ein erfindungsgemäßes Polymer, Oligomer oder Dendrimer und wenigstens ein weiteres organisches Halbleitermaterial ausgewählt aus der Gruppe bestehend aus Lochtransportmaterialien.

Weiterhin bevorzugt sind Zusammenetzungen enthaltend eine oder mehrere erfindungsgemäße Verbindungen der Formel (1) oder wenigstes ein erfindungsgemäßes Polymer, Oligomer oder Dendrimer und wenigstens einen p-Dotanden.

Weiterhin bevorzugt sind Zusammenetzungen enthaltend eine oder mehrere erfindungsgemäße Verbindungen der Formel (1) oder wenigstes ein erfindungsgemäßes Polymer, Oligomer oder Dendrimer und wenigstens ein weiteres organisches Halbleitermaterial ausgewählt aus der Gruppe bestehend aus der Matrixmaterialien.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Miniemulsionen sein. Es kann bevorzugt sein, hierfür Mischungen aus zwei oder mehr Lösemitteln zu verwenden.

Gegenstand der Erfindung ist daher weiterhin eine Formulierung, insbesondere eine Lösung, Dispersion oder Miniemulsion, enthaltend mindestens eine Verbindung gemäß Formel (1), eine Zusammensetzung enthaltend mindestens eine Verbindung gemäß Formel (1) oder mindestens ein Polymer, Oligomer oder Dendrimer enthaltend mindestens eine Einheit gemäß Formel (1) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Geeignete und bevorzugte Lösungsmittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-tetramethylbenzol, 1-Methylnaphtalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzyl ether, Diethylenglycolbutylmethylether, Triethylenglycolbutylmethylether, Diethylenglycoldibutylether, Triethylenglycoldimethylether, Diethylen-glycolmonobutylether, Tripropylenglycoldimethylether, Tetraethylenglycol-dimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösungsmittel.

Die erfindungsgemäßen Verbindungen eignen sich für den Einsatz in elektronischen Vorrichtungen, insbesondere in organischen Elektrolumineszenzvorrichtungen (z.B. OLEDs oder OLECs). Abhängig von der Substitution werden die Verbindungen in unterschiedlichen Funktionen und Schichten eingesetzt.

Weiterer Gegenstand der Erfindung ist daher die Verwendung einer Verbindung der Formel (1) in einer elektronischen Vorrichtung, bevorzugt in einer lochtransportierenden und/oder in einer emittierenden Schicht.

Die erfindungsgemäße elektronische Vorrichtung ist bevorzugt gewählt aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs, LECs oder LEECs), organischen Laserdioden (O-Laser) und organischen lichtemittierenden Dioden (OLEDs). Besonders bevorzugt sind die organischen Elektrolumineszenzvorrichtungen, ganz besonders bevorzugt die OLECs und OLEDs und insbesondere bevorzugt die OLEDs.

Gegenstand der Erfindung sind, wie bereits oben ausgeführt, elektronische Vorrichtungen, enthaltend mindestens eine Verbindung gemäß Formel (1). Dabei sind die elektronischen Vorrichtungen bevorzugt ausgewählt aus den oben genannten Vorrichtungen. Besonders bevorzugt sind organische Elektrolumineszenzvorrichtungen und gannz besonders bevorzugt sind OLEDs enthaltend Anode, Kathode und mindestens eine emittierende Schicht, dadurch gekennzeichnet, dass mindestens eine organische Schicht, die eine emittierende Schicht, eine Lochtransportschicht oder eine andere Schicht sein kann, mindestens eine Verbindung gemäß Formel (1) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei die drei Schichten blaue, grüne und orange oder rote Emission zeigen (für den prinzipiellen Aufbau siehe z. B. WO 2005/011013). Die erfindungsgemäßen Verbindungen können in solchen Vorrichtungen in einer Lochtransportschicht, einer emittierenden Schicht und/oder in einer anderen Schicht vorhanden sein. Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Es ist erfindungsgemäß bevorzugt, wenn die Verbindung gemäß Formel (1) in einer elektrolumineszierneden Vorrichtung enthaltend einen oder mehrere phosphoreszierende Dotanden eingesetzt wird. Dabei kann die Verbindung in unterschiedlichen Schichten, bevorzugt in einer Lochtransportschicht, einer Lochinjektionsschicht oder in einer emittierenden Schicht, verwendet werden. Die Verbindung gemäß Formel (1) kann aber auch erfindungsgemäß in einer elektronischen Vorrichtung enthaltend einen oder mehrere fluoreszierende Dotanden eingesetzt werden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele der oben beschriebenen Emitter können den Anmeldungen WO 00/70655, WO 01/41512, WO 02/02714, WO 02/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Auch kann der Fachmann ohne erfinderisches Zutun weitere phosphoreszierende Komplexe in Kombination mit den Verbindungen gemäß Formel (1) in organischen Elektrolumineszenzvorrichtungen einsetzen.

Explizite Beispiele für geeignete phosphoreszierende Emitterverbindungen können weiterhin der folgenden Tabelle entnommen werden.

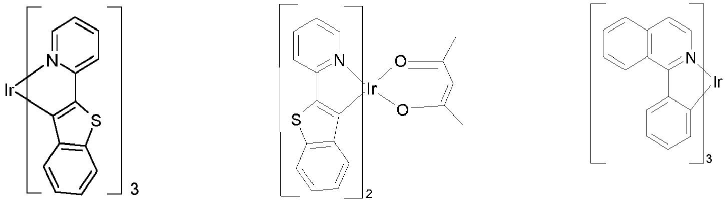

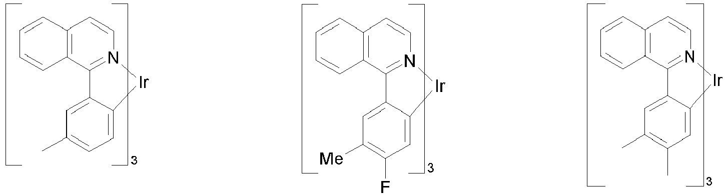

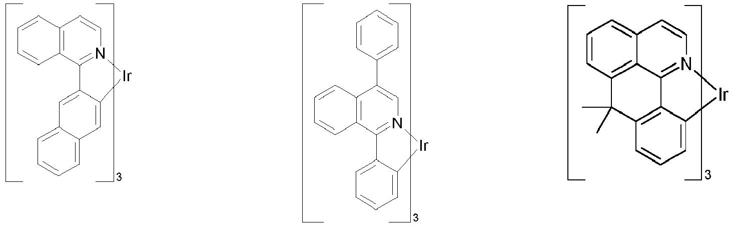

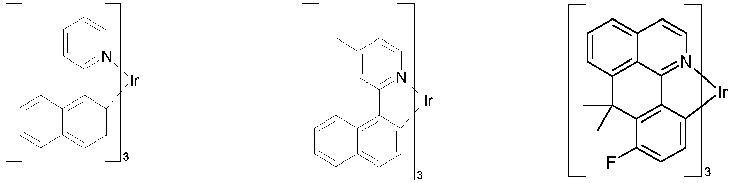

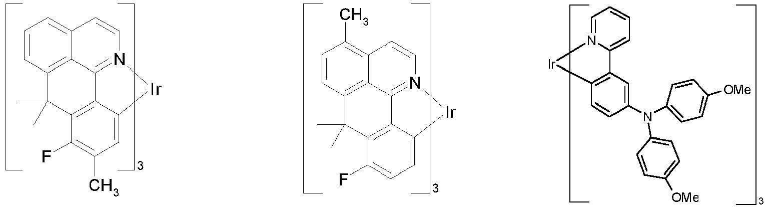

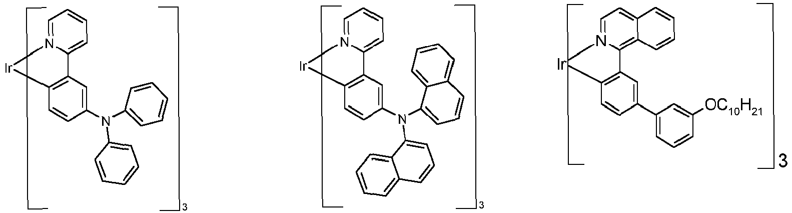

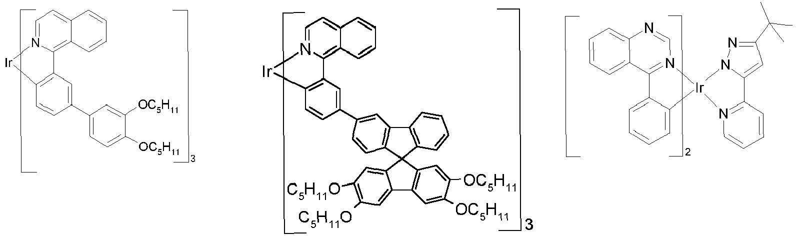

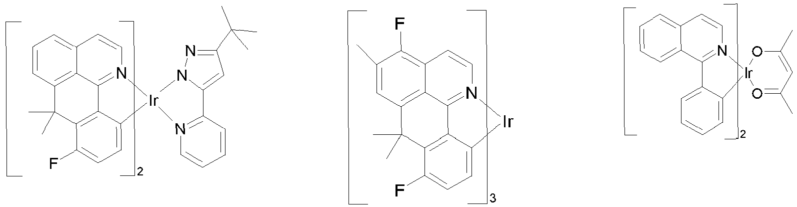

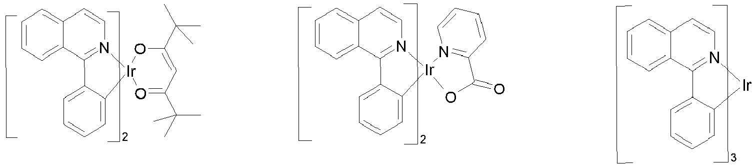

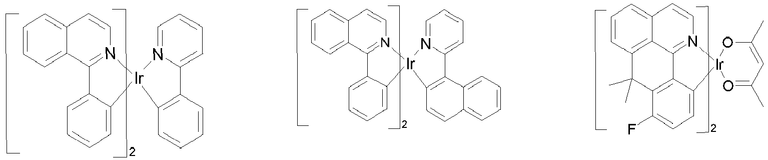

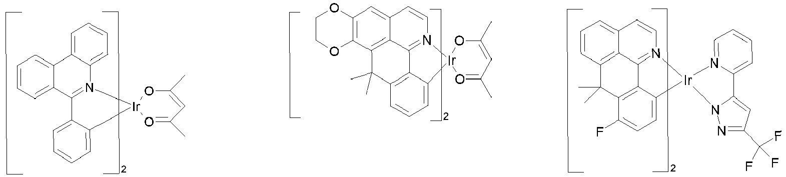

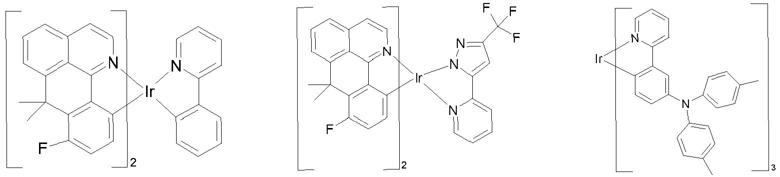

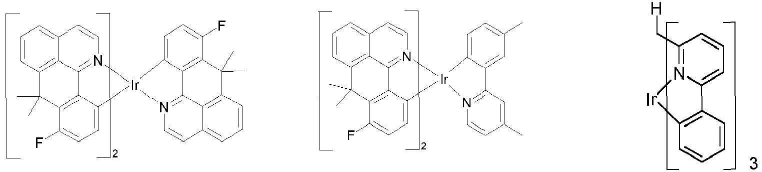

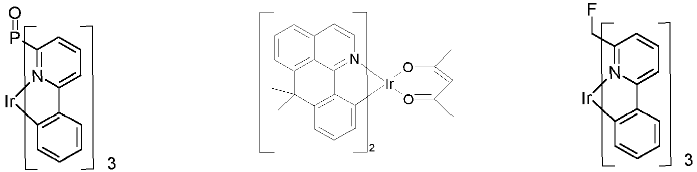

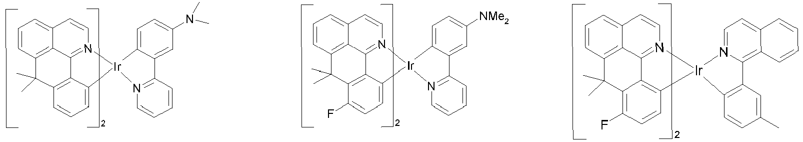

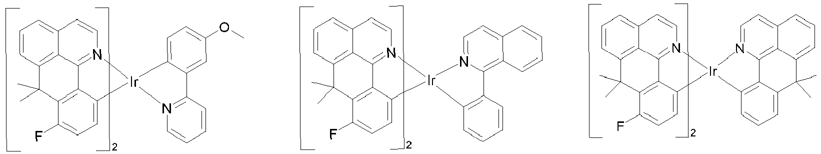

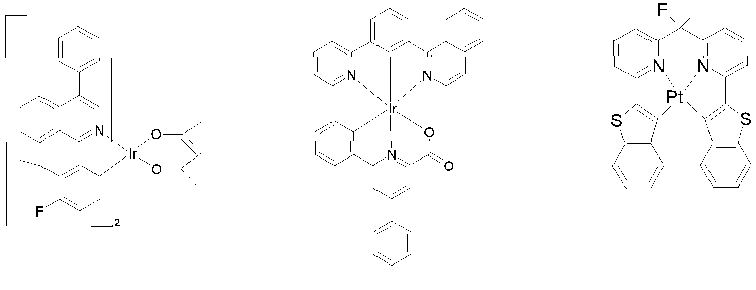

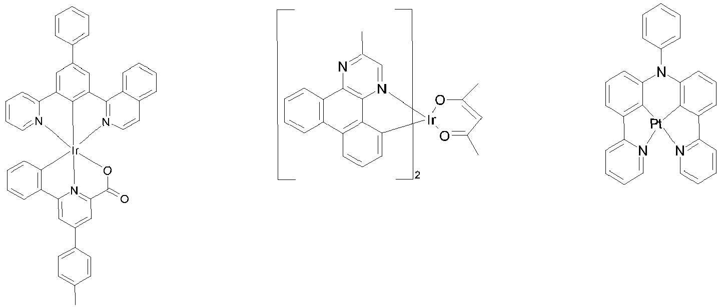

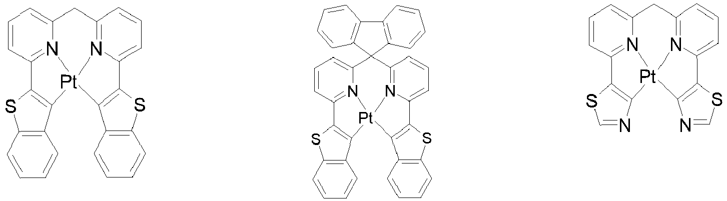

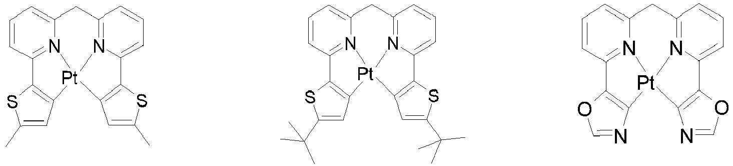

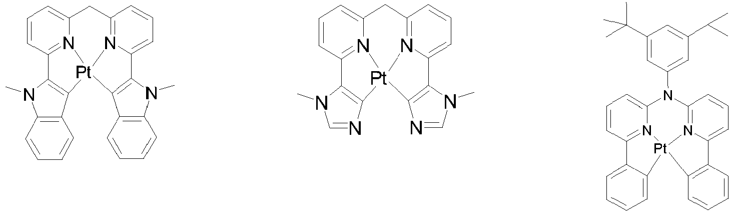

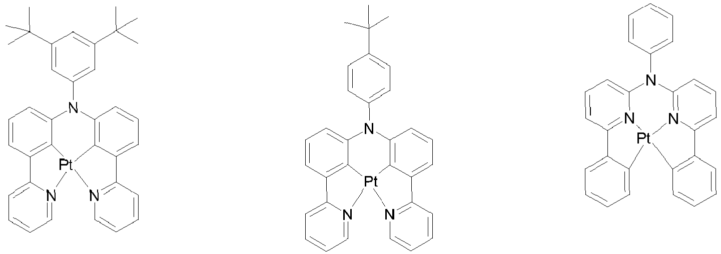

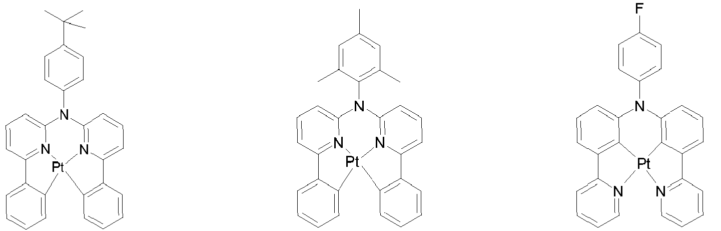

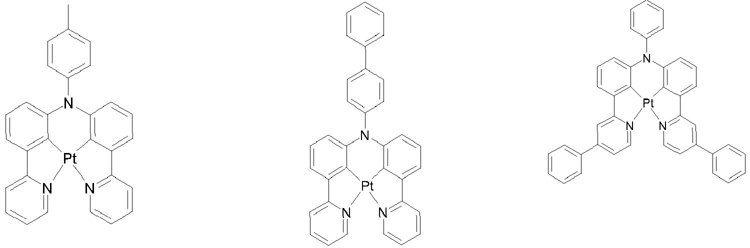

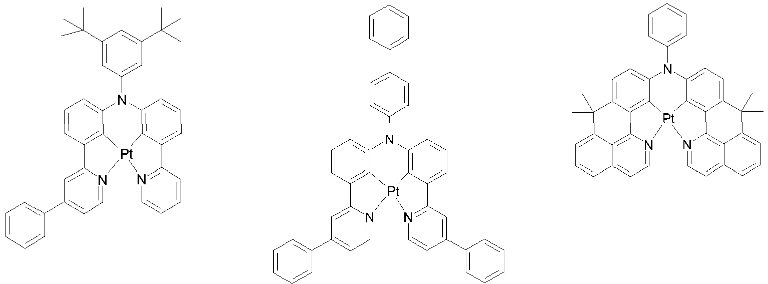

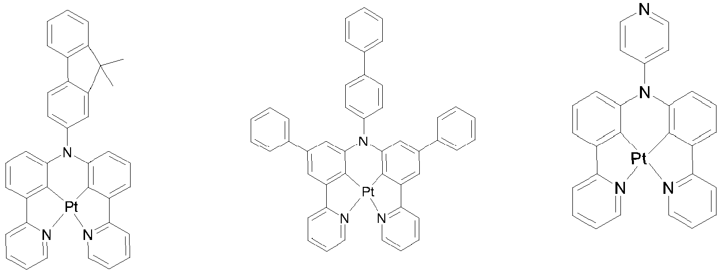

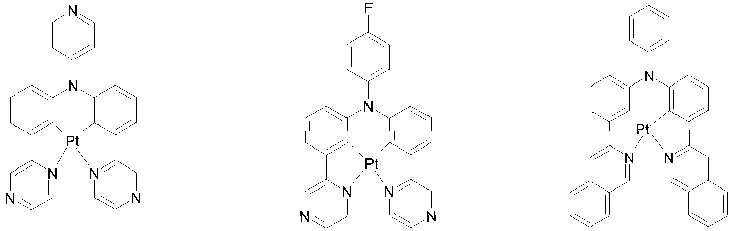

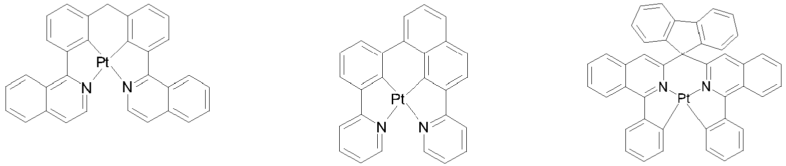

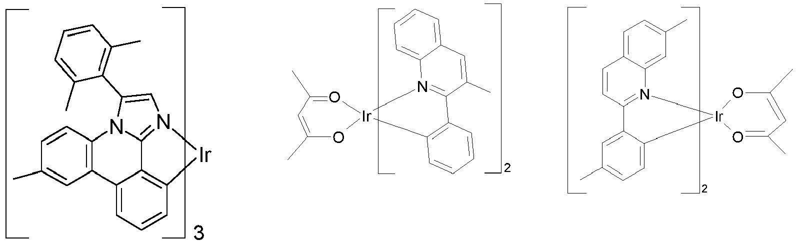

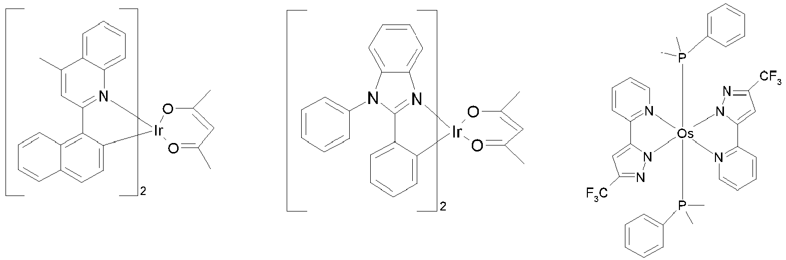

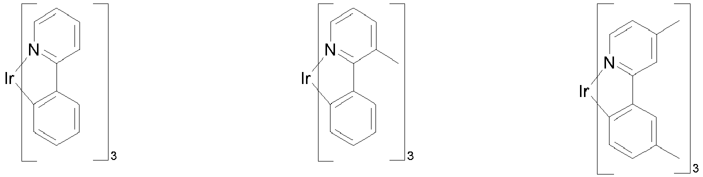

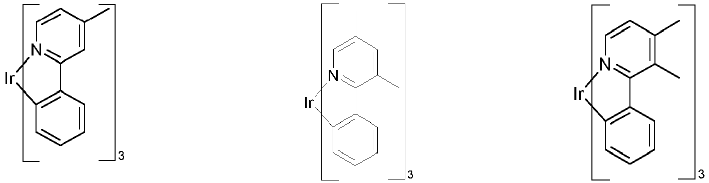

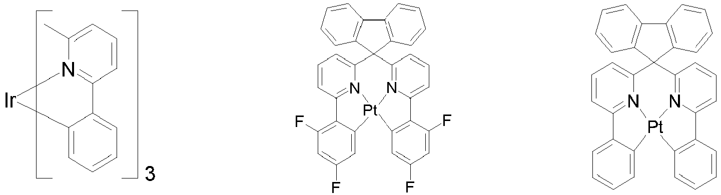

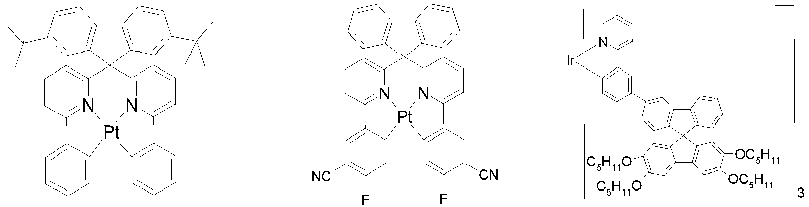

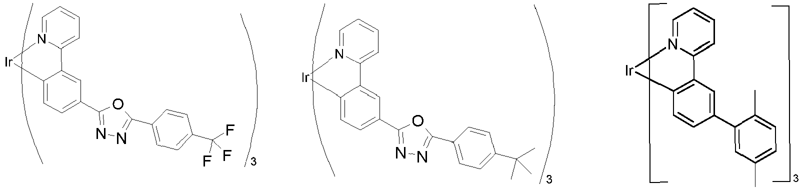

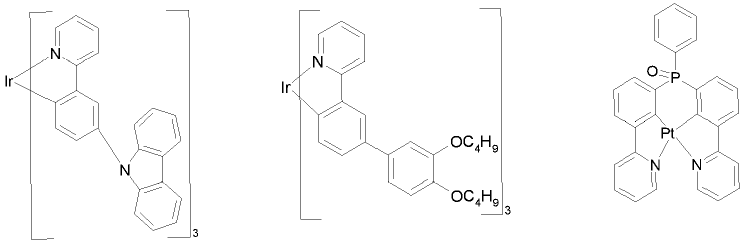

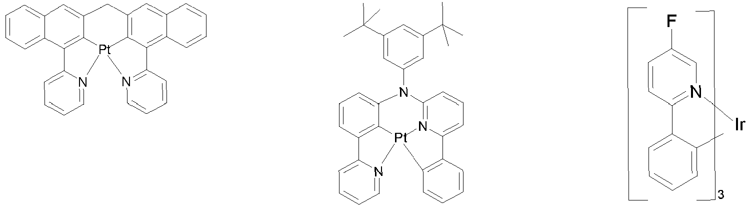

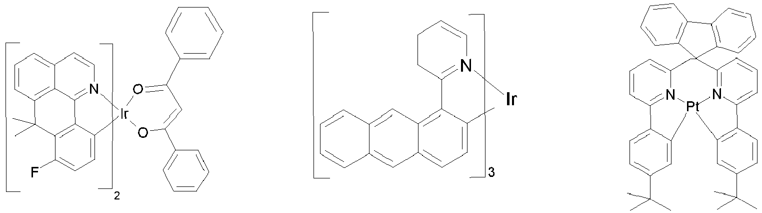

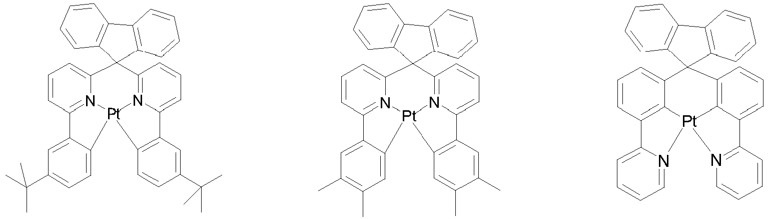

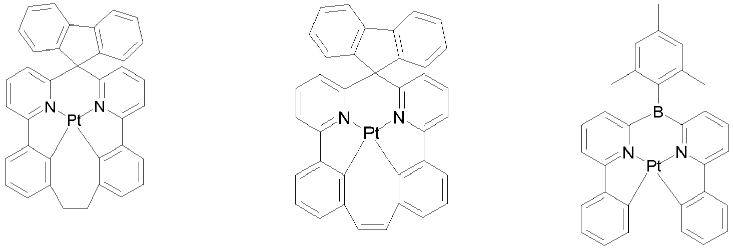

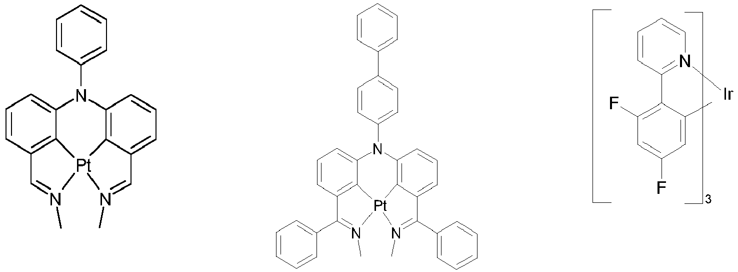

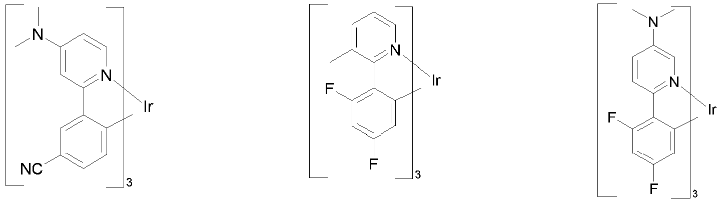

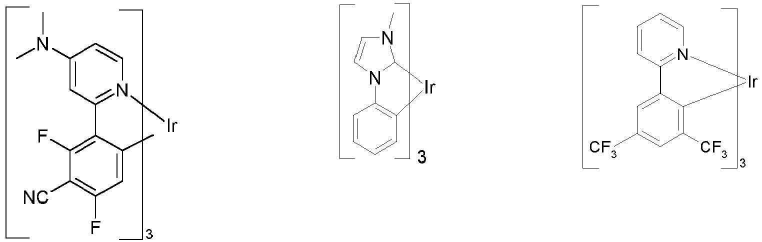

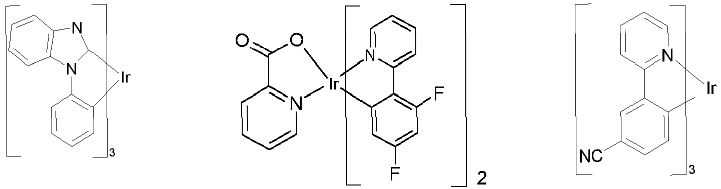

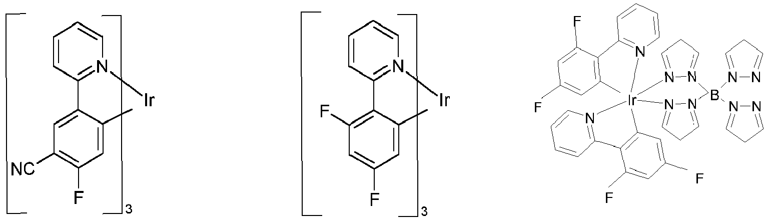

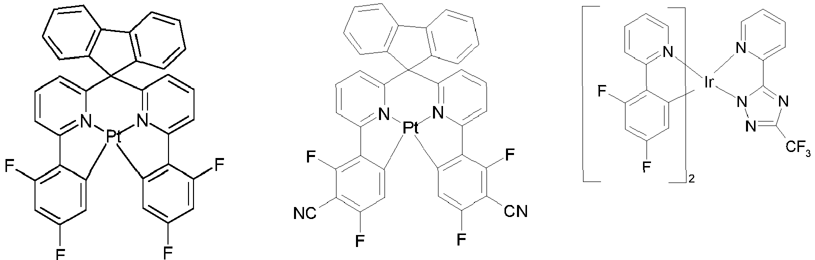

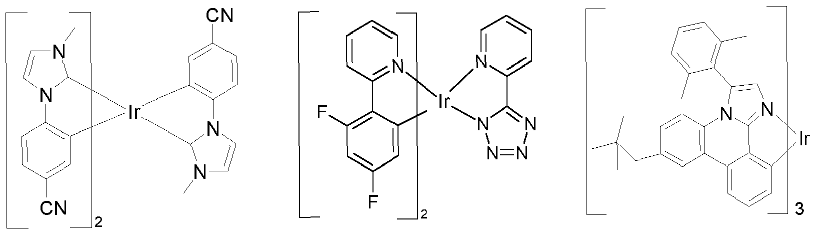

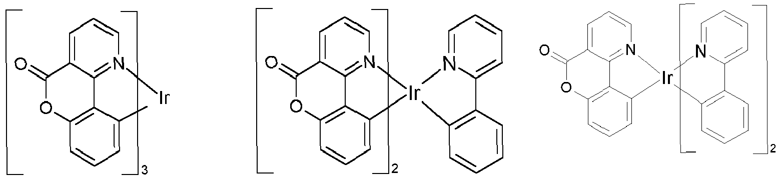

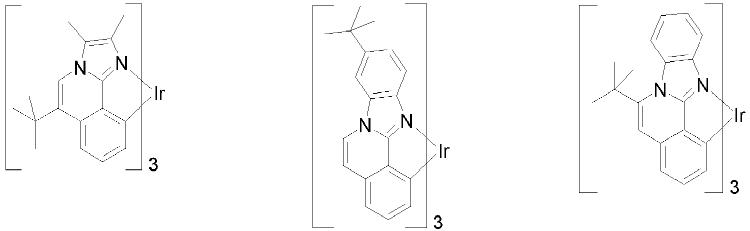

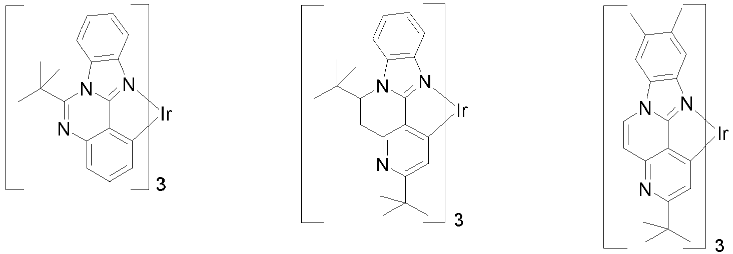

In einer bevorzugten Ausführungsform der Erfindung werden die Verbindungen gemäß der allgemeinen Formel (1) als Lochtransportmaterial eingesetzt. Die Verbindungen werden dann bevorzugt in einer Lochtransportschicht, eine Elektronblockierendenschicht und/oder in einer Lochinjektionsschicht eingesetzt. Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen der Lochinjektionsschicht und der Emissionsschicht liegt. Die Lochtransportschicht kann direkt an die Emissionschicht angrenzen. Wenn die Verbindungen gemäß Formel (1) als Lochtransportmaterial oder als Lochinjektionsmaterial verwendet werden, kann es bevorzugt sein, wenn sie mit Elektronenakzeptor-Verbindungen dotiert sind (p-Doping), beispielsweise mit F₄-TCNQ, F₆-TNAP oder mit Verbindungen, wie sie in EP 1476881 oder EP 1596445 beschrieben werden. In einer weiteren bevorzugten Ausführungsform der Erfindung wird eine Verbindung gemäß Formel (1) als Lochtransportmaterial in Kombination mit einem Hexaazatriphenylenderivat, wie in US 2007/0092755 beschrieben, verwendet. Besonders bevorzugt wird das Hexaazatriphenylenderivat dabei in einer separaten Schicht eingesetzt.

Werden die Verbindungen gemäß der allgemeinen Formel (1) als Lochtransportmaterial in einer Lochtransportschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen in der Lochtransportschicht eingesetzt werden.

In einer weiteren Ausführungsform der vorliegenden Erfindung werden die Verbindungen der allgemeinen Formel (1) als emittierende Materialien eingesetzt. Die Verbindungen werden hierzu bevorzugt in einer Emissionsschicht eingesetzt. Die Emissionsschicht enthält neben wenigstens einer der Verbindungen nach der allgemeinen Formel (1) weiterhin wenigstens ein Host Material. Dabei kann der Fachmann aus den bekannten Hostmaterialien ohne Schwierigkeiten und ohne erfinderisch zu sein auswählen.

In einer weiteren Ausführungsform der vorliegenden Erfindung werden die Verbindungen der allgemeinen Formel (1) als Matrixmaterial in Kombination mit einem oder mehreren Dotanden, vorzugsweise phosphoreszierenden Dotanden, eingesetzt.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindungen gemäß der allgemeinen Formel (1) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Die Mixed-Matrix-Systeme können einen oder mehrere Dotanden umfassen, bevorzugt einen oder mehrere phosphoreszierende Dotanden. Allgemein werden Mixed-Matrix-Systeme bevorzugt in phosphoreszierenden organischen Elektrolumineszenzvorrichtungen eingesetzt.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende Dotanden oder den bevorzugten Matrixmaterialien für fluoreszierende Dotanden, je nachdem welche Art von Dotand im mixed-Matrix-System eingesetzt wird.

Bevorzugte phosphoreszierende Dotanden zur Verwendung in Mixed-Matrix-Systemen sind die in der obenstehenden Tabelle aufgeführten phosphoreszierenden Dotanden.

Unter p-Dotanden werden hierin Oxidationsmittel, d.h. Elektronenakzeptoren verstanden. Bevorzugte Beispiele für p-Dotanden sind F₄-TCNQ, F₆-TNAP, NDP-2 (Fa. Novaled), NDP-9 (Fa. Novaled), Chinone (z.B. EP 1538684 A1, WO 2006/081780 A1, WO 2009/003455 A1, WO 2010/097433 A1), Radialene (z.B. EP 1988587 A1, US 2010/102709 A1, EP 2180029 A1, WO 2011/131185 A1, WO 2011134458 A1, US 2012/223296 A1), S-haltige Übergangsmetallkomplexe (z.B. WO 2007/134873 A1, WO 2008/061517 A2, WO 2008/061518 A2, DE 102008051737 A1, WO 2009/089821 A1, US 2010/096600 A1), Bisimidazole (z.B. WO 2008/138580 A1), Phthalocyanine (z.B. WO 2008/058525 A2), Bora-Tetraazapentalene (z.B. WO 2007/115540 A1) Fullerene (z.B. DE 102010046040 A1) und Hauptgruppenhalogenide (z.B. WO 2008/128519 A2).

Im Folgenden werden die in den erfindungsgemäßen Vorrichtungen in den betreffenden Funktionen bevorzugt eingesetzten Materialien aufgeführt.

Bevorzugte fluoreszierende Dotanden sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind.

Als Matrixmaterialien, bevorzugt für fluoreszierende Dotanden, kommen neben den erfindungsgemäßen Verbindungen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Bevorzugte Matrixmaterialien für phosphoreszierende Dotanden sind neben den erfindungsgemäßen Verbindungen aromatische Amine, insbesondere Triarylamine, z. B. gemäß US 2005/0069729, Carbazolderivate (z. B. CBP, N,N-Biscarbazolylbiphenyl) oder Verbindungen gemäß WO 2005/039246, US 2005/0069729, JP 2004/288381, EP 1205527 oder WO 2008/086851, verbrückte Carbazolderivate, z. B. gemäß WO 2011/088877 und WO 2011/128017, Indenocarbazolderivate, z. B. gemäß WO 2010/136109 und WO 2011/000455, Azacarbazolderivate, z. B. gemäß EP 1617710, EP 1617711, EP 1731584, JP 2005/347160, Indolocarbazolderivate, z. B. gemäß WO 2007/063754 oder WO 2008/056746, Ketone, z. B. gemäß WO 2004/093207 oder WO 2010/006680, Phosphinoxide, Sulfoxide und Sulfone, z. B. gemäß WO 2005/003253, Oligophenylene, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Aluminiumkomplexe, z. B. BAlq, Diazasilol- und Tetraazasilol-Derivate, z. B. gemäß WO 2010/054729, Diazaphosphol-Derivate, z. B. gemäß WO 2010/054730 und Aluminiumkomplexe, z. B. BAlQ.

Geeignete Ladungstransportmaterialien, wie sie in der Lochinjektions- bzw. Lochtransportschicht oder in der Elektronentransportschicht der erfindungsgemäßen organischen Elektrolumineszenzvorrichtung verwendet werden können, sind neben den erfindungsgemäßen Verbindungen beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Kathode der organischen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (1) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere Verbindungen gemäß der allgemeinen Formel (1) in Displays, als Lichtquellen in Beleuchtungsanwendungen sowie als Lichtquellen in medizinischen und/oder kosmetischen Anwendungen (z.B. Lichttherapie) eingesetzt werden.

Vorrichtungen enthaltend die Verbindungen der allgemeinen Formel (1) können sehr vielseitig eingesetzt werden. So können, beispielsweise, elektrolumineszierende Vorrichtuungen enthaltend eine oder mehrere Verbindungen nach der allgemeinen Formel (1) in Displays für Fernseher, Mobiltelefone, Computer und Kameras eingesetzt werden. Die Vorrichtungen können aber auch in Beleuchtungsanwendungen verwendet werden. Weiterhin können elektrolumineszierende Vorrichtungen, z. B. in OLEDs oder OLECs, enthaltend wenigstens eine der Verbindung nach der allgemeinen Formel (1) in der Medizin oder Kosmetik zu Phototherapie genutzt werden. Somit kann eine Vielzahl von Erkrankungen (Psoriasis, atopische Dermatitis, Inflammation, Akne, Hautkrebs etc.) oder die Vermeidung sowie Reduktion von Hautfaltenbildung, Hautrötung und Hautalterung behandelt werden. Weiterhin können die lichtemittierenden Vorrichtungen dazu genutzt werden, um Getränke, Speisen oder Lebensmittel frisch zu halten oder um Geräte (bspw. medizinische Geräte) zu sterilisieren.

Die erfindungsgemäßen Verbindungen bzw. die erfindungsgemäßen organischen Elektrolumineszenzvorrichtungen zeichnen sich durch folgende überraschende Vorteile gegenüber dem Stand der Technik aus:
1. Die erfindungsgemäßen Verbindungen eignen sich sehr gut für den Einsatz in einer Lochtransportschicht oder einer Lochinjektionsschicht in elektronischen Vorrichtungen, wie beispielsweise in organischen Elektrolumineszenzvorrichtungen, insbesondere aufgrund ihrer hohen Lochbeweglichkeit. Dabei eignen sie sich insbesondere auch für die Verwendung in einer Schicht, die direkt an eine phosphoreszierende emittierende Schicht angrenzt, da die erfindungsgemäßen Verbindungen nicht die Lumineszenz löschen.
2. Die erfindungsgemäßen Verbindungen, eingesetzt als Matrixmaterial für fluoreszierende oder phosphoreszierende Emitter führen zu sehr hohen Effizienzen sowie zu langen Lebensdauern. Dies gilt insbesondere, wenn die Verbindungen als Matrixmaterial zusammen mit einem weiteren Matrixmaterial und einem phosphoreszierenden Emitter eingesetzt werden.
3. Die erfindungsgemäßen Verbindungen, eingesetzt in organischen Elektrolumineszenzvorrichtungen, führen zu hohen Effizienzen und zu steilen Strom-Spannungs-Kurven mit niedrigen Einsatz- und Betriebsspannungen.
4. Die Verwendung der erfindungsgemäßen Verbindungen in elektronischen Vorrichtungen, insbesondere eingesetzt als Lochtransportoder Lochinjektionsmaterial führt zu hohen Effizienzen, geringen Betriebspannungen sowie zu langen Lebensdauern.
5. Die erfindungsgemäßen Verbindungen weisen eine relativ niedrige Sublimationstemperatur, eine hohe Temperaturstabilität sowie eine hohe Oxidationsstabilität und eine hohe Glasübergangtemperatur auf, was sowohl für die Prozessierbarkeit, beispielweise aus Lösung oder aus der Gasphase, als auch für die Verwendung in elektronischen Vorrichtungen vorteilhaft ist.
6. Die erfindungsgemäßen Verbindungen weisen eine hohe Oxidationsstabilität auf, was sich insbesondere positiv auf die Handhabung dieser Verbindungen sowie auf die Lagerstabilität für Lösungen auswirkt.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen.

### Beispiele

### Beispiel 1

### Synthese von Biphenyl-4-yl-(9,9-dimethyl-9H-fluoren-2-yl)-[4-(9-methyl-9-phenyl-9H-fluoren-4-yl)-phenyl]-amin (1-1) sowie der Verbindungen (1-2) bis (1-7)

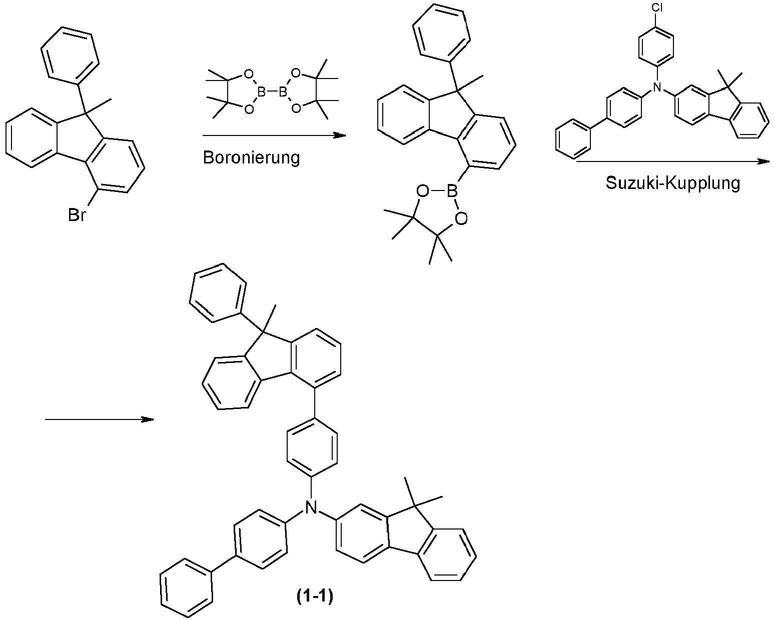

### Ausgangsmaterial: 4-Bromo-9-methyl-9-diphenyl-9H-fluoren

50 g (157 mmol) 2,2'-Dibromo-biphenyl werden in einem ausgeheizten Kolben in 500 mL getrocknetem THF gelöst. Die Reaktionsmischung wird auf -78°C gekühlt. Bei dieser Temperatur werden 63 mL einer 2,5 M Lösung n-BuLi in Hexan (157 mmol) langsam zugetropft (Dauer: ca 1 Stunde). Der Ansatz wird 1 h bei -70°C nachgerührt. Anschließend werden 18,9 g Acetophenon (119 mmol) in 100 mL THF gelöst und bei -70°C zugetropft. Nach beendeter Zugabe wird die Reaktionsmischung langsam auf Raumtemperatur erwärmt, mit NH₄Cl gequencht und anschließend am Rotationsverdampfer eingeengt. Die einrotierte Lösung wird vorsichtig mit 510 mL Essigsäure versetzt und anschließen werden 100 mL rauchende HCl zugegeben. Der Ansatz wird auf 75°C erhitzt und 4 h auf dieser Temperatur gehalten. Dabei fällt ein weißer Feststoff aus. Der Ansatz wird dann auf Raumtemperatur abgekühlt, der ausgefallene Feststoff wird abgesaugt und mit Methanol nachgewaschen. Der Rückstand wird im Vakuum bei 40°C getrocknet. Ausbeute beträgt 48 g (121 mmol) (77% der Theorie)

### 4,4,5,5-Tetramethyl-2-(9-methyl-9-phenyl-9H-fluoren-4-yl)-[1,3,2]dioxaborolan

50 g (125 mmol) des 4-Brom-fluorenderivats, 38,3 g (150 mmol) Bis(pinacolato)diboran und 35 g (378 mmol) Kalliumacetat werden in 700 mL Dioxan suspendiert. Zu dieser Suspension werden 3.08 g (3,78 mmol) 1,1-Bis(diphenylphosphino)ferrocen-dichloropalladium(II) Komplex mit DCM gegeben. Die Reaktionsmischung wird 14 h unter Rückfluss erhitzt. Nach dem Erkalten wird die organische Phase abgetrennt, dreimal mit 400 mL Wasser gewaschen und anschließend zur Trockne eingeengt. Der Rückstand wird aus Toluol umkristallisiert (42 g, 75% Ausbeute).

Analog dazu werden folgende Verbindungen hergestellt:

| **Edukt** | **Produkt** | **Ausbeute** |
|---|---|---|
| 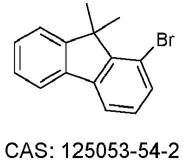 | 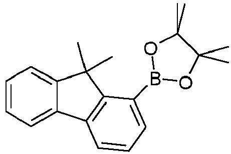 | 83% |
| CAS: 125053-54-2 | | |
| 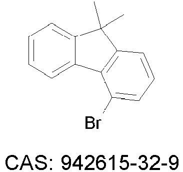 | 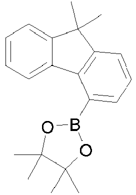 | 78% |
| CAS: 942615-32-9 | | |
| 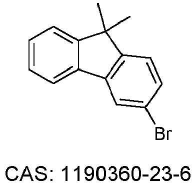 | 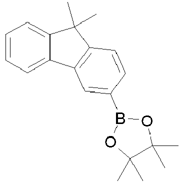 | 88% |
| CAS: 1190360-23-6 | | |
| 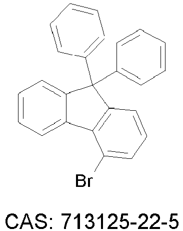 | 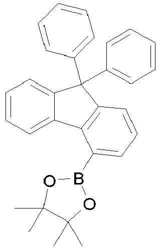 | 91% |
| CAS: 713125-22-5 | | |

### Zwischenstufe: Biphenyl-4-yl-(4-chloro-phenyl)-(9,9-dimethyl-9H-fluoren-2-yl)-amin

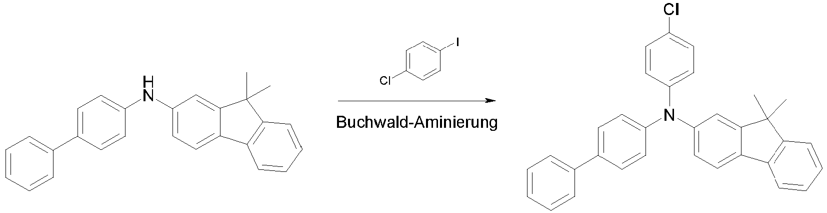

40 g Biphenyl-4-yl-(9,9-dimethyl-9H-fluoren-2-yl)-amin (111 mmol) und 26,4 g 4-Chlor-iodbenzol (111 mmol) werden in 700 mL Toluol gelöst. Die Lösung wird entgast und mit N₂ gesättigt. Danach wird sie mit 4,43 mL (4.42 mmol) einer Tri-tert-Butylphosphin 1 M-Lösung und 0,5 g (2,21 mmol) Palladium(II)acetat versetzt und anschließend werden 15,9 g Natrium-tert-butylat (186 mmol) zugegeben. Die Reaktionsmischung wird 12 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert. Die Ausbeute beträgt 44,5 g (85% d. Th).

Analog dazu werden folgende Verbindungen hergestellt:

| **Edukt 1** | **Edukt 2** | **Produkt** | **Ausbeute** |
|---|---|---|---|
| 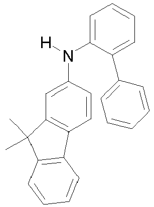 | 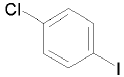 | 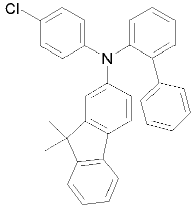 | 78 % |
| 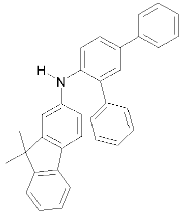 | 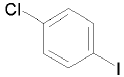 | 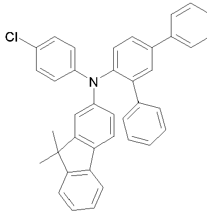 | 80% |
| 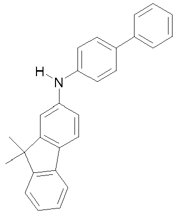 | 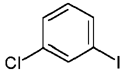 | 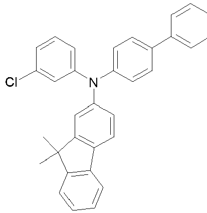 | 81% |
| 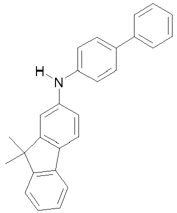 | 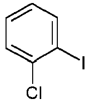 | 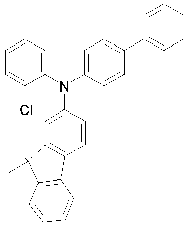 | 81% |
| 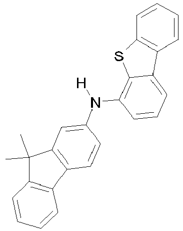 | 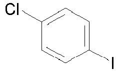 | 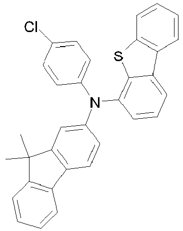 | 92% |
| 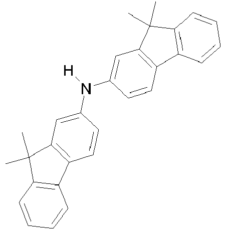 | 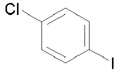 | 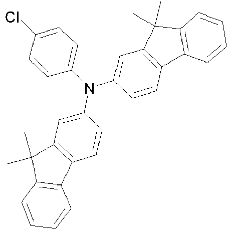 | 75% |
| 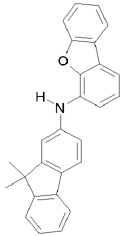 | 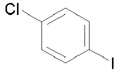 | 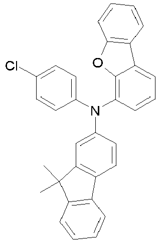 | 78% |
| 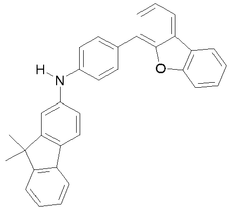 | 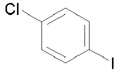 | 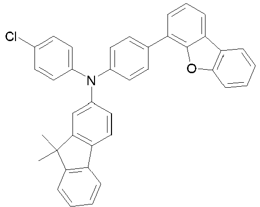 | 81% |
| 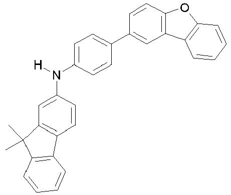 | 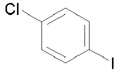 | 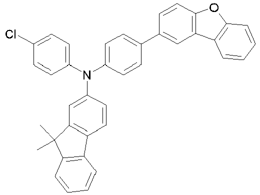 | 75% |
| 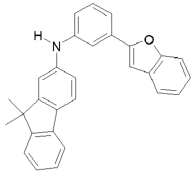 | 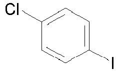 | 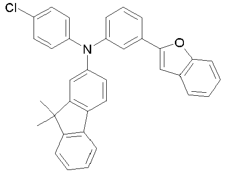 | 86% |
| 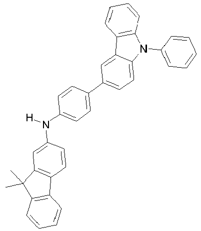 | 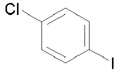 | 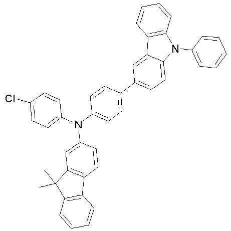 | 88% |
| 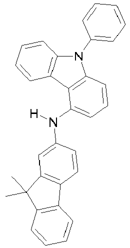 | 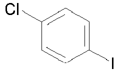 | 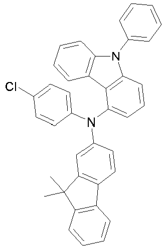 | 73% |

### Synthese der Verbindung (1-1)

20 g (44 mmol) 4-Fluoren-pinacolboronester Derivat, 20,8 g (44 mmol) des Chlor-Derivats und 13,4 g Caesiumfluorid (88,1 mmol) werden in 265 mL Dioxan suspendiert. Zu dieser Suspension werden 3,9 g (5,28 mmol) Palladium dichlorid-bis(tricyclohexylphosphin) gegeben, und die Reaktionsmischung wird 18 h unter Rückfluss erhitzt. Nach dem Erkalten wird die organische Phase abgetrennt, über Kieselgel filtriert, dreimal mit 100 mL Wasser gewaschen und anschließend zur Trockne eingeengt. Nach Filtration des Rohproduktes über Kieselgel mit Toluol wird der verbleibende Rückstand aus Heptan/Toluol umkristallisiert und abschließend im Hochvakuum sublimiert. Die Reinheit beträgt 99.9%. Die Ausbeute beträgt 26,5 g (80% d. Th).

### Synthese der Verbindungen (1-2) bis (1-15)

Analog zu der in Beispiel 1 beschriebenen Synthese von Verbindung (1-1) werden auch die folgenden Verbindungen (1-2) bis (1-15) hergestellt.

| **Edukt 1** | **Edukt 2** | **Produkt** | **Ausbeute** |
|---|---|---|---|
| 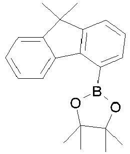 | 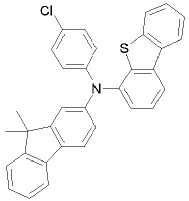 | 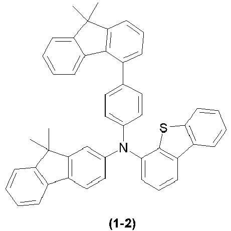 | 78% |
| 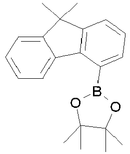 | 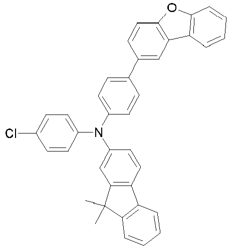 | 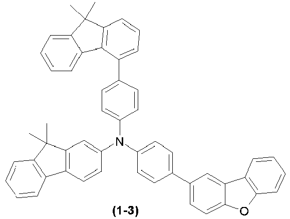 | 70% |
| 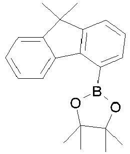 | 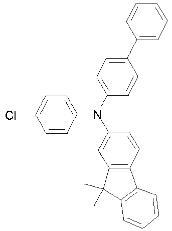 | 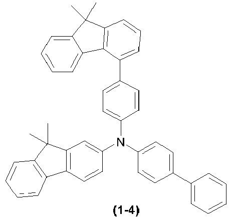 | 78% |
| 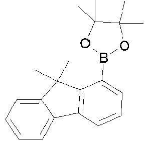 | 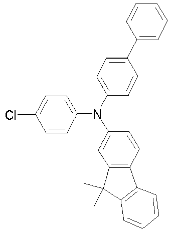 | 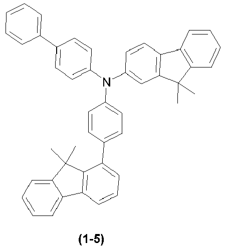 | 80% |
| 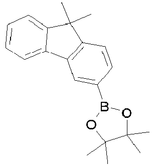 | 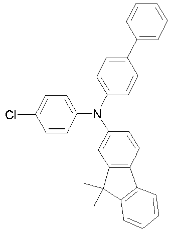 | 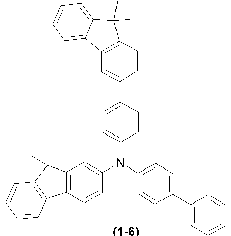 | 75% |
| 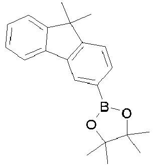 | 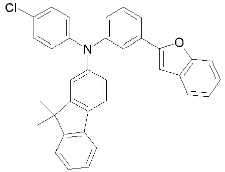 | 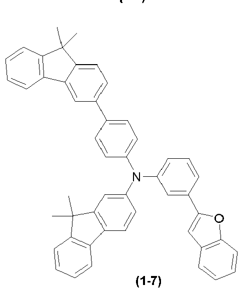 | 68% |
| 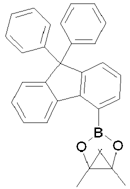 | 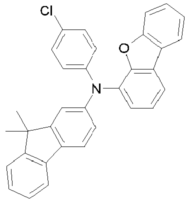 | 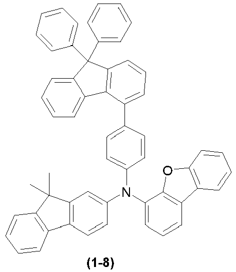 | 80% |
| 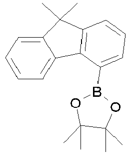 | 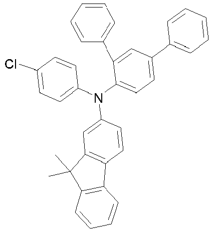 | 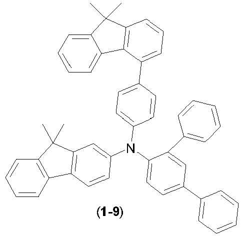 | 75% |
| 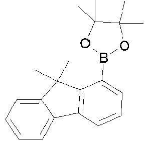 | 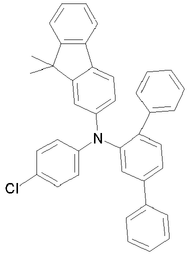 | 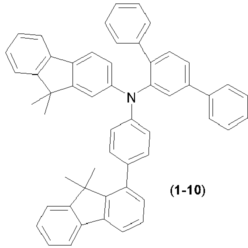 | 73% |
| 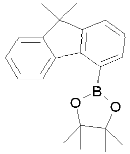 | 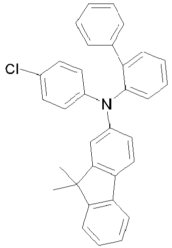 | 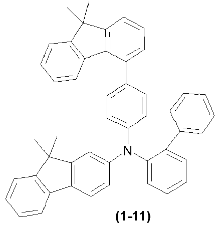 | 80% |
| 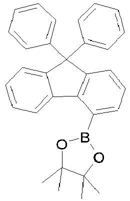 | 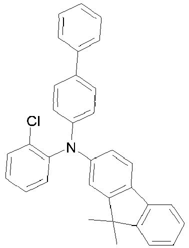 | 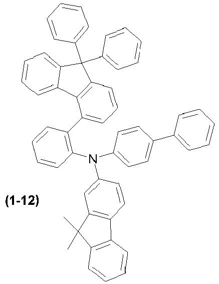 | 77% |
| 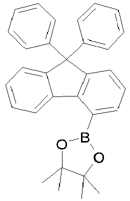 | 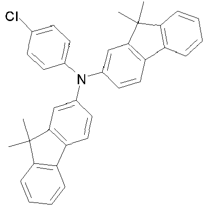 | 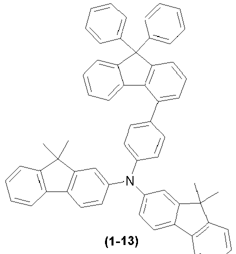 | 85% |
| 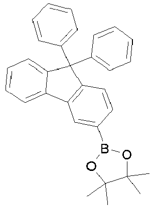 | 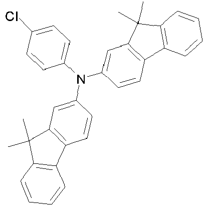 | 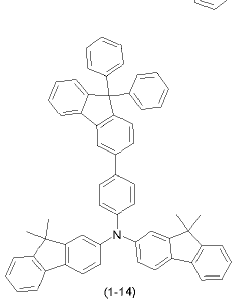 | 85% |
| 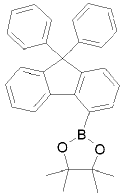 | 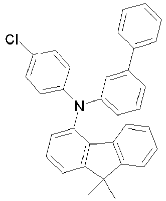 | 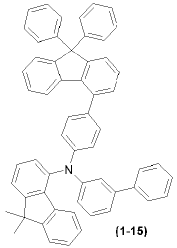 | 88% |
| 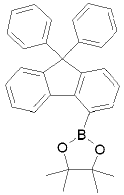 | 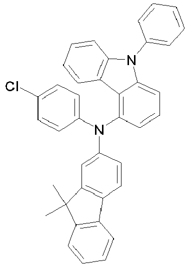 | 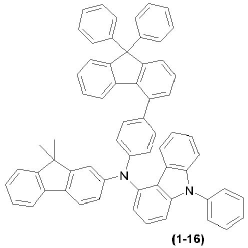 | 75% |
| 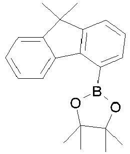 | 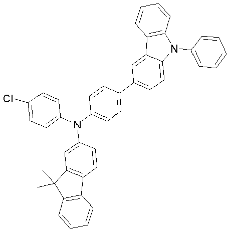 | 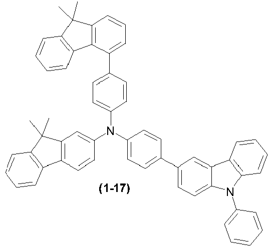 | 80% |

### Beispiel 2

### Synthese der Verbindung [4-(7-Dibenzofuran-4-yl-9,9-diphenyl-9H-fluoren-4-yl)-phenyl]-(9,9-dimethyl-9H-fluoren-2-yl)-phenyl-amin (2-1) sowie der Verbindungen (2-2) bis (2-5)

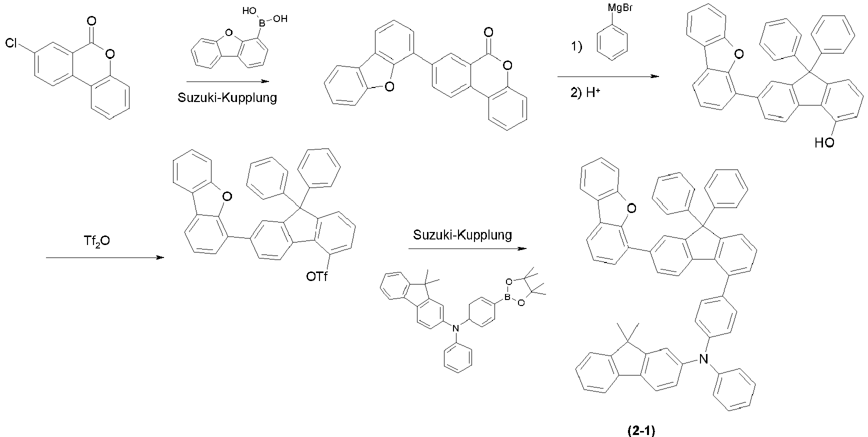

### 8-Dibenzofuran-4-yl-benzo[c]chromen-6-on

30.0 g (142 mmol) Dibenzofuran-4-Boronsäure, 32 g (142 mmol) 8-Chloro-benzo[c]chromen-6-on (CAS: 742058-81-7) und 43 g Caesiumfluorid (283 mmol) werden in 800 mL Dioxan suspendiert. Zu dieser Suspension werden 12.5 g (17 mmol) Palladium dichlorid-bis(tricyclohexylphosphin) gegeben, und die Reaktionsmischung wird 18 h unter Rückfluss erhitzt. Nach Erkalten wird die organische Phase abgetrennt, über Kieselgel filtriert, dreimal mit 100 mL Wasser gewaschen und anschließend zur Trockene eingeengt. Nach Filtration des Rohproduktes über Kieselgel mit Toluol wird der verbleibende Rückstand aus Heptan/Toluol umkristallisiert Die Ausbeute beträgt 45 g (88% d. Th).

Analog dazu werden folgende Verbindungen hergestellt:

| **Edukt 1** | **Edukt 2** | **Produkt** | **Ausbeute** |
|---|---|---|---|
| 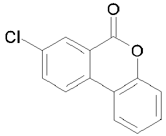 | 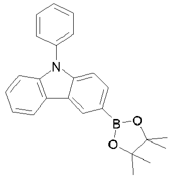 | 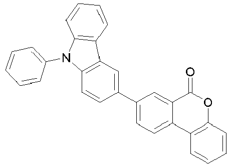 | 84% |
| 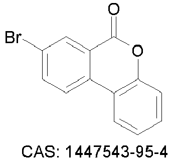 | 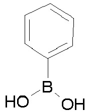 | 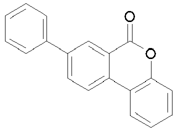 | 90% |
| 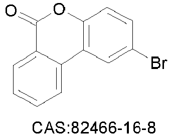 | 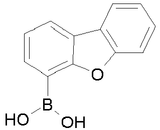 | 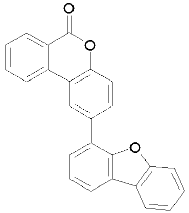 | 85% |
| 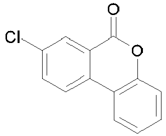 | 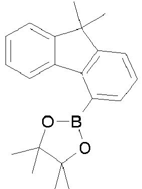 | 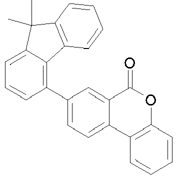 | 76% |
| 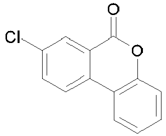 | 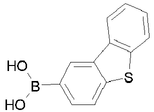 | 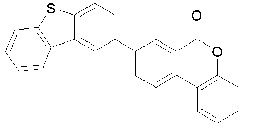 | 81% |
| 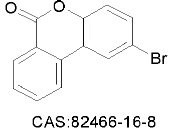 | 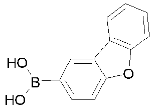 | 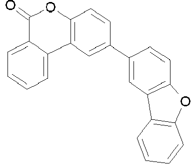 | 87% |

### 7-Dibenzofuran-4-yl-9,9-diphenyl-9H-fluoren-4-ol

25.4 g (70 mmol) 8-Dibenzofuran-4-yl-benzo[c]chromen-6-on werden in einem ausgeheizten Kolben in 340 mL getrocknetem THF gelöst. Die Lösung wird mit N₂ gesättigt. Die klare Lösung wird auf -10°C abgekühlt und dann werden 70 mL (210 mmol) einer 3M-Phenhylmagnesiumbromid Lösung zugegeben. Die Reaktionsmischung wird langsam auf Raumtemperatur erwärmt und dann mit Essigsäureanhidrid gequencht (70 mmol). Das Gemisch wird im Anschluss zwischen Essigester und Wasser verteilt, die organische Phase dreimal mit Wasser gewaschen, über Na₂SO₄ getrocknet und einrotiert. Die einrotierte Lösung wird vorsichtig mit 310 ml Essigsäure versetzt und anschließen werden 70 ml rauchende HCl zugegeben. Der Ansatz wird auf 75°C erhitzt und 4 h auf dieser Temperatur gehalten. Dabei fällt ein weißer Feststoff aus. Der Ansatz wird dann auf Raumtemperatur abgekühlt, der ausgefallene Feststoff wird abgesaugt und mit Methanol nachgewaschen. Der Rückstand wird im Vakuum bei 40°C getrocknet. Nach Filtration des Rohproduktes über Kieselgel mit (Heptane:Essigsester, 1:1) erhält man 26 g (75% der Theorie)

Analog dazu werden folgende bromierten Verbindungen hergestellt:

| **Edukt 1** | **Edukt 2** | **Produkt** | **Ausbeute** |
|---|---|---|---|
| 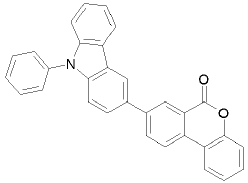 | 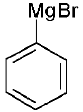 | 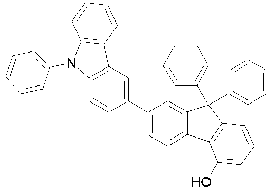 | 72% |
| 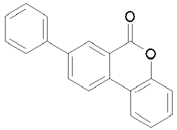 | 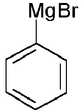 | 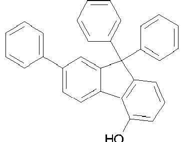 | 80% |
| 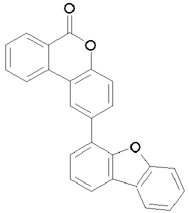 | 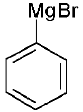 | 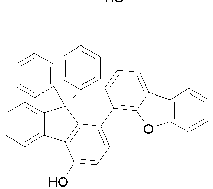 | 77% |
| 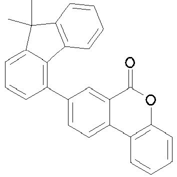 | CH₃MgBr | 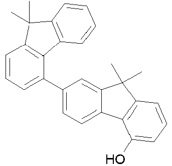 | 72% |
| 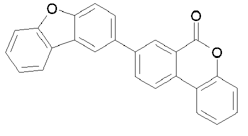 | 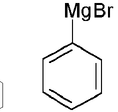 | 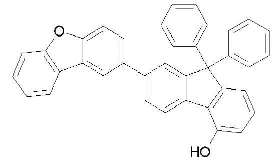 | 66% |
| 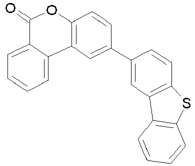 | 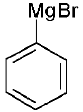 | 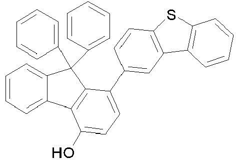 | 74% |

### (9,9-Dimethyl-9H-fluoren-2-yl)-phenyl-[4-(4,4,5,5-tetramethyl-[1,3,2]dioxaborolan-2-yl)-phenyl]-amin

32,1 g (81,1 mmol) des 4-chloro-phenylamin derivates, 20,6 g (81,1 mmol) Bis(pinacolato)diboran und 13,5 g (137,9 mmol) Kalliumacetat werden in 600 mL Dioxan suspendiert. Zu dieser Suspension werden 5,9 g (8,1 mmol) Dichlorobis(tricyclohexylphosphine)palladium(II). Die Reaktionsmischung wird 12 h unter Rückfluss erhitzt. Nach dem Erkalten wird die organische Phase abgetrennt, dreimal mit 200 mL Wasser gewaschen und anschließend zur Trockne eingeengt. Der Rückstand wird aus Toluol umkristallisiert (28.8 g, 73% Ausbeute).

Analog dazu werden folgende Verbindungen hergestellt:

| **Edukt 1** | **Produkt** | **Ausbeute** |
|---|---|---|
| 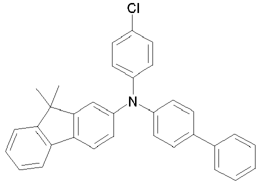 | 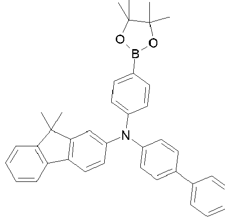 | 79% |
| 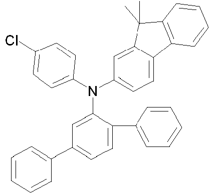 | 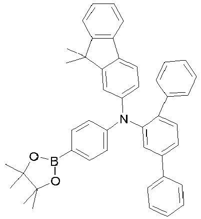 | 80% |
| 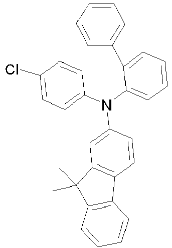 | 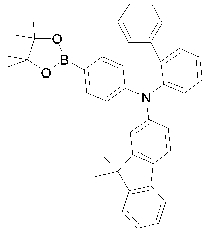 | 72% |
| 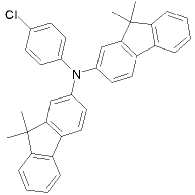 | 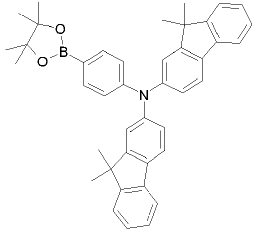 | 79% |
| 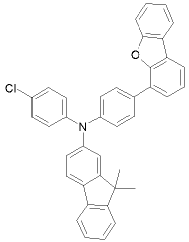 | 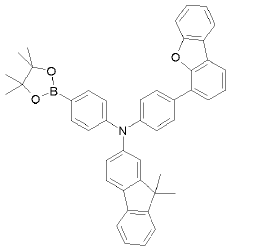 | 78% |

### Verbindung [4-(7-Dibenzofuran-4-yl-9,9-diphenyl-9H-fluoren-4-yl)-phenyl]-(9,9-dimethyl-9H-fluoren-2-yl)-phenyl-amin (2-1)

25 g (50 mmol) 7-Dibenzofuran-4-yl-9,9-diphenyl-9H-fluoren-4-ol werden in einem ausgeheizten Kolben in 300 mL getrocknetem THF gelöst. Die Lösung wird mit N₂ gesättigt. Die klare Lösung wird auf 5°C abgekühlt und dann werden 20 mL (150 mmol) Triethylamin, 122 mg 4-dimethylaminopyridine und 8,65 mL trifluoromethansulfonsäure anhydride zugegeben. Die Reaktionsmischung wird langsam auf Raumtemperatur erwärmt. Das Reaktiongemisch wird im Anschluss mit Heptan verdünnt, einrotiert und Wasser verteilt, die organische Phase dreimal mit Wasser gewaschen, über Na₂SO₄ getrocknet und einrotiert. Nach Filtration des Rohproduktes über Kieselgel mit Heptane:Essigsester, 1:1) erhält man 30 g Trifluoro-methanesulfonic acid 7-dibenzofuran-4-yl-9,9-diphenyl-9H-fluoren-4-yl ester (98% der Theorie)

23,5 g (36,4 mmol) Trifluoro-methanesulfonic acid 7-dibenzofuran-4-yl-9,9-diphenyl-9H-fluoren-4-yl ester, 18.61 g (38.2 mmol) (9,9-Dimethyl-9H-fluoren-2-yl)-phenyl-[4-(4,4,5,5-tetramethyl-[1,3,2]dioxaborolan-2-yl)-phenyl]-amine, 7,64 g natriummetaborat-Tetrahydrat (54,6 mmol) und 53 µL hydraziniumhydroxid (1,1 mmol) werden in 500 mL THF und 200 mL Wasser suspendiert. Zu dieser Suspension werden 1.02 g (1,46 mmol) Palladium dichlorid-bis(triphenylphosphin) gegeben, und die Reaktionsmischung wird 3 h auf 70°C erhitzt. Nach dem Erkalten wird das Gemisch zwischen Essigester und Wasser verteilt, die organische Phase dreimal mit Wasser gewaschen, über Na₂SO₄ g getrocknet und anschließend zur Trockene eingeengt. Nach Filtration des Rohproduktes über Kieselgel mit Toluol wird der verbleibende Rückstand aus Heptan/Toluol umkristallisiert und anschliessen sublimiert. Die Ausbeute beträgt 18,4 g (60% d. Th)

### Synthese der Verbindungen (2-2) bis (2-6)

Analog dazu werden folgende Verbindungen hergestellt:

| **Edukt 1** | **Edukt 2** | **Produkt** | **Ausbeute** |
|---|---|---|---|
| 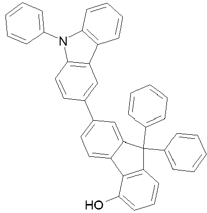 | 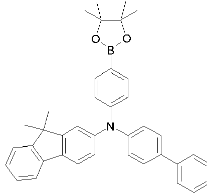 ] | 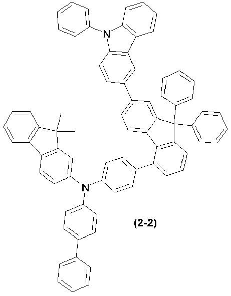 | 78% |
| 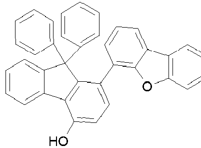 | 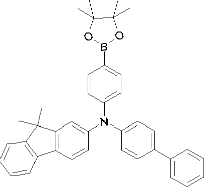 | 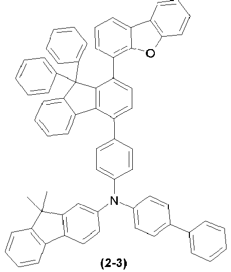 | 73% |
| 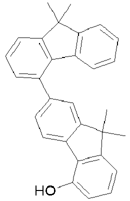 | 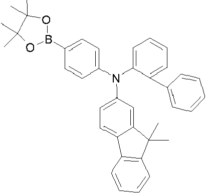 | 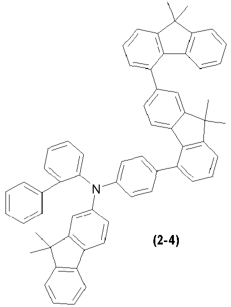 | 71% |
| 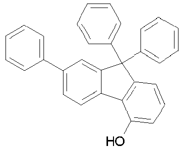 | 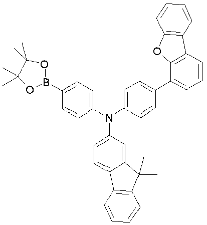 | 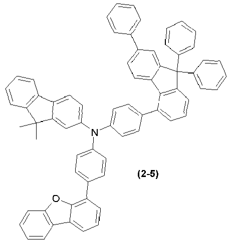 | 65% |
| 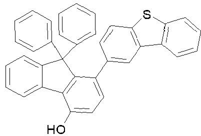 | 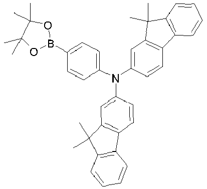 | 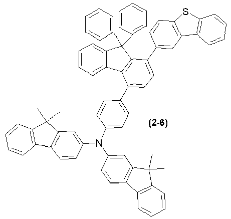 | 52% |

### Beispiel 3

### Synthese der Verbindung Biphenyl-4-yl-biphenyl-2-yl-(5-{4-[(9,9-dimethyl-9H-fluoren-2-yl)-phenyl-amino]-phenyl}-9,9-diphenyl-9H-fluoren-2-yl)-amin (3-1) sowie der Verbindungen (3-2) bis (3-6)

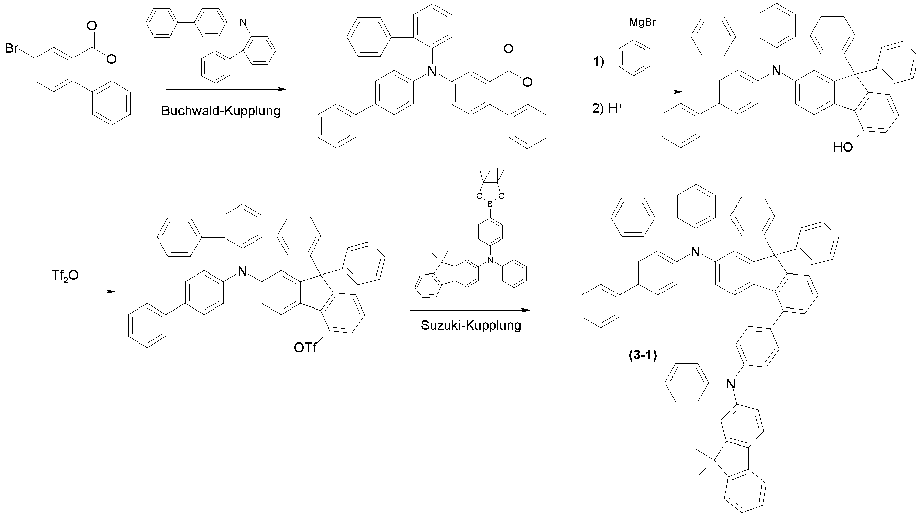

### 8-(Biphenyl-4-yl-biphenyl-2-yl-amino)-benzo[c]chromen-6-on

19.0 g Biphenyl-2-yl-biphenyl-4-yl-amin (59 mmol) und 16.3 g 8-Bromobenzo[c]chromen-6-one (CAS:1447543-95-4) (59 mmol) werden in 400 mL Toluol gelöst: Die Lösung wird entgast und mit N₂ gesättigt. Danach wird sie mit 2.36 mL (2.36 mmol) einer 1 M Tri-tert-Butylphosphin-Lösung und 0.27 g (1.18 mmol) Palladium(II)acetat versetzt. Anschließend werden 11.6 g Natrium-tert-butylat (109 mmol) zugegeben. Die Reaktionsmischung wird 3 h unter Schutzatmosphäre zum Sieden erhitzt. Das Gemisch wird im Anschluss zwischen Toluol und Wasser verteilt, die organische Phase dreimal mit Wasser gewaschen, über Na₂SO₄ getrocknet und einrotiert. Nach Filtration des Rohproduktes über Kieselgel mit Toluol wird der verbleibende Rückstand aus Heptan/Toluol. Die Ausbeute beträgt 27 g (90% der Theorie).

Analog dazu werden folgende Verbindungen hergestellt:

| **Edukt 1** | **Edukt 2** | **Produkt** | **Ausbeute** |
|---|---|---|---|
| 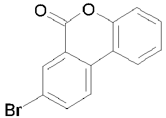 | 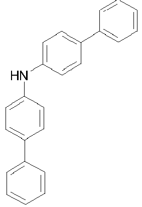 | 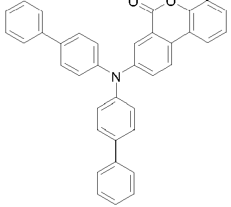 | 90% |
| 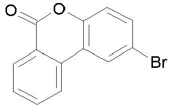 | 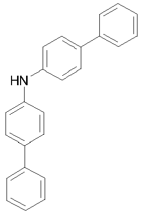 | 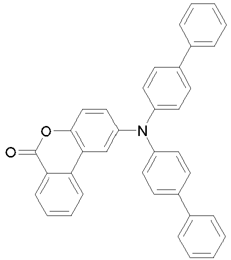 | 85% |
| 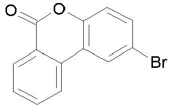 | 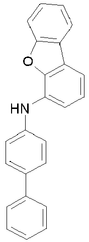 | 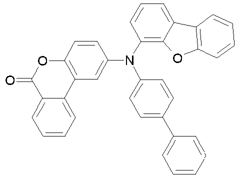 | 75% |

### 7-(Biphenyl-4-yl-biphenyl-2-yl-amino)-9,9-diphenyl-9H-fluoren-4-ol

Analog zu 7-Dibenzofuran-4-yl-9,9-diphenyl-9H-fluoren-4-ol werden folgende Verbindungen hergestellt:

| **Edukt 1** | **Edukt 2** | **Produkt** | **Ausbeute** |
|---|---|---|---|
| 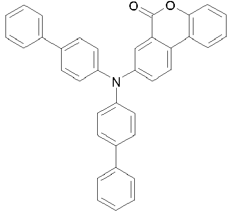 | 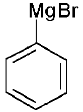 | 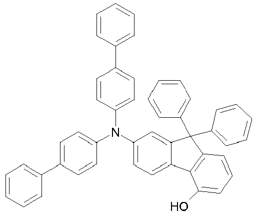 | 83% |
| 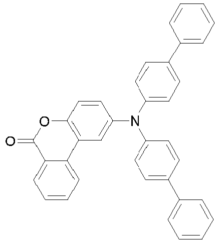 | 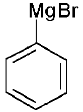 | 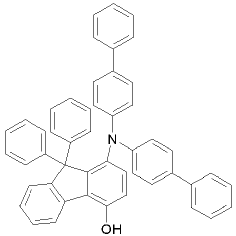 | 71% |
| 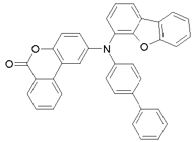 | CH₃MgBr | 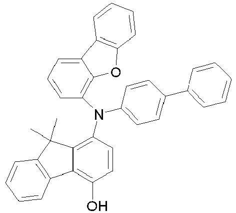 | 66% |

### Synthese der Verbindungen Biphenyl-4-yl-biphenyl-2-yl-(5-{4-[(9,9-dimethyl-9H-fluoren-2-yl)-phenyl-amino]-phenyl}-9,9-diphenyl-9H-fluoren-2-yl)-amine (3-1) sowie der Verbindungen (3-2) bis (3-5)

Analog zu Verbindung [4-(7-Dibenzofuran-4-yl-9,9-diphenyl-9H-fluoren-4-yl)-phenyl]-(9,9-dimethyl-9H-fluoren-2-yl)-phenyl-amin **(2-1)** werden Verbindungen **(3-1)** bis **(3-5)** hergestellt:

| **Edukt 1** | **Edukt 2** | **Produkt** | **Ausbeute** |
|---|---|---|---|
| 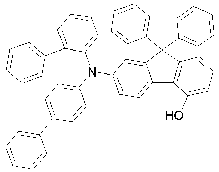 | 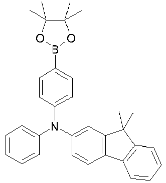 | 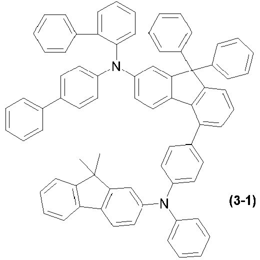 | 74% |
| 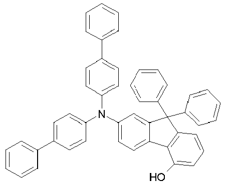 | 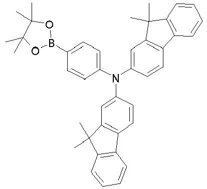 | 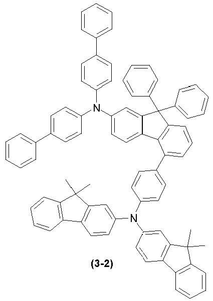 | 74% |
| 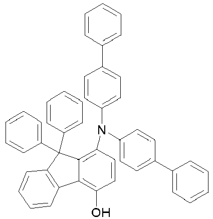 | 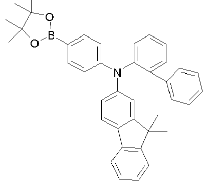 | 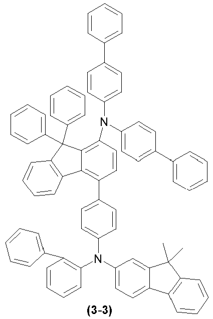 | 72% |
| 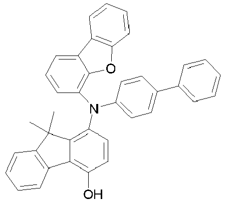 | 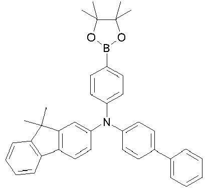 | 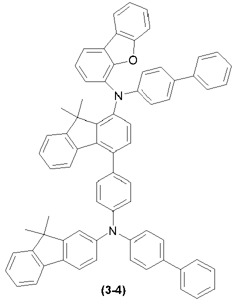 | 68% |
| 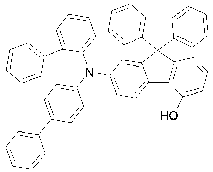 | 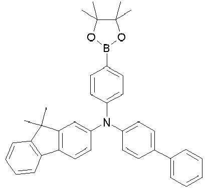 | 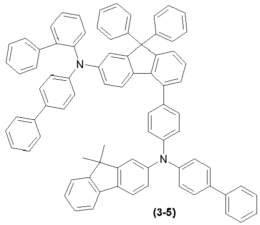 | 72% |

### Beispiel 4

### Synthese der Verbindung Biphenyl-4-yl-(9,9-dimethyl-9H-fluoren-2-yl)-{4-[9,9-dimethyl-4-(9-phenyl-9H-carbazol-3-yl)-9H-fluoren-1-yl]-phenyl}-amine (4-1) sowie der Verbindungen (4-2) bis (4-3)

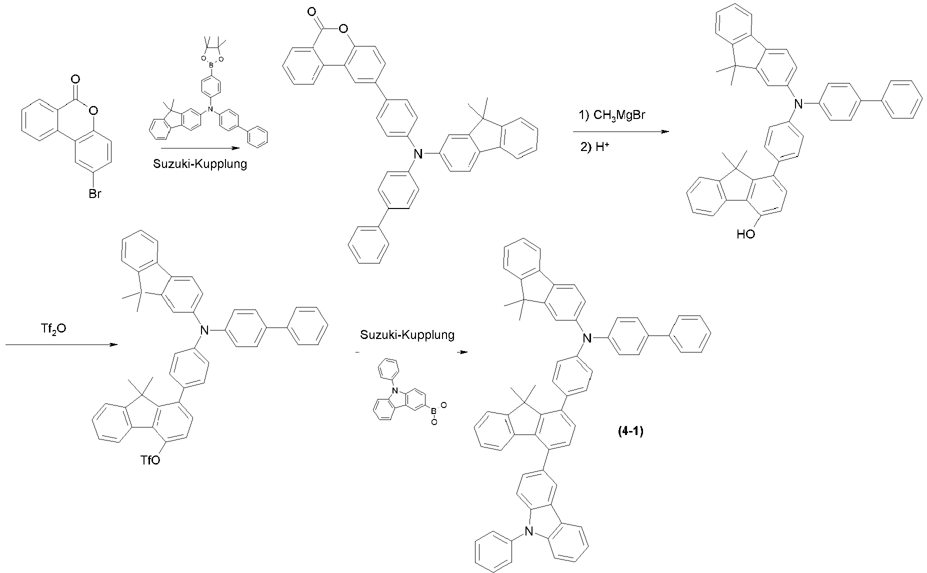

### 2-{4-[Biphenyl-4-yl-(9,9-dimethyl-9H-fluoren-2-yl)-amino]-phenyl}-benzo[c]chromen-6-on

20 g (35,5 mmol) des pinacolboronester Derivates, 9.76 g (35,5 mmol) des 3-Brom-benzo[c]chromen-6-on und 35 mL eine 2M-Na2CO3- Lösung (71,6 mmol) werden in 600 mL Dioxan suspendiert. Zu dieser Suspension werden 0,75 g (0,89 mmol) 1,1-Bis(diphenylphosphino)ferrocendichloropalladium(II) Komplex mit DCM gegeben, und die Reaktionsmischung wird 14 h unter Rückfluss erhitzt. Nach dem Erkalten wird die organische Phase abgetrennt, über Kieselgel filtriert, dreimal mit 100 mL Wasser gewaschen und anschließend zur Trockne eingeengt. Nach Filtration des Rohproduktes über Kieselgel mit Toluol wird der verbleibende Rückstand aus Heptan/Toluol umkristallisiert . Die Ausbeute beträgt 17,7 g (79% d. Th).

Analog dazu werden folgende Verbindungen hergestellt:

| **Edukt 1** | **Edukt 2** | **Produkt** | **Ausbeute** |
|---|---|---|---|
| 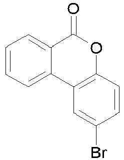 | 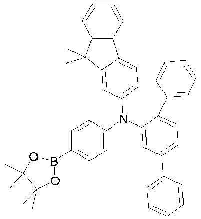 | 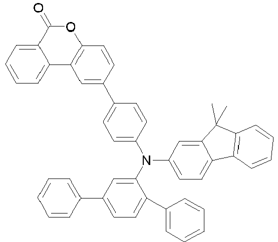 | 80% |
| 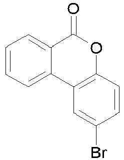 | 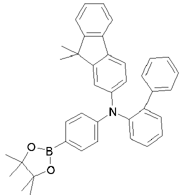 | 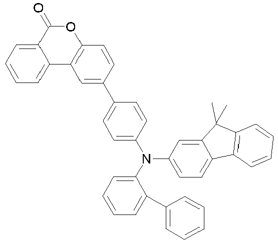 | 77% |

### 1-{4-[Biphenyl-4-yl-(9,9-dimethyl-9H-fluoren-2-yl)-amino]-phenyl}-9,9-dimethyl-9H-fluoren-4-ol

Analog zu den in der Beispiel 3 beschrieben Synthese von 7-Dibenzofuran-4-yl-9,9-diphenyl-9H-fluoren-4-ol wird das1-{4-[Biphenyl-4-yl-(9,9-dimethyl-9H-fluoren-2-yl)-amino]-phenyl}-9,9-dimethyl-9H-fluoren-4-ol hergestellt:

| **Edukt 1** | **Edukt 2** | **Produkt** | **Ausbeute** |
|---|---|---|---|
| 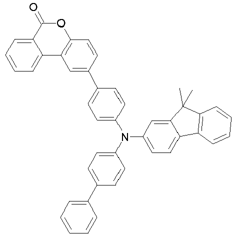 | CH₃MgBr | 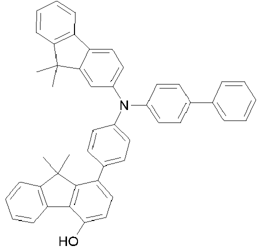 | 84% |

Analog dazu werden folgende Verbindungen hergestellt:

| **Edukt 1** | **Edukt 2** | **Produkt** | **Ausbeute** |
|---|---|---|---|
| 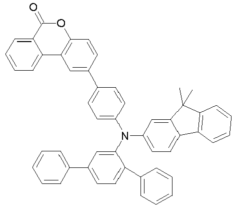 | 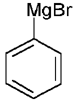 | 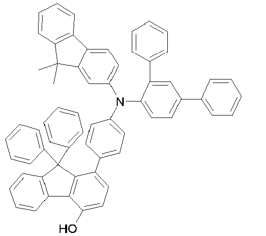 | 64% |
| 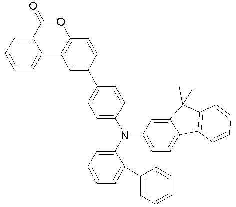 | 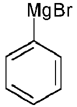 | 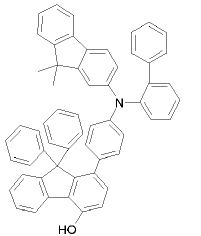 | 70% |

### Synthese der Verbindungen (4-1) bis (4-4)

Analog zu Verbindung [4-(7-Dibenzofuran-4-yl-9,9-diphenyl-9H-fluoren-4-yl)-phenyl]-(9,9-dimethyl-9H-fluoren-2-yl)-phenyl-amine Verbindung (2-1) werden Verbindung **(4-1)** bis **(4-4)** hergestellt:

| **Edukt 1** | **Edukt 2** | **Produkt** | **Ausbeute** |
|---|---|---|---|
| 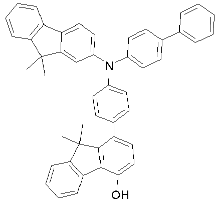 | 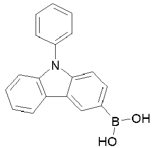 | 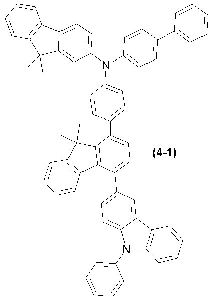 | 80% |
| 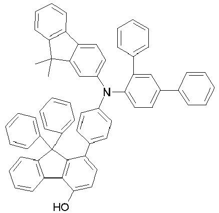 | 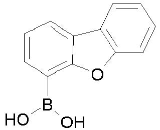 | 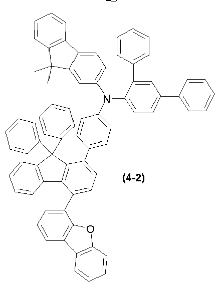 | 71% |
| 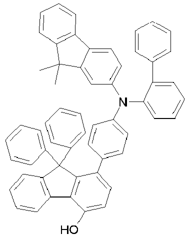 | 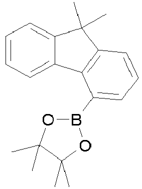 | 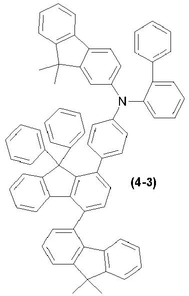 | 75% |

### Beispiel 5

### Vergleichsbeispiele

### Synthese der Vergleichsverbindungen (V3) und (V4)

Analog zu der in Beispiel 1 beschriebenen Synthese von Verbindung **(1-1)** werden auch die folgenden Verbindungen (V3) und (V4) hergestellt.

| **Edukt 1** | **Edukt 2** | **Produkt** | **Ausbeute** |
|---|---|---|---|
| 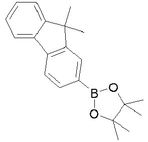 | 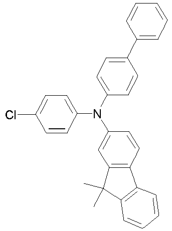 | 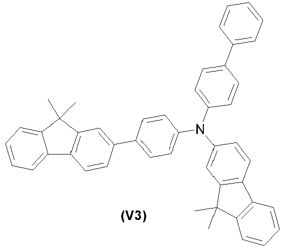 | 79% |
| 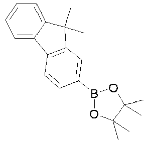 | 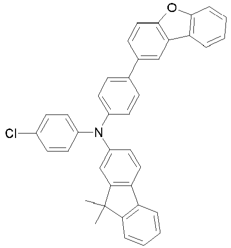 | 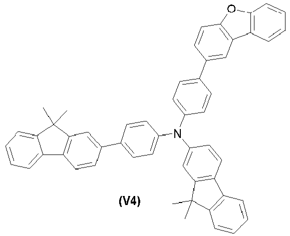 | 85% |

### Synthese der Vergleich Verbindungen (V1) und (V2)

Analog zu der in Beispiel 1 beschriebenen Synthese von **Zwischenstufe** werden auch die folgenden Verbindungen (**V1**) und (**V2**) hergestellt.

| **Edukt 1** | **Edukt 2** | **Produkt** | **Ausbeute** |
|---|---|---|---|
| 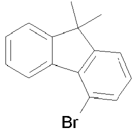 | 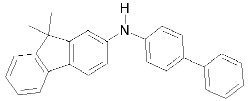 | 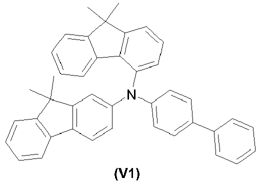 | 79% |
| 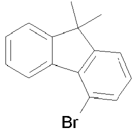 | 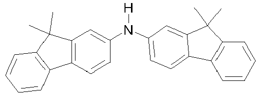 | 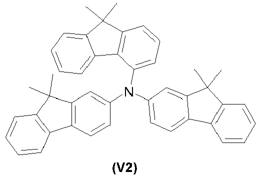 | 75% |

### Beispiel 6

### Herstellung der OLEDs

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (z.B. Materialien) angepasst wird.

In den folgenden erfinderischen Beispielen E1 bis E4 und in den Referenzbeispielen V1-V4 werden die Daten verschiedener OLEDs vorgestellt. Als Substrate werden Glasplättchen verwendet, die mit strukturiertem ITO (Indium Zinn Oxid) der Dicke 50 nm beschichtet sind. Die OLEDs haben prinzipiell folgenden Schichtaufbau: Substrat / p-dotierte Lochtransportschicht (HIL1) / Lochtransportschicht (HTL) / p-dotierte Lochtransportschicht (HIL2) / Lochtransportschicht (EBL) / Emissionsschicht (EML) / Elektronentransportschicht (ETL) / Elektroneninjektionsschicht (EIL) und abschließend eine Kathode. Die Kathode wird durch eine 100 nm dicke Aluminiumschicht gebildet. Die zur Herstellung der OLEDs benötigten Materialien sind in Tabelle 1 gezeigt, die verschiedenen Bauteilaufbauten in Tabelle 2.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H1:SEB(5%) bedeutet hierbei, dass das Material H1 in einem Volumenanteil von 95 % und SEB in einem Anteil von 5 % in der Schicht vorliegt. Analog kann auch die Elektronentransportschicht oder die Lochinjektionsschichten aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe EQE @ 10 mA/cm² bezeichnet die externe Quanteneffizienz bei einer Stromdichte von 10mA/cm². LD80 @ 60 mA/cm² ist die Lebensdauer bis das OLED bei einer Starthelligkeit bei konstantem Strom von 60 mA/cm² auf 80 % der Anfangsintensität abgefallen ist.

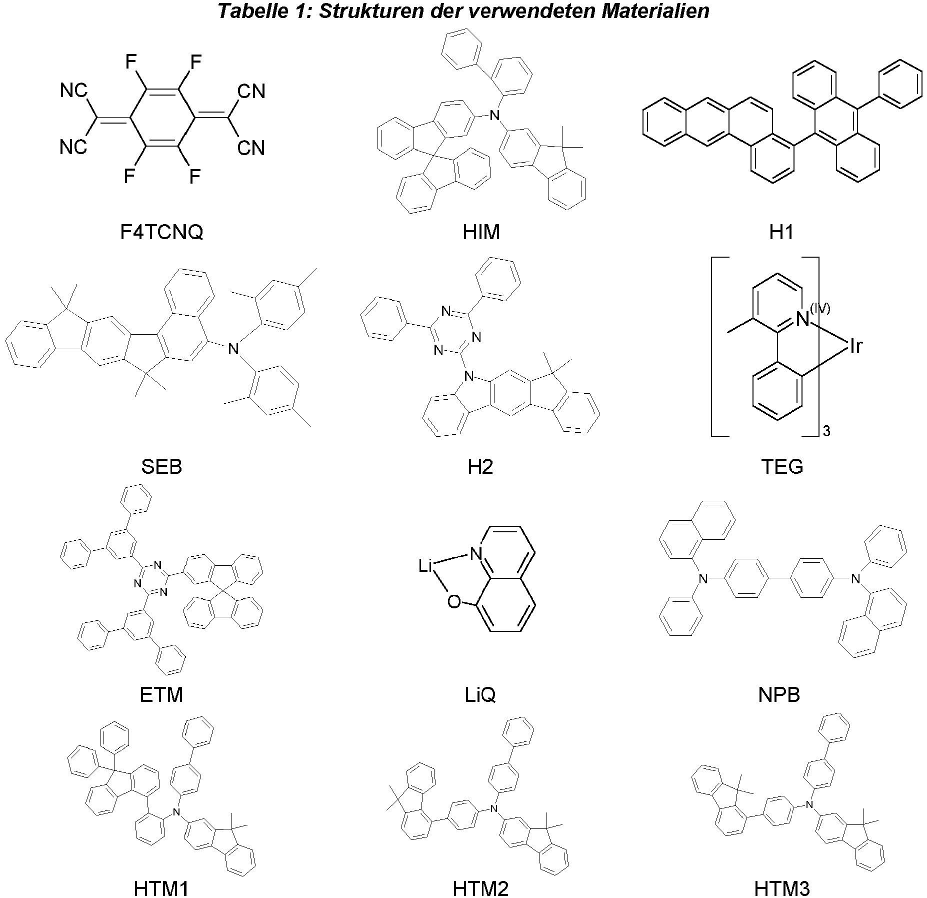

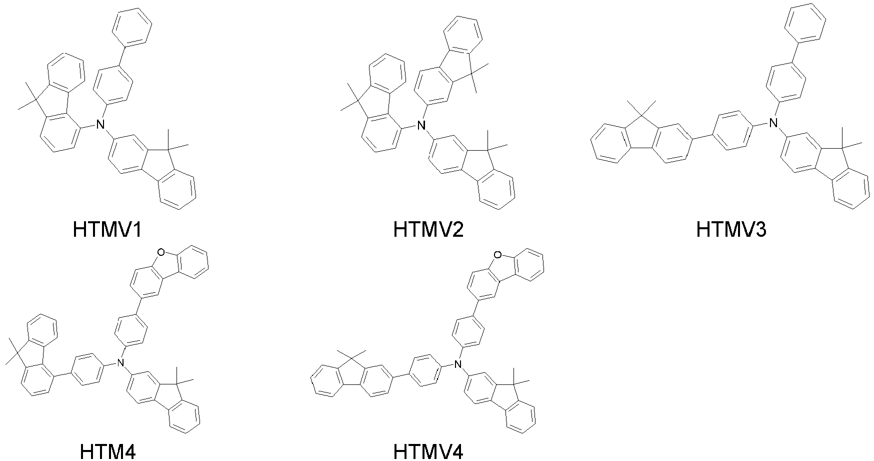

| ***Tabelle 2: Aufbau der OLEDs: HIL1(HIM.F4TCNQ(5%)-20nm)*/*HTL*/*HIL2*/*EBL*/*EML*/*ETL(ETM)*/*EIL(Liq-1 nm)*** | | | | | |
|---|---|---|---|---|---|
| ***Exp.*** | ***HTL*** | ***HIL2*** | ***EBL*** | ***EML*** | ***ETL*** |
| | *Dicke* / *nm* | *Dicke* / *nm* | *Dicke* / *nm* | *Dicke* / *nm* | *Dicke* / *nm* |
| *V1* | *HIM* | *NPB:F4TCNQ(5%)* | *NPB* | *H1:SEB(5%)* | *ETM:LiQ(50%)* |
| | *155 nm* | *20 nm* | *20 nm* | *20 nm* | *30 nm* |
| *E1* | *HIM* | *HTM1:F4TCNQ(5%)* | *HTM1* | *H1:SEB1(5%)* | *ETM:LiQ(50%)* |
| | *155 nm* | *20 nm* | *20 nm* | *20 nm* | *30 nm* |
| *E2* | *HIM* | *HTM2:F4TCNQ(5%)* | *HTM2* | *H1:SEB1(5%)* | *ETM:LiQ(50%)* |
| | *155 nm* | *20 nm* | *20 nm* | *20 nm* | *30 nm* |
| *E3* | *HIM* | *HTM3:F4TCNQ(5%)* | *HTM3* | *H1:SEB1(5%)* | *ETM:LiQ(50%)* |
| | *155 nm* | *20 nm* | *20 nm* | *20 nm* | *30 nm* |
| V2 | *HIM* | *HTMV1:F4TCNQ(5%)* | *HTMV1* | *H1:SEB1(5%)* | *ETM:LiQ(50%)* |
| | *155 nm* | *20 nm* | *20 nm* | *20 nm* | *30 nm* |
| *V3* | *HIM* | *HTMV2:F4TCNQ(5%)* | *HTMV2* | *H1:SEB1(5%)* | *ETM:LiQ(50%)* |
| | *155 nm* | *20 nm* | *20 nm* | *20 nm* | *30 nm* |
| *V4* | *HIM* | *HTMV3:F4TCNQ(5%)* | *HTMV3* | *H1:SEB1(5%)* | *ETM:LiQ(50%)* |
| | *155 nm* | *20 nm* | *20 nm* | *20 nm* | *30 nm* |
| *E4* | *HIM* | *HTM4:F4TCNQ(5%)* | *HTM4* | *H1:SEB1(5%)* | *ETM:LiQ(50%)* |
| | *155 nm* | *20 nm* | *20 nm* | *20 nm* | *30 nm* |
| *V5* | *HIM* | *HTMV4:F4TCNQ(5%)* | *HTMV4* | *H1:SEB1(5%)* | *ETM:LiQ(50%)* |
| | *155 nm* | *20 nm* | *20 nm* | *20 nm* | *30 nm* |
| *E5* | *HIM* | *HTM1:F4TCNQ(5%)* | *HTM1* | *H2:TEG(10%)* | *ETM:LiQ(50%)* |
| | *210 nm* | *20 nm* | *20 nm* | *30 nm* | *40 nm* |
| *E6* | *HIM* | *HTM2:F4TCNQ(5%)* | *HTM2* | *H2:TEG(10%)* | *ETM:LiQ(50%)* |
| | *210 nm* | *20 nm* | *20 nm* | *30 nm* | *40 nm* |
| *V6* | *HIM* | *NPB:F4TCNQ(5%)* | *NPB* | *H2:TEG(10%)* | *ETM:LiQ(50%)* |
| | *210 nm* | *20 nm* | *20 nm* | *30 nm* | *40 nm* |
| V7 | *HIM* | *HTMV1:F4TCNQ(5%)* | *HTMV1* | *H2:TEG(10%)* | *ETM:LiQ(50%)* |
| | *210 nm* | *20 nm* | *20 nm* | *30 nm* | *40 nm* |

In einem Singulett blau Bauteil zeigen im Vergleich zu den Referenzproben V1 (6,2 %), V2 (8,1 %), V3 (8,1%) und V4 (7,5%) die erfindungsgemäßen Proben E1 (8,5 %), E2 (9,4 %) und E3 (9,2%) eine höhere Quanteneffizienz bei 10 mA/cm² Die Lebensdauer LD80 bei 60 mA/cm² ist bei den erfindungsgemäßen Proben E1 (250 h), E2 (282 h) und E3 (260 h) deutlich besser als die Referenzproben V1 (125 h), V2 (216 h), V3 (170 h) und V4 (195 h).

In einem Singulett blau Bauteil zeigt im Vergleich zu der Referenzprobe V5 (8.3 %) die erfindungsgemäße Probe E4 (8,8 %) eine höhere Quanteneffizienz bei 10 mA/cm². Die Lebensdauer LD80 bei 60 mA/cm² ist bei der erfindungsgemäßen Probe E4 (240 h) deutlich besser als die Referenzproben V5 (195 h).

In einem Triplett grün Bauteil zeigen die Referenzproben V6 (11,7 %) und V7 (20,3 %) teils erheblich niedrigere Quanteneffizienzen bei 2 mA/cm² wie die erfindungsgemäßen Proben E5 (21,2 %) und E6 (21,8 %). Auch ist die Lebensdauer (80%) bei 20 mA/cm² der erfindungsgemäßen Proben E5 (115 h) und E6 (105 h) größer als bei V6 (80 h) und V7 (100 h).

## Patentansprüche

1. Verbindung der allgemeinen Formel (1) 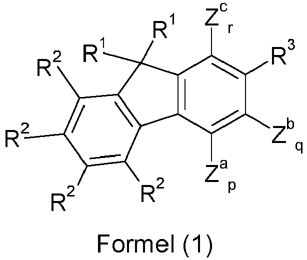 wobei für die verwendeten Symbole und Indices gilt:
R¹ ist bei jedem Auftreten gleich oder verschieden, bevorzugt gleich, H, D, F, Cl, Br, I, C(=O)R⁴, CN, Si(R⁴)₃, NO₂, P(=O)(R⁴)₂, S(=O)R⁴, S(=O)₂R⁴, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch -R⁴C=CR⁴-, -C≡C-, Si(R⁴)₂, C=O, C=S, C=NR⁴, -C(=O)O-, -C(=O)NR⁴-, P(=O)(R⁴), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R⁴ substituiert sein kann, oder eine Aryloxygruppe mit 5 bis 60 aromatischen Ringatomen, die mit einem oder mehreren Resten R⁴ substituiert sein kann, oder einer Aralkylgruppe mit 5 bis 60 aromatischen Ringatomen, die jeweils mit einem oder mehreren Resten R⁴ substituiert sein kann, wobei die beiden Reste R¹ miteinander verknüpft sein können und einen Ring bilden können, so dass eine Spiroverbindung in Position 9 des Fluorens entsteht, wobei Spirobifluorene ausgeschlossen sind;
R², R³ sind bei jedem Auftreten gleich oder verschieden, bevorzugt gleich, H, D, F, Cl, Br, I, C(=O)R⁴, CN, Si(R⁴)₃, NO₂, P(=O)(R⁴)₂, S(=O)R⁴, S(=O)₂R⁴, N(R⁴)₂, eine geradkettige Alkyl-, Alkoxyoder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch -R⁴C=CR⁴-, -C≡C-, Si(R⁴)₂, C=O, C=S, C=NR⁴, -C(=O)O-, -C(=O)NR⁴-, P(=O)(R⁴), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R⁴ substituiert sein kann, oder eine Aryloxygruppe mit 5 bis 60 aromatischen Ringatomen, die mit einem oder mehreren Resten R⁴ substituiert sein kann, oder einer Aralkylgruppe mit 5 bis 60 aromatischen Ringatomen, die jeweils mit einem oder mehreren Resten R⁴ substituiert sein kann, wobei zwei oder mehr Reste R² oder zwei oder mehr Reste R³ miteinander verknüpft sein können und einen Ring bilden können, bevorzugt sind sie nicht zu einem Ring verknüpft;
R⁴ ist bei jedem Auftreten gleich oder verschieden H, D, F, Cl, Br, I, C(=O)R⁵, CN, Si(R⁵)₃, NO₂, P(=O)(R⁵)₂, S(=O)R⁵, S(=O)₂R⁵, N(R⁵)₂, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁵ substituiert sein können und wobei eine oder mehrere CH₂-Gruppen in den oben genannten Gruppen durch-R⁵C=CR⁵-, -C≡C-, Si(R⁵)₂, C=O, C=S, C=NR⁵, -C(=O)O-, -C(=O)NR⁵-, P(=O)(R⁵), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R⁵ substituiert sein kann, oder eine Aryloxy- oder Heteroaryloxygruppe mit 5 bis 30 aromatischen Ringatomen, die durch einen oder mehrere Reste R⁵ substituiert sein kann;
R⁵ ist ausgewählt aus der Gruppe bestehend aus H, D, F, einem aliphatischem Kohlenwasserstoffrest mit 1 bis 20 C-Atomen oder einem aromatischem oder heteroaromatischen Ringsystem mit 5 bis 30 C-Atomen, in dem ein oder mehrere H-Atome durch D oder F ersetzt sein können, wobei zwei oder mehr benachbarte Substituenten R⁵ miteinander ein mono- oder polycyclisches, aliphatisches, Ringsystem bilden können;
p, q, sind 0 oder 1, wobei p + q + r = 1 gilt, bevorzugt ist p =1 oder r = 1 und ganz bevorzugt ist p = 1;
Z^{a}₀, Z^{b}₀, Z^{c}₀ sind gleich oder verschieden bei jedem Auftreten gleich R³
z^{a}₁, Z^{b}₁, Z^{c}₁ sind gleich 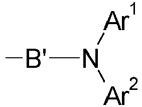
B' ist eine Arylengruppe mit 6 bis 30 Ringatomen oder eine Heteroarylengruppe mit 5 bis 30 Ringatomen, die jeweils mit einem oder mehreren Resten R⁴ substituiert sein können,
Ar¹, Ar² sind bei jedem Auftreten gleich oder verschieden ein Aromat mit 6 bis 60 Ringatomen oder ein Heteroaromat mit 5 bis 60 Ringatomen, welche mit einem oder mehreren Resten R⁶, die gleich oder verschieden voneinander sind, substituiert sein können; wobei sowohl die Gruppe Ar¹ als auch die Gruppe Ar² jeweils eine Fluorengruppe enthält;
R⁶ ist bei jedem Auftreten gleich oder verschieden H, D, F, Cl, Br, I, C(=O)R⁵, CN, Si(R⁵)₃, NO₂, P(=O)(R⁵)₂, S(=O)R⁵, S(=O)₂R⁵, eine geradkettige Alkyl-, Alkoxy- oder Thioalkylgruppe mit 1 bis 20 C-Atomen oder eine verzweigte oder cyclische Alkyl-, Alkoxyoder Thioalkylgruppe mit 3 bis 20 C-Atomen oder eine Alkenyloder Alkinylgruppe mit 2 bis 20 C-Atomen, wobei die oben genannten Gruppen jeweils mit einem oder mehreren Resten R⁵ substituiert sein können und wobei eine oder mehrere CH₂-Gruppen in den oben genannten Gruppen durch -R⁵C=CR⁵-, -C≡C-, Si(R⁵)₂, C=O, C=S, C=NR⁵, -C(=O)O-, -C(=O)NR⁵-, P(=O)(R⁵), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R⁵ substituiert sein kann, oder eine Aryloxy- oder Heteroaryloxygruppe mit 5 bis 30 aromatischen Ringatomen, die durch einen oder mehrere Reste R⁵ substituiert sein kann.

2. Verbindung gemäß Anspruch 1, **dadurch gekennzeichnet, dass** sie die allgemeine Formel (1a) hat 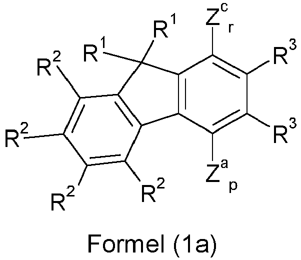 wobei für die verwendeten Symbole die Definitionen aus Anspruch 1 gelten.

3. Verbindung gemäß Anspruch 1, **dadurch gekennzeichnet, dass** sie die allgemeine Formel (2) hat 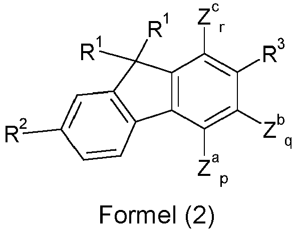 wobei für die verwendeten Symbole die Definitionen aus Anspruch 1 gelten.

4. Verbindung gemäß einem oder mehreren der Ansprüche 1 bis 3, **dadurch gekennzeichnet, dass** sie die allgemeine Formel (3) hat 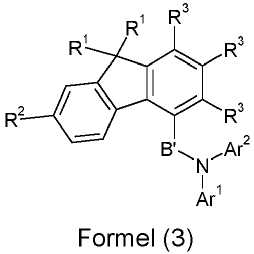 wobei für die verwendeten Symbole die Definitionen aus Anspruch 1 gelten.

5. Verbindung gemäß einem oder mehreren der Ansprüche 1 bis 3, **dadurch gekennzeichnet, dass** sie die allgemeine Formel (5) hat 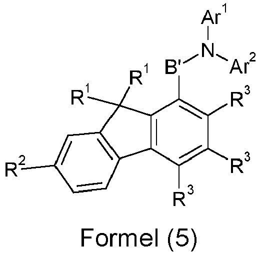 wobei für die verwendeten Symbole die Definitionen aus Anspruch 1 gelten.

6. Verbindung gemäß einem oder mehreren der Ansprüche 1 bis 5, **dadurch gekennzeichnet, dass** beide R¹ identisch sind.

7. Verbindung gemäß einem oder mehreren der Ansprüche 1 bis 6, **dadurch gekennzeichnet, dass** B' eine eine Phenylen-, Biphenylen-, Terphenylen-, Naphthylen-, Pyridinylen-, Pyrimidinylen-, Pyrazinylen-, Pyridazinylen-, Triazinylen-, Fluorenylen-, Dibenzofuranylen- oder Dibenzothiophenylengruppe ist, die mit einem oder mehreren Resten R⁴ substituiert sein kann.

8. Verbindung gemäß einem oder mehreren der Ansprüche 1 bis 7, **dadurch gekennzeichnet, dass** B' eine Phenylen-, Biphenylen-, Terphenylen-, Naphthylen-, Dibenzofuranylen- oder Dibenzothiophenylengruppe ist, die mit einem oder mehreren Resten R⁴ substituiert sein kann, bevorzugt ist B' eine Phenylengruppe, die mit einem oder mehreren Resten R⁴ substituiert sein kann und ganz bevorzugt ist B' eine unsubstituierte Phenylengruppe.

9. Verbindung gemäß einem oder mehreren der Ansprüche 1 bis 8, **dadurch gekennzeichnet, dass** die Verbidung die allgemeine Formel (123) aufweist, wobei wobei x gleich 0, 1, 2, 3 oder 4 ist und wobei y gleich 0, 1, 2 oder 3 ist

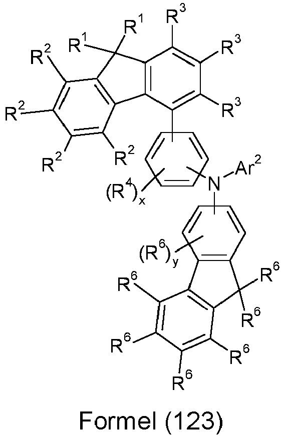

10. Verbindung gemäß einem oder mehreren der Ansprüche 1 bis 9, **dadurch gekennzeichnet, dass** die Verbindung die allgemeine Formel (124) aufweist.

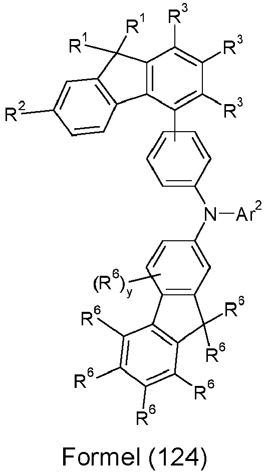

11. Verbindung gemäß einem oder mehreren der Ansprüche 1 bis 9, **dadurch gekennzeichnet, dass** die Verbindung die allgemeine Formel (124b) aufweist.

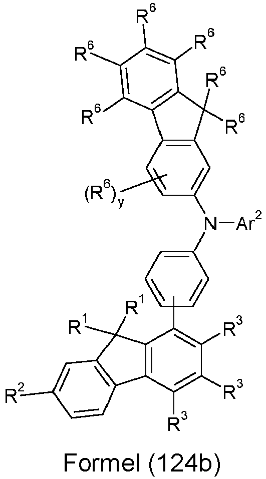

12. Verbindung gemäß einem oder mehreren der Ansprüche 1 bis 11, **dadurch gekennzeichnet, dass** beide Gruppen Ar¹ und Ar² mindestens zwei aromatische oder heteroaromatische Ringe enthalten.

13. Verfahren zur Herstellung einer Verbindung nach einem oder mehreren der Ansprüche 1 bis 12 mittels einstufiger Buchwald Kupplung durch Umsetzung eines Fluorenderivats, das eine Abgangsgruppe enthält, mit Ar²-NH-Ar¹.

14. Verfahren zur Herstellung einer Verbindung gemäß einem oder mehreren der Ansprüche 1 bis 12 mittels zweistufiger Buchwald Kupplung durch stufenweise Umsetzung eines Fluorenderivats, das eine Abgangsgruppe enthält, mit (1) Ar²-NH₂ und (2) NH₂-Ar¹.

15. Verfahren zur Herstellung einer Verbindung gemäß einem oder mehrere der Ansprüche 1 bis 12, **dadurch gekennzeichnet, dass** die Verbindung aus einem Benzochromen-6-on hergestellt wird.

16. Verfahren gemäß Anspruch 15 umfassend die folgenden Schritte:
a) Addition einer organometallischen Verbindung an ein Benzochromen-6-on und anschließende
b) säurekatalysierte Cyclisierung zur einem 4-Hydroxy-Fluorenderivat und anschließende
c) Umwandlung der Hydroxygruppe in Position 4 des Fluorens in eine Abgangsgruppe und anschließende
d) Umsetzung des Fluorens in das gewünschte Produkt.

17. Oligomer, Polymer oder Dendrimer enthaltend eine oder mehrere Verbindungen gemäß einem oder mehreren der Ansprüche 1 bis 12, wobei die Bindung(en) zum Polymer, Oligomer oder Dendrimer an beliebigen, in Formel (1) mit R¹ bis R⁶ substituierten Positionen lokalisiert sein können.

18. Zusammensetzung enthaltend eine oder mehrere Verbindung gemäß einem oder mehreren der Ansprüche 1 bis 12 oder ein oder mehrere Polymerre, Oligomere oder Dendrimere gemäß Anspruch 17 sowie wenigstens ein weiteres Material ausgewählt aus der Gruppe bestehend aus fluoreszierenden Emitter, phosphoreszierenden Emitter, Host Materialien, Matrix Materialien, Elektronentransportmaterialien, Elektroneninjektionsmaterialien, Lochleitermaterialien, Lochinjektionsmaterialien, Elektronenblockiermaterialien, Lochblockiermaterialien und p-Dotanden.

19. Formulierung enthaltend mindestens eine Verbindung gemäß einem oder mehreren der Ansprüche 1 bis 12 oder mindestens ein Polymer, Oligomer oder Dendrimer gemäß Anspruch 17 oder mindestens eine Zusammensetzung nach Anspruch 18 sowie mindestens ein Lösungsmittel.

20. Verwendung wenigstens einer Verbindung gemäß einem oder mehreren der Ansprüche 1 bis 12 oder eines Polymers, Oligomers oder Dendrimers gemäß Anspruch 17 in einer elektronischen Vorrichtung, bevorzugt in einer organischen Elektrolumineszenzvorrichtung, ganz bevorzugt in einer organischen lichtemittierenden Diode (OLED) oder organischen lichtemittierenden elektrochemischen Zelle (OLEC, LEEC, LEC), ganz besonders bevorzugt in einer OLED, bevorzugt in einer Emissionsschicht (EML), Elektronentransportschicht (ETL) und in einer Lochblockierschicht (HBL), ganz bevorzugt in einer EML und ETL und ganz besonders bevorzugt in einer EML.

21. Elektronische Vorrichtung, enthaltend mindestens eine Verbindung gemäß einem oder mehreren der Ansprüche 1 bis 12 oder mindestens ein Polymer, Oligomer oder Dendrimer gemäß Anspruch 17 oder mindestens eine Zusammensetzung gemäß Anspruch 18.

22. Elektronische Vorrichtung gemäß Anspruch 21, **dadurch gekennzeichnet, dass** sie gewählt ist aus organischen integrierten Schaltungen (OICs), organischen Feld-Effekt-Transistoren (OFETs), organischen Dünnfilmtransistoren (OTFTs), organischen Elektrolumineszenzvorrichtungen, organischen Solarzellen (OSCs), organischen optischen Detektoren, organischen Photorezeptoren, bevorzugt eine organische Elektroluminesszenzvorrichtung.

23. Elektronische Vorrichtung gemäß Anspruch 21 oder 22 **dadurch gekennzeichnet, dass** sie eine organische Elektrolumineszenzvorrichtung ist, die ausgewählt ist auch der Gruppe bestehend aus organischen lichtemittierenden Transistoren (OLETs), organischen Feld-Quench-Devices (OFQDs), organischen lichtemittierenden elektrochemischen Zellen (OLECs, LECs, LEECs), organischen Laserdioden (O-Laser) und organischen lichtemittierenden Dioden (OLEDs), bevorzugt OLECs und OLEDs, ganz bevorzugt OLEDs.

24. Elektronische Vorrichtung gemäß einem oder meherern der Ansprüche 21 bis 23, **dadurch gekennzeichnet, dass** die Verbindung gemäß einem oder mehreren der Ansprüche 1 bis 12 oder das Polymer, Oligomer oder Dendrimer gemäß Anspruch 17 oder eine Zusammensetzung nach Anspruch 18 in einer oder mehreren der folgenden Funktionen eingesetzt wird:
- als Lochtransportmaterial in einer Lochtransport- oder Lochinjektionsschicht,
- als Matrixmaterial in einer emittierenden Schicht,
- als Elektronenblockiermaterial,
- als Excitonenblockiermaterial
wobei die Verwendung als als Lochtransportmaterial in einer Lochtransport- oder Lochinjektionsschicht bevorzugt ist.

25. Verfahren zur Herstellung einer elektronischen Vorrichtung gemäß einem oder mehreren der Ansprüche 21 bis 24, **dadurch gekennzeichnet, dass** mindestens eine organische Schicht durch Gasphasenabscheidung oder aus Lösung aufgebracht wird.

26. Elektronische Vorrichtung gemäß Anspruch 23, zur Verwendung in der Medizin zur Phototherapie, bevorzugt zur Verwendung zur Phototherapie der Haut, ganz bevorzugt zur Verwendung zur Behandlung oder Vorbeugung von Psoriasis, atopischer Dermatitis, Ikterus, Neugeborenenikterus, Vitiligo, Inflammation, Schwerzen und zur Verwendung zur Wundheilung.

27. Verwendung der Vorrichtung gemäß Anspruch 23 in der Kosmetik, bevorzugt zur Reduktion oder Verhinderung von Hautalterung (Skin Ageing), Hautfalten, Krähenfüsse, Akne, Komedonen und Zellulithe.

28. Verwendung der Vorrichtung gemäß Anspruch 23 in Displays oder zur Beleuchtung.

## Claims

1. Compound of the general formula (1) 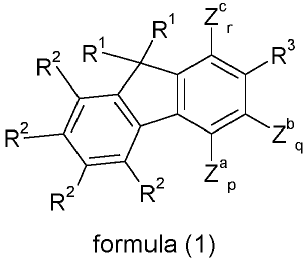 where the following applies to the symbols and indices used:
R¹ is on each occurrence, identically or differently, preferably identically, H, D, F, Cl, Br, I, C(=O)R⁴, CN, Si(R⁴)₃, NO₂, P(=O)(R⁴)₂, S(=O)R⁴, S(=O)₂R⁴, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-men-tioned groups may in each case be substituted by one or more radicals R⁴ and where one or more CH₂ groups in the abovementioned groups may be replaced by -R⁴C=CR⁴-, -C≡C-, Si(R⁴)₂, C=O, C=S, C=NR⁴, -C(=O)O-, -C(=O)NR⁴-, P(=O)(R⁴), -O-, -S-, SO or SO₂ and where one or more H atoms in the above-mentioned groups may be replaced by D, F, Cl, Br, I, CN or NO₂, or an aromatic or heteroaromatic ring system having 5 to 30 aromatic ring atoms, which may in each case be substituted by one or more radicals R⁴, or an aryloxy group having 5 to 60 aromatic ring atoms, which may be substituted by one or more radicals R⁴, or an aralkyl group having 5 to 60 aromatic ring atoms, which may in each case be substituted by one or more radicals R⁴, where the two radicals R¹ may be linked to one another and may form a ring, so that a spiro compound forms in position 9 of the fluorene, where spirobifluorenes are excluded;
R², R³ are on each occurrence, identically or differently, preferably identically, H, D, F, Cl, Br, I, C(=O)R⁴, CN, Si(R⁴)₃, NO₂, P(=O)-(R⁴)₂, S(=O)R⁴, S(=O)₂R⁴, N(R⁴)₂, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may in each case be substituted by one or more radicals R⁴ and where one or more CH₂ groups in the above-mentioned groups may be replaced by -R⁴C=CR⁴-, -C≡C-, Si(R⁴)₂, C=O, C=S, C=NR⁴, -C(=O)O-, -C(=O)NR⁴-, P(=O)(R⁴), -O-, -S-, SO or SO₂ and where one or more H atoms in the above-mentioned groups may be replaced by D, F, Cl, Br, I, CN or NO₂, or an aromatic or heteroaromatic ring system having 5 to 30 aromatic ring atoms, which may in each case be substituted by one or more radicals R⁴, or an aryloxy group having 5 to 60 aromatic ring atoms, which may be substituted by one or more radicals R⁴, or an aralkyl group having 5 to 60 aromatic ring atoms, which may in each case be substituted by one or more radicals R⁴, where two or more radicals R² or two radicals R³ may be linked to one another and may form a ring, they are preferably not linked to form a ring;
R⁴ is on each occurrence, identically or differently, H, D, F, Cl, Br, I, C(=O)R⁵, CN, Si(R⁵)₃, NO₂, P(=O)(R⁵)₂, S(=O)R⁵, S(=O)₂R⁵, N(R⁵)₂, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may in each case be substituted by one or more radicals R⁵ and where one or more CH₂ groups in the above-mentioned groups may be replaced by -R⁵C=CR⁵-, -C=C-, Si(R⁵)₂, C=O, C=S, C=NR⁵, -C(=O)O-, -C(=O)NR⁵-, P(=O)(R⁵), -O-, -S-, SO or SO₂ and where one or more H atoms in the above-mentioned groups may be replaced by D, F, Cl, Br, I, CN or NO₂, or an aromatic or heteroaromatic ring system having 5 to 30 aromatic ring atoms, which may in each case be substituted by one or more radicals R⁵, or an aryloxy or heteroaryloxy group having 5 to 30 aromatic ring atoms, which may be substituted by one or more radicals R⁵;
R⁵ is selected from the group consisting of H, D, F, an aliphatic hydrocarbon radical having 1 to 20 C atoms or an aromatic or heteroaromatic ring system having 5 to 30 C atoms, in which one or more H atoms may be replaced by D or F, where two or more adjacent substituents R⁵ may form a mono- or polycyclic, aliphatic ring system with one another;
p, q, r are 0 or 1, where p + q + r = 1, preferably p = 1 or r = 1 and very preferably p = 1;
Z^{a}₀, Z^{b}₀, Z^{c}₀ are, identically or differently on each occurrence, equal to R³;
z^{a}₁, Z^{b}₁, Z^{c}₁ are equal to 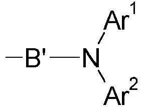
B' is an arylene group having 6 to 30 ring atoms or a heteroarylene group having 5 to 30 ring atoms, which may in each case be substituted by one or more radicals R⁴;
Ar¹, Ar² are on each occurrence, identically or differently, an aromatic group having 6 to 60 ring atoms or a heteroaromatic group having 5 to 60 ring atoms, which may in each case be substituted by one or more radicals R⁶, which are identical to or different from one another; where at least one of the two groups Ar¹ and Ar² must contain a fluorene group;
R⁶ is on each occurrence, identically or differently, H, D, F, Cl, Br, I, C(=O)R⁵, CN, Si(R⁵)₃, NO₂, P(=O)(R⁵)₂, S(=O)R⁵, S(=O)₂R⁵, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may in each case be substituted by one or more radicals R⁵ and where one or more CH₂ groups in the above-mentioned groups may be replaced by -R⁵C=CR⁵-, -C=C-, Si(R⁵)₂, C=O, C=S, C=NR⁵, -C(=O)O-, -C(=O)NR⁵-, P(=O)(R⁵), -O-, -S-, SO or SO₂ and where one or more H atoms in the above-mentioned groups may be replaced by D, F, Cl, Br, I, CN or NO₂, or an aromatic or heteroaromatic ring system having 5 to 30 aromatic ring atoms, which may in each case be substituted by one or more radicals R⁵, or an aryloxy or heteroaryloxy group having 5 to 30 aromatic ring atoms, which may be substituted by one or more radicals R⁵.

2. Compound according to Claim 1, **characterised in that** it has the general formula (1a) 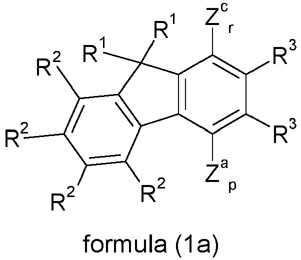 where the definitions from Claim 1 apply to the symbols used.

3. Compound according to Claim 1, **characterised in that** it has the general formula (2) 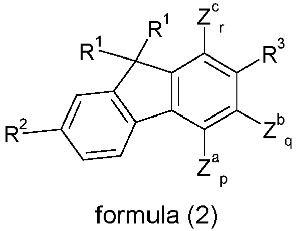 where the definitions from Claim 1 apply to the symbols used.

4. Compound according to one or more of Claims 1 to 3, **characterised in that** it has the general formula (3) 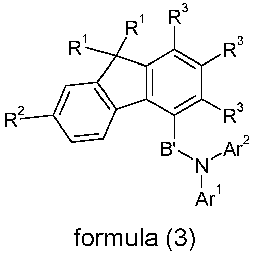 where the definitions from Claim 1 apply to the symbols used.

5. Compound according to one or more of Claims 1 to 3, **characterised in that** it has the general formula (5) 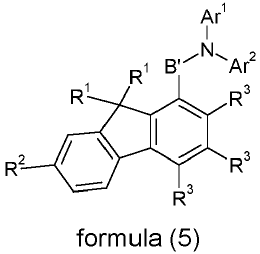 where the definitions from Claim 1 apply to the symbols used.

6. Compound according to one or more of Claims 1 to 5, **characterised in that** both R¹ are identical.

7. Compound according to one or more of Claims 1 to 6, **characterised in that** B' is a phenylene, biphenylene, terphenylene, naphthylene, pyridinylene, pyrimidinylene, pyrazinylene, pyridazinylene, triazinylene, fluorenylene, dibenzofuranylene or dibenzothiophenylene group, which may be substituted by one or more radicals R⁴.

8. Compound according to one or more of Claims 1 to 7, **characterised in that** B' is a phenylene, biphenylene, terphenylene, naphthylene, dibenzofuranylene or dibenzothiophenylene group, which may be substituted by one or more radicals R⁴, B' is preferably a phenylene group, which may be substituted by one or more radicals R⁴, and B' is very preferably an unsubstituted phenylene group.

9. Compound according to one or more of Claims 1 to 8, **characterised in that** the compound has the general formula (123), where x is equal to 0, 1, 2, 3 or 4 and where y is equal to 0, 1, 2 or 3

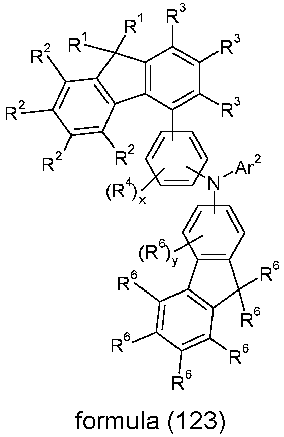

10. Compound according to one or more of Claims 1 to 9, **characterised in that** the compound has the general formula (124)

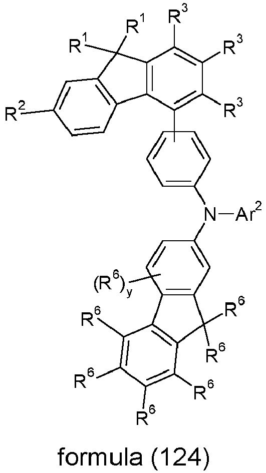

11. Compound according to one or more of Claims 1 to 9, **characterised in that** the compound has the general formula (124b)

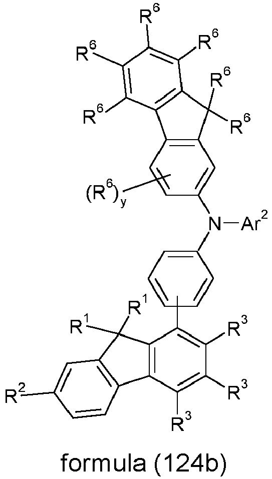

12. Compound according to one or more of Claims 1 to 11, **characterised in that** both groups Ar¹ and Ar² contain at least two aromatic or heteroaromatic rings.

13. Process for the preparation of a compound according to one or more of Claims 1 to 12 by means of one-step Buchwald coupling by reaction of a fluorene derivative containing a leaving group with Ar²-NH-Ar¹.

14. Process for the preparation of a compound according to one or more of Claims 1 to 12 by means of two-step Buchwald coupling by stepwise reaction of a fluorene derivative containing a leaving group with (1) Ar²-NH₂ and (2) NH₂-Ar¹.

15. Process for the preparation of a compound according to one or more of Claims 1 to 12, **characterised in that** the compound is prepared from a benzochromen-6-one.

16. Process according to Claim 15 comprising the following steps:
a) addition of an organometallic compound onto a benzochromen-6-one and subsequent
b) acid-catalysed cyclisation to give a 4-hydroxyfluorene derivative and subsequent
c) conversion of the hydroxyl group in position 4 of the fluorene into a leaving group and subsequent
d) conversion of the fluorene into the desired product.

17. Oligomer, polymer or dendrimer containing one or more compounds according to one or more of Claims 1 to 12, where the bond(s) to the polymer, oligomer or dendrimer may be localised at any desired positions in formula (1) that are substituted by R¹ to R⁶.

18. Composition comprising one or more compounds according to one or more of Claims 1 to 12 or one or more polymers, oligomers or dendrimers according to Claim 17 and at least one further material selected from the group consisting of fluorescent emitters, phosphorescent emitters, host materials, matrix materials, electron-transport materials, electron-injection materials, hole-conductor materials, hole-injection materials, electron-blocking materials, hole-blocking materials and p-dopants.

19. Formulation comprising at least one compound according to one or more of Claims 1 to 12 or at least one polymer, oligomer or dendrimer according to Claim 17 or at least one composition according to Claim 18 and at least one solvent.

20. Use of at least one compound according to one or more of Claims 1 to 12 or a polymer, oligomer or dendrimer according to Claim 17 in an electronic device, preferably in an organic electroluminescent device, very preferably in an organic light-emitting diode (OLED) or organic light-emitting electrochemical cell (OLEC, LEEC, LEC), very particularly preferably in an OLED, preferably in an emission layer (EML), electron-transport layer (ETL) and in a hole-blocking layer (HBL), very preferably in an EML and ETL and very particularly preferably in an EML.

21. Electronic device comprising at least one compound according to one or more of Claims 1 to 12 or at least one polymer, oligomer or dendrimer according to Claim 17 or at least one composition according to Claim 18.

22. Electronic device according to Claim 21, **characterised in that** it is selected from organic integrated circuits (OICs), organic field-effect transistors (OFETs), organic thin-film transistors (OTFTs), organic electroluminescent devices, organic solar cells (OSCs), organic optical detectors, organic photoreceptors, preferably an organic electroluminescent device.

23. Electronic device according to Claim 21 or 22, **characterised in that** it is an organic electroluminescent device which is also selected from the group consisting of organic light-emitting transistors (OLETs), organic field-quench devices (OFQDs), organic light-emitting electrochemical cells (OLECs, LECs, LEECs), organic laser diodes (O-lasers) and organic light-emitting diodes (OLEDs), preferably OLECs and OLEDs, very preferably OLEDs.

24. Electronic device according to one or more of Claims 21 to 23, **characterised in that** the compound according to one or more of Claims 1 to 12 or the polymer, oligomer or dendrimer according to Claim 17 or a composition according to Claim 18 is employed in one or more of the following functions:
- as hole-transport material in a hole-transport or hole-injection layer,
- as matrix material in an emitting layer,
- as electron-blocking material,
- as exciton-blocking material,
where the use as hole-transport material in a hole-transport or hole-injection layer is preferred.

25. Process for the production of an electronic device according to one or more of Claims 21 to 24, **characterised in that** at least one organic layer is applied by gas-phase deposition or from solution.

26. Electronic device according to Claim 23, for use in medicine for phototherapy, preferably for use for phototherapy of the skin, very preferably for use for the treatment or prevention of psoriasis, atopic dermatitis, jaundice, jaundice of the newborn, vitiligo, inflammation, pain and for use for wound healing.

27. Use of the device according to Claim 23 in the cosmetics field, preferably for the reduction or prevention of skin ageing, wrinkles, crow's feet, acne, comedones and cellulite.

28. Use of the device according to Claim 23 in displays or for lighting.

## Revendications

1. Composé de la formule générale (1) : 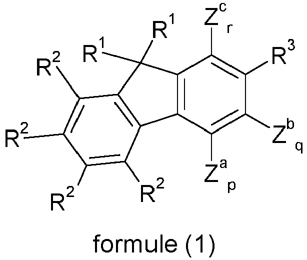 dans laquelle ce qui suit s'applique aux symboles et indices qui sont utilisés :
R¹ est pour chaque occurrence, de manière identique ou différente, de préférence de manière identique, H, D, F, Cl, Br, I, C(=O)R⁴, CN, Si(R⁴)₃, NO₂, P(=O)(R⁴)₂, S(=O)R⁴, S(=O)₂R⁴, un groupe alkyle, alcoxy ou thioalkyle en chaîne droite qui comporte 1 à 20 atome(s) de C ou un groupe alkyle, alcoxy ou thioalkyle ramifié ou cyclique qui comporte 3 à 20 atomes de C ou un groupe alkényle ou alkynyle qui comporte 2 à 20 atomes de C, dans lequel les groupes qui ont été mentionnés ci-avant peuvent dans chaque cas être substitués par un radical ou par plusieurs radicaux R⁴ et dans lequel un ou plusieurs groupe(s) CH₂ dans les groupes qui ont été mentionnés ci-avant peut/peuvent être remplacé(s) par -R⁴C=CR⁴-, -C≡C-, Si(R⁴)₂, C=O, C=S, C=NR⁴, -C(=O)O-, -C(=O)NR⁴-, P(=O)(R⁴), -O-, -S-, SO ou SO₂ et dans lequel un ou plusieurs atome(s) de H dans les groupes qui ont été mentionnés ci-avant peut/peuvent être remplacé(s) par D, F, Cl, Br, I, CN ou NO₂, ou un système de cycle aromatique ou hétéroaromatique qui comporte 5 à 30 atomes de cycle aromatique, lequel peut dans chaque cas être substitué par un radical ou par plusieurs radicaux R⁴, ou un groupe aryloxy qui comporte 5 à 60 atomes de cycle aromatique, lequel peut être substitué par un radical ou par plusieurs radicaux R⁴, ou un groupe aralkyle qui comporte 5 à 60 atomes de cycle aromatique, lequel peut dans chaque cas être substitué par un radical ou par plusieurs radicaux R⁴, dans lequel les deux radicaux R¹ peuvent être liés l'un à l'autre et peuvent former un cycle, de telle sorte qu'un composé spiro se forme à la position 9 du fluorène, dans lequel les spirobifluorènes sont exclus ;
R², R³ sont pour chaque occurrence, de manière identique ou différente, de préférence de manière identique, H, D, F, Cl, Br, I, C(=O)R⁴, CN, Si(R⁴)₃, NO₂, P(=O)(R⁴)₂, S(=O)R⁴, S(=O)₂R⁴, N(R⁴)₂, un groupe alkyle, alcoxy ou thioalkyle en chaîne droite qui comporte 1 à 20 atome(s) de C ou un groupe alkyle, alcoxy ou thioalkyle ramifié ou cyclique qui comporte 3 à 20 atomes de C ou un groupe alkényle ou alkynyle qui comporte 2 à 20 atomes de C, dans lequel les groupes qui ont été mentionnés ciavant peuvent dans chaque cas être substitués par un radical ou par plusieurs radicaux R⁴ et dans lequel un ou plusieurs groupe(s) CH₂ dans les groupes qui ont été mentionnés ci-avant peut/peuvent être remplacé(s) par -R⁴C=CR⁴-, -C=C-, Si(R⁴)₂, C=O, C=S, C=NR⁴, -C(=O)O-, -C(=O)NR⁴-, P(=O)(R⁴), -O-, -S-, SO ou SO₂ et dans lequel un ou plusieurs atome(s) de H dans les groupes qui ont été mentionnés ci-avant peut/peuvent être remplacé(s) par D, F, Cl, Br, I, CN ou NO₂, ou un système de cycle aromatique ou hétéroaromatique qui comporte 5 à 30 atomes de cycle aromatique, lequel peut dans chaque cas être substitué par un radical ou par plusieurs radicaux R⁴, ou un groupe aryloxy qui comporte 5 à 60 atomes de cycle aromatique, lequel peut être substitué par un radical ou par plusieurs radicaux R⁴, ou un groupe aralkyle qui comporte 5 à 60 atomes de cycle aromatique, lequel peut dans chaque cas être substitué par un radical ou par plusieurs radicaux R⁴, dans lequel deux radicaux R² ou plus ou deux radicaux R³ peuvent être liés l'un à l'autre ou les uns aux autres et peuvent former un cycle, ils sont de préférence non liés pour former un cycle ;
R⁴ est pour chaque occurrence, de manière identique ou différente, H, D, F, Cl, Br, I, C(=O)R⁵, CN, Si(R⁵)₃, NO₂, P(=O)(R⁵)₂, S(=O)R⁵, S(=O)₂R⁵, N(R⁵)₂, un groupe alkyle, alcoxy ou thioalkyle en chaîne droite qui comporte 1 à 20 atome(s) de C ou un groupe alkyle, alcoxy ou thioalkyle ramifié ou cyclique qui comporte 3 à 20 atomes de C ou un groupe alkényle ou alkynyle qui comporte 2 à 20 atomes de C, dans lequel les groupes qui ont été mentionnés ci-avant peuvent dans chaque cas être substitués par un radical ou par plusieurs radicaux R⁵ et dans lequel un ou plusieurs groupe(s) CH₂ dans les groupes qui ont été mentionnés ci-avant peut/peuvent être remplacé(s) par -R⁵C=CR⁵-, -C=C-, Si(R⁵)₂, C=O, C=S, C=NR⁵, -C(=O)O-, -C(=O)NR⁵-, P(=O)(R⁵), -O-, -S-, SO ou SO₂ et dans lequel un ou plusieurs atome(s) de H dans les groupes qui ont été mentionnés ci-avant peut/peuvent être remplacé(s) par D, F, Cl, Br, I, CN ou NO₂, ou un système de cycle aromatique ou hétéroaromatique qui comporte 5 à 30 atomes de cycle aromatique, lequel peut dans chaque cas être substitué par un radical ou par plusieurs radicaux R⁵, ou un groupe aryloxy ou hétéroaryloxy qui comporte 5 à 30 atomes de cycle aromatique, lequel peut être substitué par un radical ou par plusieurs radicaux R⁵ ;
R⁵ est sélectionné parmi le groupe qui est constitué par H, D, F, un radical hydrocarbone aliphatique qui comporte 1 à 20 atome(s) de C ou un système de cycle aromatique ou hétéroaromatique qui comporte 5 à 30 atomes de C, dans lequel un ou plusieurs atome(s) de H peut/peuvent être remplacé(s) par D ou F, dans lequel deux substituants R⁵ adjacents ou plus peuvent former un système de cycle aliphatique monocyclique ou polycyclique l'un avec l'autre ou les uns avec les autres ;
p, q, r sont 0 ou 1, où p + q + r = 1, de préférence p = 1 ou r = 1 et de façon très préférable p = 1 ;
Z^{a}₀, Z^{b}₀, Z^{c}₀ sont, de manière identique ou différente pour chaque occurrence, égaux à R³ ;
Z^{a}₁, Z^{b}₁, Z^{c}₁ sont égaux à 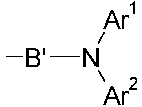
B' est un groupe arylène qui comporte 6 à 30 atomes de cycle ou un groupe hétéroarylène qui comporte 5 à 30 atomes de cycle, lequel peut dans chaque cas être substitué par un radical ou par plusieurs radicaux R⁴ ;
Ar¹, Ar² sont pour chaque occurrence, de manière identique ou différente, un groupe aromatique qui comporte 6 à 60 atomes de cycle ou un groupe hétéroaromatique qui comporte 5 à 60 atomes de cycle, lequel peut dans chaque cas être substitué par un radical ou par plusieurs radicaux R⁶, lesquels sont identiques l'un à l'autre ou sont différents l'un de l'autre ; où au moins l'un des deux groupes Ar¹ et Ar² doit contenir un groupe fluorène ;
R⁶ est pour chaque occurrence, de manière identique ou différente, H, D, F, Cl, Br, I, C(=O)R⁵, CN, Si(R⁵)₃, NO₂, P(=O)(R⁵)₂, S(=O)R⁵, S(=O)₂R⁵, un groupe alkyle, alcoxy ou thioalkyle en chaîne droite qui comporte 1 à 20 atome(s) de C ou un groupe alkyle, alcoxy ou thioalkyle ramifié ou cyclique qui comporte 3 à 20 atomes de C ou un groupe alkényle ou alkynyle qui comporte 2 à 20 atomes de C, dans lequel les groupes qui ont été mentionnés ci-avant peuvent dans chaque cas être substitués par un radical ou par plusieurs radicaux R⁵ et dans lequel un ou plusieurs groupe(s) CH₂ dans les groupes qui ont été mentionnés ci-avant peut/peuvent être remplacé(s) par -R⁵C=CR⁵-, -C=C-, Si(R⁵)₂, C=O, C=S, C=NR⁵, -C(=O)O-, -C(=O)NR⁵-, P(=O)(R⁵), -O-, -S-, SO ou SO₂ et dans lequel un ou plusieurs atome(s) de H dans les groupes qui ont été mentionnés ci-avant peut/ peuvent être remplacé(s) par D, F, Cl, Br, I, CN ou NO₂, ou un système de cycle aromatique ou hétéroaromatique qui comporte 5 à 30 atomes de cycle aromatique, lequel peut dans chaque cas être substitué par un radical ou par plusieurs radicaux R⁵, ou un groupe aryloxy ou hétéroaryloxy qui comporte 5 à 30 atomes de cycle aromatique, lequel peut être substitué par un radical ou par plusieurs radicaux R⁵.

2. Composé selon la revendication 1, **caractérisé en ce qu'**il présente la formule générale (1a) 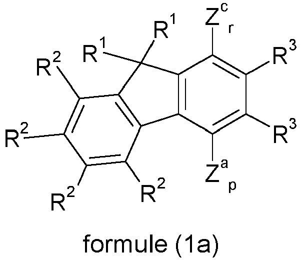 dans laquelle les définitions de la revendication 1 s'appliquent aux symboles qui sont utilisés.

3. Composé selon la revendication 1, **caractérisé en ce qu'**il présente la formule générale (2) 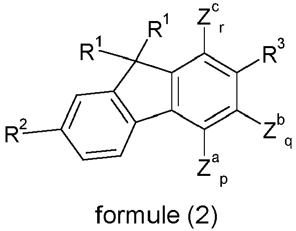 dans laquelle les définitions de la revendication 1 s'appliquent aux symboles qui sont utilisés.

4. Composé selon une ou plusieurs des revendications 1 à 3, **caractérisé en ce qu'**il présente la formule générale (3) 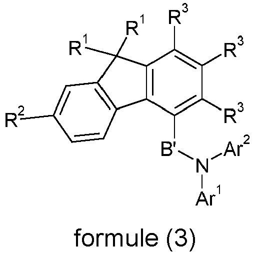 dans laquelle les définitions de la revendication 1 s'appliquent aux symboles qui sont utilisés.

5. Composé selon une ou plusieurs des revendications 1 à 3, **caractérisé en ce qu'**il présente la formule générale (5) 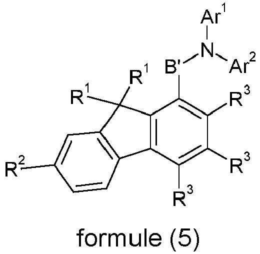 dans laquelle les définitions de la revendication 1 s'appliquent aux symboles qui sont utilisés.

6. Composé selon une ou plusieurs des revendications 1 à 5, **caractérisé en ce que** les deux R¹ sont identiques.

7. Composé selon une ou plusieurs des revendications 1 à 6, **caractérisé en ce que** B' est un groupe phénylène, biphénylène, terphénylène, naphtylène, pyridinylène, pyrimidinylène, pyrazinylène, pyridazinylène, triazinylène, fluorénylène, dibenzofuranylène ou dibenzothiophénylène, lequel peut être substitué par un radical ou par plusieurs radicaux R⁴.

8. Composé selon une ou plusieurs des revendications 1 à 7, **caractérisé en ce que** B' est un groupe phénylène, biphénylène, terphénylène, naphtylène, dibenzofuranylène ou dibenzothiophénylène, lequel peut être substitué par un radical ou par plusieurs radicaux R⁴, B' est de préférence un groupe phénylène, lequel peut être substitué par un radical ou par plusieurs radicaux R⁴, et B' est de façon très préférable un groupe phénylène non substitué.

9. Composé selon une ou plusieurs des revendications 1 à 8, **caractérisé en ce que** le composé présente la formule générale (123), dans laquelle x est égal à 0, 1, 2, 3 ou 4 et dans laquelle y est égal à 0, 1, 2 ou 3

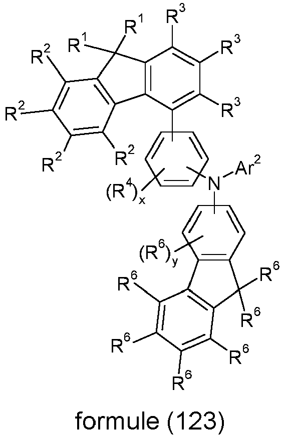

10. Composé selon une ou plusieurs des revendications 1 à 9, **caractérisé en ce que** le composé présente la formule générale (124)

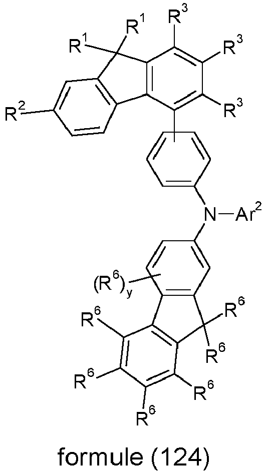

11. Composé selon une ou plusieurs des revendications 1 à 9, **caractérisé en ce que** le composé présente la formule générale (124b)

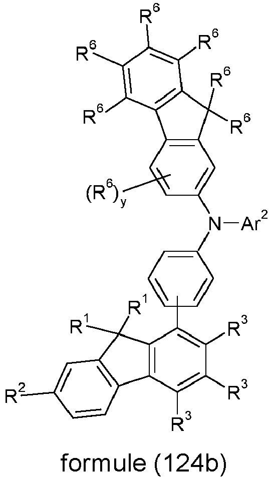

12. Composé selon une ou plusieurs des revendications 1 à 11, **caractérisé en ce que** les deux groupes Ar¹ et Ar² contiennent au moins deux cycles aromatiques ou hétéroaromatiques.

13. Procédé pour la préparation d'un composé selon une ou plusieurs des revendications 1 à 12 au moyen d'un couplage de Buchwald à une seule étape par réaction d'un dérivé de fluorène qui contient un groupe partant avec Ar²-NH-Ar¹.

14. Procédé pour la préparation d'un composé selon une ou plusieurs des revendications 1 à 12 au moyen d'un couplage de Buchwald à deux étapes par réaction par étape d'un dérivé de fluorène qui contient un groupe partant avec (1) Ar²-NH₂ et (2) NH₂-Ar¹.

15. Procédé pour la préparation d'un composé selon une ou plusieurs des revendications 1 à 12, **caractérisé en ce que** le composé est préparé à partir d'un benzochromen-6-one.

16. Procédé selon la revendication 15 comprenant les étapes qui suivent :
a) l'addition d'un composé organométallique sur un benzochromen-6-one et ensuite
b) la cyclisation catalysée par acide de manière à obtenir un dérivé de 4-hydroxyfluorène et ensuite
c) la conversion du groupe hydroxyle à la position 4 du fluorène selon un groupe partant et ensuite
d) la conversion du fluorène selon le produit souhaité.

17. Oligomère, polymère ou dendrimère contenant un ou plusieurs composé(s) selon une ou plusieurs des revendications 1 à 12, où la/les liaison(s) sur le polymère, l'oligomère ou le dendrimère peut/peuvent être localisée(s) au niveau de n'importe quelles positions souhaitées dans la formule (1) qui sont substituées par R¹ à R⁶.

18. Composition comprenant un ou plusieurs composé(s) selon une ou plusieurs des revendications 1 à 12 ou un ou plusieurs polymère(s), oligomère(s) ou dendrimère(s) selon la revendication 17 et au moins un autre matériau qui est sélectionné parmi le groupe qui est constitué par les émetteurs fluorescents, les émetteurs phosphorescents, les matériaux hôtes, les matériaux de matrice, les matériaux de transport d'électrons, les matériaux d'injection d'électrons, les matériaux conducteurs de trous, les matériaux d'injection de trous, les matériaux de blocage d'électrons, les matériaux de blocage de trous et les dopants de type p.

19. Formulation comprenant au moins un composé selon une ou plusieurs des revendications 1 à 12 ou au moins un polymère, un oligomère ou un dendrimère selon la revendication 17 ou au moins une composition selon la revendication 18 et au moins un solvant.

20. Utilisation d'au moins un composé selon une ou plusieurs des revendications 1 à 12 ou d'un polymère, d'un oligomère ou d'un dendrimère selon la revendication 17 dans un dispositif électronique, de préférence dans un dispositif électroluminescent organique, de façon très préférable dans une diode à émission de lumière organique (OLED) ou dans une cellule électrochimique à émission de lumière organique (OLEC, LEEC, LEC), de façon très particulièrement préférable dans une OLED, de préférence dans une couche d'émission (EML), dans une couche de transport d'électrons (ETL) et dans une couche de blocage de trous (HBL), de façon très préférable dans une EML et une ETL et de façon très particulièrement préférable dans une EML.

21. Dispositif électronique comprenant au moins un composé selon une ou plusieurs des revendications 1 à 12 ou au moins un polymère, un oligomère ou un dendrimère selon la revendication 17 ou au moins une composition selon la revendication 18.

22. Dispositif électronique selon la revendication 21, **caractérisé en ce qu'**il est sélectionné parmi les circuits intégrés organiques (OIC), les transistors à effet de champ organiques (OFET), les transistors à film mince organiques (OTFT), les dispositifs électroluminescents organiques, les cellules solaires organiques (OSC), les détecteurs optiques organiques, les photorécepteurs organiques, de préférence un dispositif électroluminescent organique.

23. Dispositif électronique selon la revendication 21 ou 22, **caractérisé en ce qu'**il s'agit d'un dispositif électroluminescent organique qui est également sélectionné parmi le groupe qui est constitué par les transistors à émission de lumière organiques (OLET), les dispositifs à extinction de champ organiques (OFQD), les cellules électrochimiques à émission de lumière organiques (OLEC, LEC, LEEC), les diodes laser organiques (O-laser) et les diodes à émission de lumière organiques (OLED), de préférence les OLEC et les OLED, de façon très préférable les OLED.

24. Dispositif électronique selon une ou plusieurs des revendications 21 à 23, **caractérisé en ce que** le composé selon une ou plusieurs des revendications 1 à 12 ou le polymère, l'oligomère ou le dendrimère selon la revendication 17 ou une composition selon la revendication 18 est utilisé(e) au niveau d'une ou de plusieurs des fonctions qui suivent :
- en tant que matériau de transport de trous dans une couche de transport de trous ou d'injection de trous,
- en tant que matériau de matrice dans une couche d'émission,
- en tant que matériau de blocage d'électrons,
- en tant que matériau de blocage d'excitons,
dans lequel l'utilisation en tant que matériau de transport de trous dans une couche de transport de trous ou dans une couche d'injection de trous a la préférence.

25. Procédé pour la production d'un dispositif électronique selon une ou plusieurs des revendications 21 à 24, **caractérisé en ce qu'**au moins une couche organique est appliquée au moyen d'un dépôt en phase gazeuse ou à partir d'une solution.

26. Dispositif électronique selon la revendication 23, pour une utilisation en médecine pour la photothérapie, de préférence pour une utilisation pour la photothérapie de la peau, de façon très préférable pour une utilisation pour le traitement ou la prévention du psoriasis, de la dermatite, de la jaunisse, de la jaunisse des nouveaux nés, du vitiligo, des inflammations, de la douleur et pour une utilisation pour la cicatrisation des plaies.

27. Utilisation du dispositif selon la revendication 23 dans le domaine des cosmétiques, de préférence pour la réduction ou la prévention du vieillissement de la peau, des rides, des pattes d'oie, de l'acné, des comédons et de la cellulite.

28. Utilisation du dispositif selon la revendication 23 dans des affichages ou pour l'éclairage.
